# EUROPEAN PATENT SPECIFICATION

(11) **EP 3 833 786 B1**
(45) Date of publication and mention of the grant of the patent: **27.12.2023**
(21) Application number: 19749727.4
(22) Date of filing: 07.08.2019
(51) Int. Cl.: A61K 9/127, A61K 9/00, C12N 15/67

(54) **RECOMBINANT NUCLEIC ACID CONSTRUCT**
REKOMBINANTES NUKLEINSÄUREKONSTRUKT
CONSTRUCTION D'ACIDE NUCLÉIQUE DE RECOMBINAISON

(30) Priority: 10.08.2018 EP 18188489; 12.09.2018 EP 18194023; 28.12.2018 EP 18248243
(43) Date of publication of application: 16.06.2021
(73) Proprietor: Pantherna Therapeutics GmbH, 16761 Hennigsdorf (DE)
(72) Inventor: GIESE, Klaus, 14193 Berlin (DE); KEIL, Oliver, 13467 Berlin (DE); KAUFMANN, Jörg, 13465 Berlin (DE)
(74) Representative: Bohmann, Armin K.
(86) International application number: PCT/EP2019/071173
(87) International publication number: WO 2020/030672

(56) References cited:
- WO-A1-2016/091391
- WO-A1-2017/201332
- WO-A1-2018/115525
- WO-A2-00/06759

## Description

The present invention is related to a recombinant nucleic acid construct, a vector, preferably an expression vector, comprising a recombinant nucleic acid construct, a cell comprising the recombinant nucleic acid construct and/or the vector, a delivery vehicle comprising the recombinant nucleic acid construct, a pharmaceutical composition comprising the recombinant nucleic acid construct, the recombinant nucleic acid construct for use in a method for the treatment and/or prevention of a disease, the recombinant nucleic acid construct for use in a method for restoring a cellular function of a cell, a method for the treatment and/or prevention of a disease and a method for restoring a cellular function of a cell.

Nucleic acid molecules serve various purposes in biological systems. One out of several purposes is to store genetic information, another one is to convey genetic information from deoxyribonucleic acid to the ribosome where such genetic information is translated into an amino acid sequence. The latter step involves the use of messenger ribonucleic acid (mRNA).

mRNA shows a very basic design in eukaryotic cells and typically comprises, in 5'-> 3' direction, a Cap structure, a 5' untranslated region (5' UTR), a coding sequence typically starting with a AUG codon attached to a coding sequence (CDS) terminating with a stop codon, a 3' untranslated region (3' UTR) and a poly-A-tail.

mRNA based therapy have been proposed as therapeutics since the beginning of the biotech. In principle, the administered mRNA sequence can cause a cell to make a protein, which in turn could directly treat a disease or could function as a vaccine; more indirectly the protein could interfere with an element of a pathway in such way that the pathway is either inhibited or stimulated, thereby treating or ameliorating a disease.

WO 2017/201332 A1 is related to mRNA therapy of for the treatment of very long-chain acyl-CoA dehydrogenase deficiency (VLCADD).

WO 2018/115525 A1 is related to mRNAs usable as vaccines against lass virus (LASV) infections.

WO 00/06759 A2 is related to plasmid constructs for delivery of therapeutic anti-angiogenic encoding nucleic acids to cells for modulating tumor activity.

WO 2016/091391 A1 is related to an artificial nucleic acid molecule comprising an open reading frame and a 3' UTD comprising at least one poly(A) sequence of polyadenylation signal.

The problem underlying the present invention is the provision of a recombinant nucleic acid construct, preferably of an mRNA, whereby such recombinant nucleic acid construct allows for high expression of the coding sequence of the recombinant nucleic acid construct.

Another problem underlying the present invention is the provision of a recombinant nucleic acid construct, preferably of an mRNA, whereby such recombinant nucleic acid construct allows for the expression of the coding sequence of the recombinant nucleic acid construct in an endothelial cell.

These and other problems underlying the present invention are solved by the subject matter of the attached independent claims; preferred embodiments may be taken from the attached dependent claims.

More specifically, these and other problems are solved in a first aspect by a recombinant nucleic acid construct comprising in 5'-> 3' direction
- a 5' UTR,
- a coding region coding for an effector molecule, and
- a 3' UTR,

wherein the 5' UTR is a 5' UTR of a gene coding for MCP-1 or a derivative thereof having a nucleotide identity of at least 85 %,
wherein the 3' UTR is a 3' UTR of a gene coding for vWF or a derivative thereof having a nucleotide identity of at least 85 %,
wherein the effector molecule is effective in restoring a cellular function of a cell or is effective in exercising a therapeutic effect in or on a cell, and
wherein the recombinant nucleic acid construct is different from a wild type mRNA coding for the effector molecule.

In an embodiment of the first aspect, the 5' UTR and the 3' UTR of the recombinant construct are e from different species.

In an embodiment of the first aspect, the 5' UTR and the 3' UTR of the recombinant construct are from the same species.
In an embodiment of the first aspect, the construct comprises
(a) a poly-A tail, preferably at the 3' terminal end of the recombinant nucleic acid construct; and/or
(b) a CAP structure, preferably at the 5' terminal end of the recombinant nucleic acid construct;
   and/or
(c) a IRES (internal ribosomal entry site) sequence, preferably at the 5' terminal end of the recombinant nucleic acid construct.

In an embodiment of the first aspect, the construct comprises a nucleic acid sequence coding for a signal peptide, preferably the signal peptide is in-frame with the nucleic acid sequence coding for an effector molecule and is arranged between the 5' UTR and the coding region coding for an effector molecule, more preferably the nucleotide sequence coding for a signal peptide is selected from the group comprising a nucleotide sequence coding for a signal peptide of MCP-1 or a derivative thereof having a nucleotide identity of at least 85 %, nucleotide sequence coding for a signal peptide of IL-6 or a derivative thereof having a nucleotide identity of at least 85 %, a nucleotide sequence coding for a signal peptide of Ang-2 or a derivative thereof having a nucleotide identity of at least 85 %, and a nucleotide sequence coding for a signal peptide of Ang-1 or a derivative thereof having a nucleotide identity of at least 85 %, most preferably the signal peptide allows secretion of the effector molecule.

In an embodiment of the first aspect, the cell a cellular function of which is restored and/or the cell in or on which a therapeutic effect is exercised is an endothelial cell, preferably a vascular endothelial cell, more preferably the vascular endothelial cell is a microvascular endothelial cell.

In an embodiment of the first aspect, the cellular function is one which can be restored by an effector molecule having anti-permeability effect of endothelial cells, an anti-vascular leakage effect, an anti-apoptotic effect of endothelial cells or an anti-inflammatory effect of endothelial cells or an anti-stress response effect.

In an embodiment of the first aspect, the effect is linked to or associated with the TIE-2 signalling pathway, VEGF-receptor pathway, NOTCH signalling pathway, PI3-kinase pathway, eNOS signalling pathway, sirtuin-dependent metabolic and energy homeostasis pathway, oxidative stress pathway, shear stress response pathway, ET-1 signal transduction pathway, NO-mediated signal transduction pathway, and mechanochemical transduction pathway.

In an embodiment of the first aspect, the recombinant nucleic acid construct is an mRNA.

In an embodiment of the first aspect, the recombinant nucleic acid construct consists of ribonucleotides, deoxyribonucleotides or a combination thereof.

More specifically, these and other problems are solved in a second aspect by a vector comprising a recombinant nucleic acid construct according to the first aspect, including any embodiment thereof.

More specifically, these and other problems are solved in a third aspect by a cell comprising a recombinant nucleic acid construct according to the first aspect, including any embodiment thereof, and/or a vector according to the second aspect, including any embodiment thereof.

More specifically, these and other problems are solved in a fourth aspect by a delivery vehicle comprising a recombinant nucleic acid construct according to the first aspect, including any embodiment thereof, wherein the delivery vehicle is a cationic lipid delivery particle, preferably the particle is a nanoparticle, more preferably the average size of the nanoparticle is from about 30nm to about 200 nm, preferably from about 30 nm to about 140 nm and more preferably from about 30 nm to about 60 nm.

More specifically, these and other problems are solved in a fifth aspect by a pharmaceutical composition comprising a recombinant nucleic acid construct according to the first aspect, including any embodiment thereof, a vector according to the second aspect, including any embodiment thereof, a cell according to the third aspect, including any embodiment thereof, and/or a delivery vehicle according to the fourth aspect, including any embodiment thereof, and a pharmaceutically acceptable diluent.

More specifically, these and other problems are solved in a sixth aspect by the recombinant nucleic acid construct according to the first aspect, including any embodiment thereof, for use in a method for the treatment and/or prevention of a disease or for use in a method for restoring a cellular function of a cell.

Disclosed but not part of the invention as defined in the claims is a recombinant nucleic acid construct comprising in 5'-> 3' direction
- a 5' UTR,
- a coding region coding for an effector molecule, and
- a 3' UTR,

wherein the 5' UTR is selected from the group comprising a 5' UTR of a gene coding for a 5' UTR of a gene coding for RPL12s.c. or a derivative thereof having a nucleotide identity of at least 85 %, a 5' UTR of a gene coding for Ang-2 or a derivative thereof having a nucleotide identity of at least 85 %, a 5' UTR of a gene coding for HSP70 or a derivative thereof having a nucleotide identity of at least 85 %, a 5' UTR of a gene coding for H3.3. or a derivative thereof having a nucleotide identity of at least 85 %, a 5' UTR of a gene coding for Galectin-9 or a derivative thereof having a nucleotide identity of at least 85 %, a 5' UTR of a gene coding for GADD34 or a derivative thereof having a nucleotide identity of at least 85 %, a 5' UTR of a gene coding for EDN1 or a derivative thereof having a nucleotide identity of at least 85 %, a 5' UTR of a gene coding for HSP70m5 or a derivative thereof having a nucleotide identity of at least 85 %, a 5' UTR of a gene coding for E-selectin or a derivative thereof having a nucleotide identity of at least 85 % a 5' UTR of a gene coding for ICAM-1 or a derivative thereof having a nucleotide identity of at least 85 %, a 5' UTR of a gene coding for IL-6 or a derivative thereof having a nucleotide identity of at least 85 % and a 5' UTR of a gene coding for vWF or a derivative thereof having a nucleotide identity of at least 85 %;
wherein 3' UTR is selected from the group comprising a 3' UTR of a gene coding for a 3' UTR of a gene coding for MCP-1 or a derivative thereof having a nucleotide identity of at least 85 %, a 3' UTR of a gene coding for RPL12s.c. or a derivative thereof having a nucleotide identity of at least 85 %, a 3' UTR of a gene coding for HSP70 or a derivative thereof having a nucleotide identity of at least 85 %, a 3' UTR of a gene coding for H3.3. or a derivative thereof having a nucleotide identity of at least 85 %, a 3' UTR of a gene coding for GADD34 or a derivative thereof having a nucleotide identity of at least 85 %, a 3' UTR of a gene coding for EDN1 or a derivative thereof having a nucleotide identity of at least 85 %, and a 3' UTR of a gene coding for IL-6 or a derivative thereof having a nucleotide identity of at least 85 %,
wherein the effector molecule is effective in restoring a cellular function of a cell or is effective in exercising a therapeutic effect in or on a cell, and
wherein the recombinant nucleic acid construct is different from a wild type mRNA coding for the effector molecule.

In an embodiment of the first aspect, the 5' UTR and the 3' UTR of the recombinant construct are of different origin, preferably are from different endogenous species.

In an embodiment of the first aspect, the 5' UTR and the 3' UTR of the recombinant construct are of the same origin, preferably are from the same endogenous genes species.

In an embodiment of the first aspect, the construct comprises a poly-A tail, preferably at the 3' terminal end of the recombinant nucleic acid construct.

In an embodiment of the first aspect, the construct comprises a CAP structure, preferably at the 5' terminal end of the recombinant nucleic acid construct.

In an embodiment of the first aspect, the construct comprises a IRES (internal ribosomal entry site) sequence, preferably at the 5' terminal end of the recombinant nucleic acid construct.

In an embodiment of the first aspect, the construct comprises nucleic acid sequence coding for a signal peptide, preferably the signal peptide is in-frame with nucleic acid sequence coding for the effector molecule and is arranged between the 5' UTR and the coding region coding for an effector molecule.

In an embodiment of the first aspect, the signal peptide allows secretion of the effector molecule.

In an embodiment of the first aspect, the nucleotide sequence coding for a signal peptide is selected from the group comprising a nucleotide sequence coding for a signal peptide of MCP-1 or a derivative thereof having a nucleotide identity of at least 85 %, nucleotide sequence coding for a signal peptide of IL-6 or a derivative thereof having a nucleotide identity of at least 85 %, a nucleotide sequence coding for a signal peptide of Ang-2 or a derivative thereof having a nucleotide identity of at least 85 %, and a nucleotide sequence coding for a signal peptide of Ang-1 or a derivative thereof having a nucleotide identity of at least 85 %.

In an embodiment of the first aspect, the cellular function is one which can be restored by an effector molecule having anti-permeability effect of endothelial cells, an anti-vascular leakage effect, an anti-apoptotic effect of endothelial cells or an anti-inflammatory effect of endothelial cells or an anti-stress response effect, preferably the effect is linked to or associated with the Tie-2 signalling pathway, VEGF-receptor pathway, NOTCH signalling pathway, PI3-kinase pathway, eNOS signalling pathway, sirtuin-dependent metabolic and energy homeostasis pathway, oxidative stress pathway, shear stress response pathway, ET-1 signal transduction pathway, NO-mediated signal transduction pathway, and mechanochemical transduction pathway.

In an embodiment of the sixth aspect, the recombinant nucleic acid construct according to the first aspect, including any embodiment thereof, is for use in a method for the treatment and/or prevention of a disease, wherein the effector molecule is expressed by an endothelial cell. In an embodiment and as preferably used herein restoring a cellular function comprises and, respectively, encompasses killing of a cell, more preferably killing of tumor endothelial cells and/or tumor cells; in another embodiment and as preferably used herein, restoring cellular function comprises and, respectively, encompasses neoangiogenesis, more preferably neoangiogenesis in case of vascular diseases.

In a n embodiment of the sixth aspect, the treatment and/or prevention of a disease involves restoration of the cellular function of a cell.

In an embodiment of the sixth aspect, the recombinant nucleic acid construct according to the first aspect, including any embodiment thereof, is for use in a method for restoring the cellular function of a cell, wherein the effector molecule is expressed by an endothelial cell.

In an embodiment of the sixth aspect, the recombinant nucleic acid construct according to the first aspect, including any embodiment thereof, is for use in a method for the treatment and/or prevention of a disease, wherein the effector molecule is expressed by an endothelial cell.

In an embodiment of the sixth aspect, the treatment and/or prevention of a disease involves restoration of a cellular function of a cell.

In an embodiment of the sixth aspect, the recombinant nucleic acid construct according to the first aspect, including any embodiment thereof, is for use of in a method for restoring a cellular function of a cell, wherein the effector molecule is expressed by an endothelial cell.

In an embodiment of the sixth aspect, the effector molecule is expressed in the method by an endothelial cell.

In an embodiment of the first aspect, the endothelial cell is a non-dividing endothelial cell, a non-proliferating endothelial cell or a resting endothelial cell. In an embodiment thereof and as preferably used herein the endothelial is a vascular endothelial cell, a lymphatic endothelial cells, an endothelial stem cell (ESC) or an endothelial progenitor cells (EPC).

In an embodiment of the first aspect, the endothelial cell is a senescent endothelial cell.

In an embodiment of the first aspect, the endothelial cell is impaired by age and/or showing stress-related defects.

In an embodiment of the first aspect, the recombinant nucleic acid construct additionally comprises one element selected from the group comprising a cap structure, an IRES sequence, a further start codon providing sequence, a nucleic acid sequence coding for a signal peptide and a poly-A tail.

In an embodiment of the first aspect,
the recombinant nucleic acid construct is one selected from the group comprising in 5'-> 3' direction
a)
   - a 5' non -translated region,
   - a coding region coding for an effector molecule,
   - a 3' non-translated region, and
   - a poly-A tail;
b)
   - a 5' non -translated region,
   - a further start codon providing sequence,
   - a coding region coding for an effector molecule,
   - a 3' non-translated region, and
   - a poly-A tail;
c)
   - a cap structure,
   - a 5' non -translated region,
   - a coding region coding for an effector molecule, and
   - a 3' non-translated region;
d)
   - a cap structure,
   - a 5' non -translated region,
   - a further start codon providing sequence,
   - a coding region coding for an effector molecule, and
   - a 3' non-translated region;
e)
   - an IRES sequence,
   - a 5' non -translated region,
   - a coding region coding for an effector molecule, and
   - a 3' non-translated region;
f)
   - an IRES sequence,
   - a 5' non -translated region,
   - a further start codon providing sequence,
   - a coding region coding for an effector molecule, and
   - a 3' non-translated region;
g)
   - a cap structure,
   - a 5' non -translated region,
   - a coding region coding for an effector molecule,
   - a 3' non-translated region, and
   - a poly-A tail;
h)
   - a cap structure,
   - a 5' non -translated region,
   - a further start codon providing sequence,
   - a coding region coding for an effector molecule,
   - a 3' non-translated region, and
   - a poly-A tail;
i)
   - an IRES sequence,
   - a 5' non -translated region,
   - a coding region coding for an effector molecule,
   - a 3' non-translated region, and
   - a poly-A tail;
j)
   - an IRES sequence,
   - a 5' non -translated region,
   - a further start codon providing sequence,
   - a coding region coding for an effector molecule,
   - a 3' non-translated region, and
   - a poly-A tail;
k)
   - a 5' non -translated region,
   - a nucleic acid sequence coding for a signal peptide,
   - a coding region coding for an effector molecule,
   - a 3' non-translated region, and
   - a poly-A tail;
l)
   - a 5' non -translated region,
   - a further start codon providing sequence,
   - a nucleic acid sequence coding for a signal peptide,
   - a coding region coding for an effector molecule,
   - a 3' non-translated region, and
   - a poly-A tail;
m)
   - a cap structure,
   - a 5' non -translated region,
   - a nucleic acid sequence coding for a signal peptide,
   - a coding region coding for an effector molecule, and
   - a 3' non-translated region;
   - a cap structure
   - a 5' non -translated region,
   - a further start codon providing sequence,
   - a nucleic acid sequence coding for a signal peptide,
   - a coding region coding for an effector molecule, and
   - a 3' non-translated region;
o)
   - an IRES sequence,
   - a 5' non -translated region,
   - a nucleic acid sequence coding for a signal peptide,
   - a coding region coding for an effector molecule, and
   - a 3' non-translated region;
p)
   - an IRES sequence,
   - a 5' non -translated region,
   - a further start codon providing sequence,
   - a nucleic acid sequence coding for a signal peptide,
   - a coding region coding for an effector molecule, and
   - a 3' non-translated region;
q)
   - a cap structure,
   - a 5' non -translated region,
   - a nucleic acid sequence coding for a signal peptide,
   - a coding region coding for an effector molecule,
   - a 3' non-translated region, and
   - a poly-A tail;
r)
   - a cap structure,
   - a 5' non -translated region,
   - a further start codon providing sequence,
   - a nucleic acid sequence coding for a signal peptide,
   - a coding region coding for an effector molecule,
   - a 3' non-translated region, and
   - a poly-A tail;
s)
   - an IRES sequence,
   - a 5' non -translated region,
   - a nucleic acid sequence coding for a signal peptide,
   - a coding region coding for an effector molecule,
   - a 3' non-translated region, and
   - a poly-A tail; and
t)
   - an IRES sequence,
   - a 5' non -translated region,
   - a further start codon providing sequence,
   - a nucleic acid sequence coding for a signal peptide,
   - a coding region coding for an effector molecule,
   - a 3' non-translated region, and
   - a poly-A tail.

In an embodiment of the first aspect, the recombinant nucleic acid comprises in 5' -> 3' direction
- a cap structure,
- a 5' non -translated region,
- a nucleic acid sequence coding for a signal peptide,
- a coding region coding for an effector molecule,
- a 3' non-translated region, and
- a poly-A tail.

In an embodiment of the first aspect, the recombinant nucleic acid comprises in 5' -> 3' direction
- an IRES sequence,
- a 5' non -translated region,
- a nucleic acid sequence coding for a signal peptide,
- a coding region coding for an effector molecule,
- a 3' non-translated region, and
- a poly-A tail.

In an embodiment of the first aspect, recombinant nucleic acid comprises in 5' -> 3' direction
- a cap structure,
- a 5' non -translated region,
- a further start codon providing sequence,
- a nucleic acid sequence coding for a signal peptide,
- a coding region coding for an effector molecule,
- a 3' non-translated region, and
- a poly-A tail.

In an embodiment of the first aspect, the recombinant nucleic acid comprises in 5' -> 3' direction
- an IRES sequence,
- a 5' non -translated region,
- a further start codon providing sequence,
- a nucleic acid sequence coding for a signal peptide,
- a coding region coding for an effector molecule,
- a 3' non-translated region, and
- a poly-A tail.

In an embodiment of the first aspect, the further start codon providing sequence is in-frame with the coding region coding for an effector molecule.

In an embodiment of the first aspect, the 5' non -translated region is a 5' UTR.

In an embodiment of the first aspect, the 5' non-translated region is from a eukaryotic organism, preferably a mammalian organism, more preferably a mammalian organism selected from the group comprising human, non-human primate such as cynomolgus, chimpanzee rhesus monkey, rat and mouse. In an alternative embodiment the 5' non-translated region is from a viral gene, preferably the virus is a virus capable of infecting a eukaryotic organism.

In an embodiment of the first aspect, the 3' non-translated region is a 3'-UTR.

In an embodiment of the first aspect, the 3' non-translated region is from a eukaryotic organism, preferably a mammalian organism, more preferably a mammalian organism selected from the group comprising human, non-human primate such as cynomolgus, chimpanzee rhesus monkey, rat and mouse. In an alternative embodiment the 5' non-translated region is from a viral gene, preferably the virus is a virus capable of infecting a eukaryotic organism.

In an embodiment of the first aspect, the 5' UTR and 3' UTR of the recombinant nucleic acid construct are of different origin, preferably are from different species.

In an embodiment of the first aspect, the 5' UTR and 3' UTR of the recombinant nucleic acid construct are of the same origin, preferably are from the same species.

In an embodiment of the first aspect, the construct comprises one or more IRESs (internal ribosomal entry sites) sequences. In a preferred embodiment, the IRES sequences replaced the Cap structure.

In an embodiment of the first aspect, the nucleotide sequence coding for a signal peptide is selected from the group comprising a nucleotide sequence coding for a signal peptide of MCP-1 or a derivative thereof having a nucleotide identity of at least 85 %, nucleotide sequence coding for a signal peptide of IL-6 or a derivative thereof having a nucleotide identity of at least 85 %, , a nucleotide sequence coding for a signal peptide of Ang-2 or a derivative thereof having a nucleotide identity of at least 85 %, and a nucleotide sequence coding for a signal peptide of Ang-1 or a derivative thereof having a nucleotide identity of at least 85 %.

In an embodiment of the first, including any embodiment thereof, the endothelial cell is an endothelial cell stimulated by an inflammatory stimulus.

In an embodiment of the first aspect, including any embodiment thereof, the cellular function is one which can be restored by an effector molecule having anti-permeability effect of endothelial cells, an anti-vascular leakage effect, an anti-apoptotic effect of endothelial cells or an anti-inflammatory effect of endothelial cells or an anti-stress response effect. In an embodiment thereof, the effector molecule stimulates vessel survival, inhibits regression, inhibits apoptosis, stimulates migration, stimulates remodelling, stimulates angiogenesis, stimulates tube-formation/invasion, stimulates proliferation, inhibits leucocyte adhesion, inhibits adhesion molecule expression, inhibits tissue factor expression, inhibits NFKappaB activity or promotes monolayer integrity.

In an embodiment of the first aspect, including any embodiment thereof, the effector molecule is an element of a pathway, wherein the pathway is selected from the group comprising the Tie-2 signalling pathway, VEGF-Receptor pathway, NOTCH signalling pathway, PI3-kinase pathway, eNOS signalling pathway, sirtuin-dependent metabolic and energy homeostasis pathway, oxidative stress pathway, shear stress response pathway ET-1 signal transduction pathway, NO-mediated signal transduction pathway, and mechanochemical transduction pathway.

In an embodiment of the first aspect, including any embodiment thereof, the effector molecule is selected from the group comprising Ang-1, Ang-4, COMP-Ang-1, hCOMP-Ang-1, COMP-Ang-1, COMP-ANG-2, Tie-2 receptor, Tie-1 receptor, PI3-kinase, preferably constitutive active PI3-kinase, hyperactive Tie-2 receptor (e.g. R849W), VE-cadherin, GRB2, GRB7, GRB14, GRB7, IQGAP1, RAC1, RAP1, DOK2, ABIN1 and ABIN2, KLF2 (Krueppel-like factor 2), alpha5 beta1 integrin, CD73, Akt 1, Akt 2 and Akt 3.

In an embodiment of the first aspect, including any embodiment thereof, the cellular function is vascular leakage and, preferably, the pathway is the Tie-2 pathway.

In an embodiment of the first aspect, including any embodiment thereof, the effector molecule is selected from the group comprising VEGF, VEGF-A, VEGF-B, PDGF, bFGF, Sirtuin, eNOS, RAS and c-MYC.

In an embodiment of the first aspect, including any embodiment thereof, wherein the effector molecule restores vascular regeneration or provides for vascular regeneration, preferably vascular endothelial vascular regeneration.

In an embodiment of the first and the sixth aspect, including any embodiment thereof, the cell a cellular function of which is restored and/or the cell in or on which a therapeutic effect is exercised is a non-endothelial cell.

In an embodiment of the first and the sixth aspect, including any embodiment thereof, the non-endothelial cell is selected from the group comprising epithelial cells, alveolar macrophages, Alveolar type I cells, alveolar type II cells, alveolar macrophages, Pneumocytes, lung epithelial cells, hematopoietic cells, bone marrow cells, bone cells, stem cells, mesenchymal cells, neural cells, glia cells, neuron cells, Astrocytes, cells of the peripheral nervous system, cardiac cells, adipocytes, vascular smooth muscle cells, cardiomyocytes, skeletal muscle cells, beta cells, pituitary cells, synovial lining cells, ovarian cells, testicular cells, B cells, T cells, reticulocytes, leukocytes, granulocytes, macrophages, neutrophils, antigen presenting cells (dendritic cells), fibroblasts, hepatocytes, tumor cells and combination thereof.

In an embodiment of the first aspect, including any embodiment thereof, the effector molecule is selected from the group comprising a tumor death ligand, a factor involved in hematopoiesis and blood clotting, a stem cell factor, a growth factors and a cytokine.

In an embodiment of the first aspect, including any embodiment thereof, the tumor death ligand is selected from the group comprising IL-12, members of the TNF gene superfamily of death ligand proteins such as Apo2L/TRAEL, IL-13, IL-10, IL-8, IL-2, interferon beta, secreted frizzled-related protein (SFRP) 1, SFRP 2, SFRP 3, SFRP 4 and SFRP5.

In an embodiment of the first aspect, including any embodiment thereof, wherein the factor involved in hematopoiesis and blood clotting is selected from the group comprising erythropoietin, Factor VII, Factor VIII, Factor IX and heparan-N-sulfatase.

In an embodiment of the first, including any embodiment thereof, the stem cell factor is selected from the group comprising Oct4, Sox2, Klf4 and c-Myc.

In an embodiment of the first, including any embodiment thereof, the growth factor and cytokine is selected from the group comprising adrenomedullin, angiopoietin family, autocrine motility factor, bone morphogenetic proteins, ciliary neurotrophic factor (CNTF), leukemia inhibitory factor (LIF), interleukin-6 (IL-6), macrophage colony-stimulating factor (m-CSF), granulocyte colony-stimulating factor (G-CSF), granulocyte macrophage colony-stimulating factor (GM-CSF), epidermal growth fac-tor (EGF), ephrins, erythropoietin (EPO), fibroblast growth factor family (FGF1-23), somatotropin, GDNF family of ligands (such as glial cell line-derived neurotrophic factor (GDNF), neurturin, persephin, and artemin), growth differentiation factor-9 (GDF9), hepatocyte growth factor (HGF), hepatoma-derived growth factor (HDGF), insulin, insulin-like growth factors -1 and-2 (IGF-1; IGF-2), keratinocyte growth factor (KGF), migration-stimulating factor (MSF), macrophage-stimulating protein (MSP), also known as hepatocyte growth factor-like protein (HGFLP), myostatin (GDF-8), neuregulins 1-4 (NRG1-4), neurotrophins (brain-derived neurotrophic factor (BDNF), nerve growth factor (NGF), neurotrophin-3 (NT-3), neurotrophin-4 (NT-4)), placental growth factor (PGF), platelet-derived growth factor (PDGF), renalase (RNLS) - anti-apoptotic survival factor, T-cell growth factor (TCGF), thrombopoietin (TPO), transforming growth factor alpha (TGF-α), transforming growth factor beta (TGF-β), tumor necrosis factor-alpha (TNF-α), vascular endothelial growth factor family (VEGF), Wnt Signaling Pathway signaling glycoproteins (WNT1, WNT2, WNT2B, WNT3, WNT3A, WNT4, WNT5A, WNT5B, WNT6, WNT7A, WNT7B, WNT8A, WNT8B, WNT9A, WNT9B, WNT10A, WNT10B, WNT11, WNT16), granulocyte colony-stimulating factor (G-CSF), granulocyte macrophage colony-stimulating factor (GM-CSF), interferon- alfa, interferon-beta, interleukin 2 (IL-2), Interleukin 11 (IL-11), Interferon-gamma, IL-1, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23,IL-24, IL-25, IL-26, IL-27, IL-28, IL-29, IL-30, IL-31, IL-32, IL-33, IL-35 and IL-36.

In an embodiment of the sixth aspect, including any embodiment thereof, the disease is characterized by or caused by vascular leakage, preferably leakage of vascular endothelial cells, more preferably leakage of micro vascular endothelial cells.

In an embodiment of the sixth aspect, including any embodiment thereof, a subject suffering from the disease shows inflammation in the lung.

In an embodiment of the sixth aspect, including any embodiment thereof, the disease is selected from the group comprising pneumonia, sepsis and trauma.

In an embodiment of the sixth aspect, including any embodiment thereof, a subject suffering from the disease shows acute respiratory distress syndrome (ARDS).

In an embodiment of the sixth aspect, including any embodiment thereof, disease is pneumonia, preferably pneumonia selected from the group comprising severe community-acquired pneumonia (sCAP), community acquired pneumonia (CAP), hospital-acquired pneumonia (HAP).

In an embodiment of the sixth aspect, including any embodiment thereof, the disease is characterized by or shows lung damage.

In an embodiment of the sixth aspect, including any embodiment thereof, lung damage is immediate lung damage.

In an embodiment of the sixth aspect, including any embodiment thereof, immediate lung damage results from inhalation of toxic gases, toxic lung edema, lung infection of a virus or bacterium, aspiration of stomach contents, aspiration of fresh water, aspiration of salt water, pulmonary contusion, fat embolism, amniotic fluid embolism and inhalation of hyperbaric oxygen.

In an embodiment of the sixth aspect, including any embodiment thereof, lung damage is indirect lung damage.

In an embodiment of the sixth aspect, including any embodiment thereof, indirect lung damage results from sepsis, bacteremia, endotoxinemia, severe trauma, polytrauma including shock, burns, pancreatitis, malaria tropica, drugs and immunosuppression, chronic alcohol abuse, chronic pulmonary diseases, low pH of serum.

In an embodiment of the sixth aspect, including any embodiment thereof, the disease or effect is vascular regeneration or longevity, wherein preferably the effector molecule is selected from the group comprising VEGF, VEGF-A, VEGF-B, PDGF, bFGF, Sirtuin (Sirt 1), PPAR-gamma, AMPK, eNOS, RAS, c-MYC, FOXO3, PI3 kinase *, Akt, Akt* (* indicates fusion or truncated versions of the proteins to generate hyperactive and constitutively active derivatives of the kinases), adenosine A1 receptor, adenosine A2A receptor, adenosine A2B receptor, telomerase, Yamanaka factors (namely Oct4, sox2, klf4, c-My).

In an embodiment of the first aspect, including any embodiment thereof, the effector molecule is an antiapoptotic factor, preferably protecting normal, i.e. non-diseased endothelium, wherein the antiapoptotic factor is preferably selected ffrom the group comprising PTEN, survivin, IAP, cIAP2 and XIAP, neuroglobin (binds cytochrome c), prosurvival pro-teins Bcl-2, Bcl-xl, Bcl-w, mcl-1 A1, NR-13, BHRF1, LMW5-HL, ORF16, KS-Bcl-2, E1b-19K and P53.

In an embodiment of the first aspect, including any embodiment thereof, the effector molecule is an apoptotic factor, preferably targeting tumor endothelium, wherein the apoptotic factor is preferably selected from the group comprising PTEN, survivin, IAP, cIAP2 and XIAP, neuroglobin (binds cytochrome c), prosurvival pro-teins Bcl-2, Bcl-xl, Bcl-w, mcl-1 A1, NR-13, BHRF1, LMW5-HL, ORF16, KS-BCl-2, E1b-19K, P53.

In an embodiment of the first aspect, including any embodiment thereof, the effector molecule is a tumor death ligand, preferably the tumor death ligand is proapoptotic or anti-angiogenic, more preferably the tumor death ligand is selected from the group comprising Members of the TNF gene superfamily of death ligand proteins such as TNF-alpha, Fas-L, Apo2L/TRAIL, IL-12, IL-13, IL-10, IL-8, IL-2, , secreted frizzled-related protein (SFRP) family (SFRP1, 2, 3, 4, 5), ELTD1, TGF-β, TNF-a, IL-6, PTEN, p53 and other tumor suppressor genes with proapoptotic function, thrombospondin C, TIMP-1, TIMP-2, interferon-alpha, interferon-beta, interferon-gamma, Ang-1 and derivatives with Tie-2 agonistic function, cytochrome c, BH3-only proteins, pro-apoptotic Bcl-2 proteins Bad, Bid, Bax, Bak and Bim, Apaf1, procaspase-8, -9 -10, caspases 2, 3, 6, 7, 8, 9 and 10.

In an embodiment of the first aspect, including any embodiment thereof, the effector molecule is anti-inflammatory, preferably the effector molecule is selected from the group comprising IkappaB and derivative with mutated phosphorylation sites, PI3 kinase and Akt kinases and hyperactive forms.

In an embodiment of the first aspect, including any embodiment thereof, the effector molecule is active in blood coagulation and/or hematopoiesis, preferably the effector molecule is selected from the group comprising erythropoietin, factor VII, factor VIII, factor IX and heparan-N-sulfatase.

In an embodiment of the first aspect, including any embodiment thereof, the effector molecule is a gene editing nuclease such as Cas9.

In an embodiment of the first, including any embodiment thereof, the effector molecule is coding for a therapeutic antibody or a decoy.

In an embodiment of the first aspect, including any embodiment thereof, the effector molecule is effective as a tumor vaccine, preferably the effector molecule is an antigen eliciting an immune response against a tumor cell.

In an embodiment of the first aspect, including any embodiment thereof, the effector molecule is a secreted protein
In an embodiment of the first aspect, including any embodiment thereof, the effector molecule is effective as an autocrine factor.

In an embodiment of the first aspect, including any embodiment thereof, the effector molecule is effective as a paracrine factor.

In an embodiment of the first aspect, including any embodiment thereof, the recombinant nucleic acid construct consists of ribonucleotides.

In an embodiment of the first aspect, including any embodiment thereof, the recombinant nucleic acid construct consists of deoxyribonucleotides.

In an embodiment of the first aspect, including any embodiment thereof, the recombinant nucleic acid construct consists of ribonucleotides and deoxyribonucleotides.

In an embodiment of the second aspect, the vector is an expression vector.

In an embodiment of the second aspect, the vector allows expression of the recombinant nucleic acid construct in a cell, preferably an endothelial cell, more preferably the vector is a plasmid or a virus.

In an embodiment of the third aspect, the cell is an endothelial cell or a mesenchymal stem cell.

In an embodiment of the third aspect, the cell is a recombinant cell.

In an embodiment of the third aspect, the cell is an isolated cell.

In an embodiment of the fourth aspect, the delivery vehicle is a cationic lipid delivery particle.

In an embodiment of the fourth aspect, the particle is a nanoparticle.

Also disclosed but not part of the invention as defined in the claims is the use in the manufacture of a medicament of the recombinant nucleic acid construct according to the first aspect, including any embodiment thereof, a vector according to the second aspect, including any embodiment thereof, a cell according to the third aspect, including any embodiment thereof, and/or a delivery vehicle according to the fourth aspect, including any embodiment thereof. In an example thereof, the medicament is for the treatment of a disease, preferably a disease described herein in connection with the second, third, fourth and fifth aspect, including any embodiment thereof.

Also disclosed but not part of the invention as defined in the claims is a method for treating a subject, preferably a human subject, wherein the method comprises administering to the subject a therapeutically effective amount of a recombinant nucleic acid construct according to the first aspect, including any embodiment thereof, a vector according to the second aspect, including any embodiment thereof, a cell according to the third aspect, including any embodiment thereof, a delivery vehicle according to the fourth aspect, including any embodiment thereof and/or a composition according to the fifth aspect, including any embodiment thereof. In an example thereof, the method is for the treatment of a disease, preferably a disease described herein.

Also disclosed but not part of the invention as defined in the claims is a method for vaccinating a subject, preferably a human subject, wherein the method comprises administering to the subject an amount effective to elicit in a or the subject an immune response, preferably a desired immune response, of a recombinant nucleic acid construct according to the first aspect, including any embodiment thereof, a vector according to the second aspect, including any embodiment thereof, a cell according to the third aspect, including any embodiment thereof, a delivery vehicle according to the fourth aspect, including any embodiment thereof and/or a composition according to the fifth aspect, including any embodiment thereof. In an example thereof, the method is for the treatment of a disease, preferably a disease described herein. In a preferred example, the effector molecule is one which is capable of or suitable for eliciting such immune response.

In an embodiment of each and any aspect, including any embodiment thereof, and as preferably used herein, a heterologous construct or recombinant construct is a construct which as such is not existing in a wild type biological system such as a virus, a cell, a tissue, an organ or an organism. More preferably, a heterologous construct or a recombinant construct is one, where at least one element contained in the construct is not combined with a coding region for the effector, wherein said element is selected from the group consisting of a 5' non-translated region, a 3' non-translated region, a cap structure, a signal sequence and a poly-A tail. Preferably, the 5' non-translated region is a 5' UTR and the 3' non-translated region is a 3' UTR.

In an embodiment of each and any aspect, including any embodiment thereof, and as preferably used herein, a therapeutic method or a method for the treatment and/or prevention of a disease is a therapy.

In an embodiment of each and any aspect of the present invention including any embodiment thereof, the coding region coding for an effector molecule is expressed by an endothelial cell. In preferred embodiment thereof, the endothelial cell expressing the effector molecule is an endothelial cell the function of which is impaired. In an alternative preferred embodiment, the endothelial cell expressing the effector molecule is an endothelial cell the function of which is not impaired; in such case, the effector molecule is exported or secreted by the effector molecule producing endothelial cell and exerts its effect on any other cell, typically any other endothelial cell, whereby such other cell is one the function of which is impaired. In a preferred embodiment, an impaired function of a cell is function of such cell which is to be restored in according with the present invention in its various aspect.

As preferably used herein, a derivative of a sequence which displays the same function as the parent sequence; preferably the parent sequence is a or the wild type sequence. In a preferred embodiment, the sequence of the derivative and of the parent case have an identity of at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. It is within the present invention that the derivative is a truncated version of the parent sequence. Such truncation may exist at the 5' end, the 3' end or both the 5' end and the 3' end. If reference is made to a parent sequence in terms of identity, such identity preferable refers to a stretch of subsequent nucleotides shared by both the derivative and the parent sequence with the sequence of the parent sequence serving as the reference sequence.

In an embodiment of each and any aspect, including any embodiment thereof, and as preferably used herein, a coding region coding for an effector molecule comprises or consists of a nucleic acid sequence coding to said effector molecule.

In an embodiment of each and any aspect, including any embodiment thereof, and as preferably used herein, a 5' non-translated region is a 5' non-translated sequence.

In an embodiment of each and any aspect, including any embodiment thereof, and as preferably used herein, a 3' non-translated region is a 5' non-translated sequence.

In an embodiment of each and any aspect, including any embodiment thereof, and as preferably used herein, the indication of any source of any one of the elements contained in the recombinant nucleic acid construct of the invention is to be understood as a substitute of or an alternative description of a nucleotide sequence. Such nucleotide sequence is preferably the one of the respective wild type sequence, more preferably the one of the respective human wild type sequence. It will be appreciated by a person skilled in the art that other species than man may provide such wild type sequence; in an embodiment of the present invention such wild type sequence for such other species than man may be used in the practicing of the present invention. It will also be appreciated by a person skilled in the art that such wild type sequence may be taken from publicly available data bases such as GenBank. The wild type sequence may be subject to changes over time, typically to correct sequencing errors. In an embodiment of the invention, the sequence is the one described in said publicly available data bases such as GenBank at the date of filing of the instant application. To the extent various respective wild type sequences exist, each and any such wild type sequence shall be encompassed by the present invention. In another embodiment, where there is more than one wild type sequences and the more than one wild type sequence is provided by different groups of people such as different geographically defined groups of people and/or different genetically defined groups of people, the wild type sequence prevailing in the most comprehensive group of said different groups of people may be regarded as the wild type sequence.

In an embodiment of each and any aspect, including any embodiment thereof, and as preferably used herein, if reference is made to a derivative of a 5' UTR of a gene indicating that the derivative has a nucleotide identity of at least 85 %, identity is identity referring or relative to the 5' UTR of the gene.

In an embodiment of each and any aspect, including any embodiment thereof, and as preferably used herein, if reference is made to a derivative of a 3' UTR of a gene indicating that the derivative has a nucleotide identity of at least 85 %, identity is identity referring or relative to the 3' UTR of the gene.

In an embodiment of each and any aspect, including any embodiment thereof, and as preferably used herein, a signal peptide is a peptide which mediates directly or indirectly the transfer or secretion of a polypeptide or a protein fused or attached to the signal peptide into the extracellular space, whereupon, preferably, the signal sequence is removed from the polypeptide and protein, respectively.

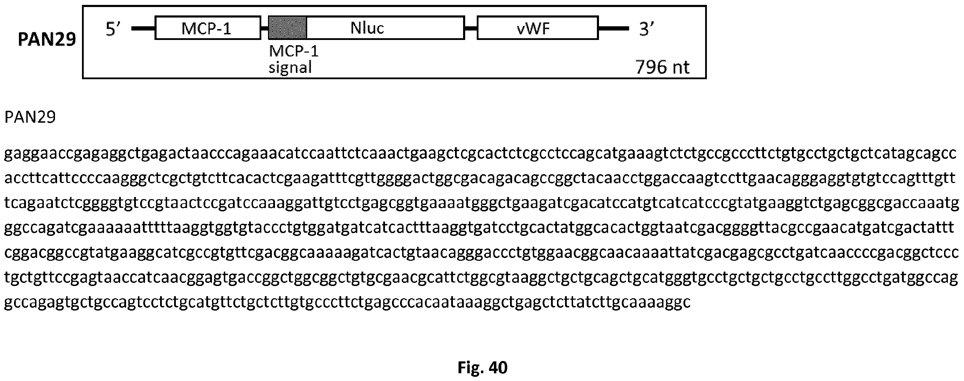

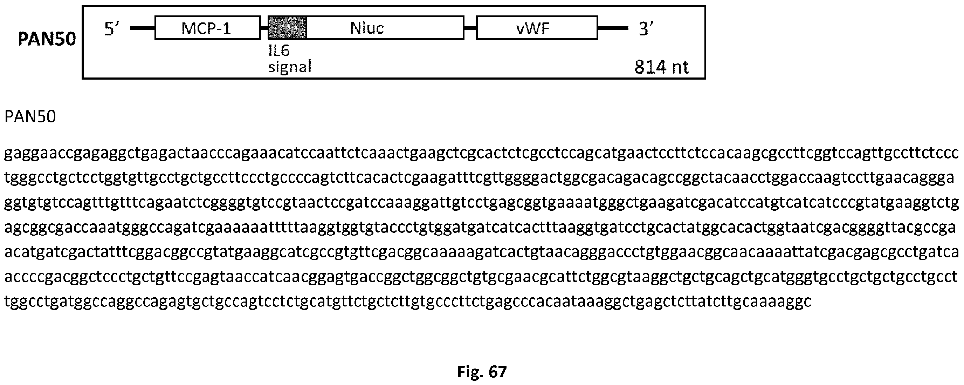

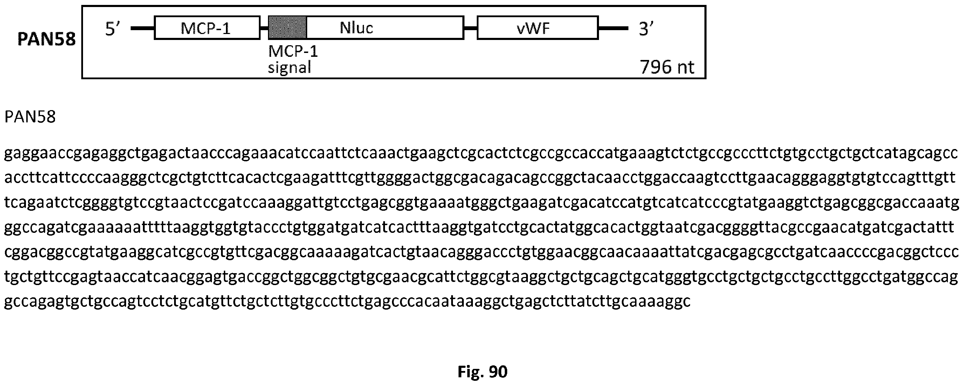

In an embodiment of each and any aspect, including any embodiment thereof, the recombinant nucleic acid construct is a recombinant nucleic acid construct disclosed in the Figs, more specifically a recombinant nucleic acid construct disclosed in Fig. 40 (PAN29), Fig 90 (PAN58) and Fig 67 (PAN50) .

In an embodiment of each and any aspect, including any embodiment thereof, and as preferably used herein, a decoy is a mRNA encoded protein which binds a ligand and neutralizes the signaling activity.

In an embodiment of each and any aspect, including any embodiment thereof, and as preferably used herein, a poly-A tail is a nucleotide sequence comprising or consisting of a plurality of A's covalently attached to each other, whereby such covalent linkage is preferably a phosphodiester linkage. In an embodiment thereof, the poly-A tail comprises about 20 to about 240 As (i.e. adenosinphosphates) nucleotides, preferably about 60 to 120 As, more preferable about 100 to 140 As and most preferably about 120 As.

The poly A tail is thought to stabilize natural messengers and synthetic sense RNA. Therefore, in one embodiment a long poly A tail can be added to an mRNA molecule thus rendering the RNA more stable. Poly A tails can be added using a variety of art-recognized techniques. For example, long poly A tails can be added to synthetic or in vitro transcribed RNA using poly A polymerase (Yokoe, et al. Nature Biotechnology. 1996; 14: 1252-1256). A transcription vector can also encode long poly A tails. In addition, poly A tails can be added by transcription directly from PCR products. Poly A may also be ligated to the 3' end of a sense RNA with RNA ligase (see, e.g., Molecular Cloning A Laboratory Manual, 2nd Ed., ed. by Sambrook, Fritsch and Maniatis (Cold Spring Harbor Laboratory Press: 1991 edition)). In one embodiment, the length of the poly A tail is at least about 90, 200, 300, 400 at least 500 nucleotides. In one embodiment, the length of the poly A tail is adjusted to control the stability of a modified sense mRNA molecule of the invention and, thus, the transcription of protein. For example, since the length of the poly A tail can influence the half-life of a sense mRNA molecule, the length of the poly A tail can be adjusted to modify the level of resistance of the mRNA to nucleases and thereby control the time course of protein expression in a cell. In one embodiment, the stabilized nucleic acid molecules are sufficiently resistant to in vivo degradation (e.g., by nucleases), such that they may be delivered to the target cell without a transfer vehicle. In yet another embodiment the nucleic acid molecule can be purified by means of HPLC in order to remove double-stranded RNA contaminants and thus to reduce innate immune system activation (see e.g. Kariko et al., Nucleic Acids Res. 2011 Nov; 39(21):e142).

In an embodiment of each and any aspect, including any embodiment thereof, and as preferably used herein, the CAP structure is a nucleotide sequence forming a CAP structure which is known to a person skilled in the art. In eukaryotic cells the CAP-structure consists of a m7G(5')ppp(5')G 5'-5'-triphosphate linkage structure. The first two ribose sugars at the 5'-end of the mRNA can be methylated in the 2'-position which is known as CAP-1 (in case the first of the two ribose sugars is methylated) and CAP-2 structure (in case the second of the two ribose sugars is methylated). The CAP-structure can be incorporated during the mRNA transcription or post-transcriptionally by using capping and 2'-O-Methyltransferase enzymes and S-adenosylmethionine (SAM) as a methyl donor. In addition, a phosphatase treatment can be used in order to remove immune stimulatory 5'-phosphates.

In a preferred embodiment of the recombinant nucleic acid construct according to each and any aspect, including any embodiment thereof, the recombinant nucleic acid construct is an mRNA.

In an embodiment of each and any aspect of the present invention, including any embodiment thereof, a 5' UTR of a gene coding for MCP-1 comprises a nucleotide sequence of SEQ ID NO: 20.

An exemplary 3' UTR of a gene coding for MCP-1 comprises a nucleotide sequence of SEQ ID NO: 23.

An exemplary 5' UTR of a gene coding for RPL12s.c. comprises a nucleotide sequence of SEQ ID NO: 24.

An exemplary 3' UTR of a gene coding for RPL12s.c. comprises a nucleotide sequence of SEQ ID NO: 25.

An exemplary 5' UTR of a gene coding for Ang-2 comprises a nucleotide sequence of SEQ ID NO: 26.

An exemplary 3' UTR of a gene coding for Ang-2 comprises a nucleotide sequence of SEQ ID NO: 29.

An exemplary 5' UTR of a gene coding for HSP70 comprises a nucleotide sequence of SEQ ID NO: 38 or 40.

An exemplary 3' UTR of a gene coding for HSP70 comprises a nucleotide sequence of SEQ ID NO: 39 or 43.

An exemplary 5' UTR of a gene coding for H3.3. comprises a nucleotide sequence of SEQ ID NO: 44.

An exemplary 3' UTR of a gene coding for H3.3. comprises a nucleotide sequence of SEQ ID NO: 45.

An exemplary 5' UTR of a gene coding for Galectin-9 (LHALS9) comprises a nucleotide sequence of SEQ ID NO: 46.

An exemplary 3' UTR of a gene coding for Galectin-9 (LHALS9) comprises a nucleotide sequence of SEQ ID NO: 47.

An exemplary 5' UTR of a gene coding for IL-6 comprises a nucleotide sequence of SEQ ID NO: 30.

An exemplary 3' UTR of a gene coding for IL-6 comprises a nucleotide sequence of SEQ ID NO: 33.

An exemplary 5' UTR of a gene coding for vWF comprises a nucleotide sequence of SEQ ID NO: 34.

In an embodiment of each and any aspect of the present invention, including any embodiment thereof, a 3' UTR of a gene coding for vWF comprises a nucleotide sequence of SEQ ID NO: 37.

An exemplary 5' UTR of a gene coding for Ang-1 comprises a nucleotide sequence of SEQ ID NO: 83.

An exemplary 3' UTR of a gene coding for Ang-1 comprises a nucleotide sequence of SEQ ID NO: 88.

An exemplary 5' UTR of a gene coding for Ang-4 comprises a nucleotide sequence of SEQ ID NO: 89.

An exemplary 3' UTR of a gene coding for Ang-4 comprises a nucleotide sequence of SEQ ID NO: 94.

An exemplary 5' UTR of construct PAN57 comprises a nucleotide sequence of SEQ ID NO: 120.

An exemplary 3' UTR of construct PAN57 comprises a nucleotide sequence of SEQ ID NO: 121.

In an embodiment of each and any aspect of the present invention, including any embodiment thereof, the various combinations of a 5' UTR and a 3' UTR as realized in each and any of the specific constructs disclosed herein is a combination of 5' UTR and a 3' UTR which may be realized in recombinant nucleic acid construct irrespective of any other constituent and in particular irrespective of any coding region coding for an effector molecule contained in a recombinant nucleic acid construct of the invention.

In an embodiment of each and any aspect of the present in invention, including any embodiment thereof, and as preferably used herein, an endogenous gene is a gene contained in any cell described herein. In accordance therewith, a recombinant nucleic acid construct where the 5' UTR and the 3' UTR are from different endogenous genes, comprises a 5' UTR from a first gene of a cell and a 3' UTR from a second gene of a cell, for example the 5' UTR is from a gene encoding MCP-1 or Ang-2, and the 3' UTR is from a gene coding for vWF. Said cell may be the same cell or a different cell and/or said cell may be the same kind of cell or a different kind of cell. Preferably, the cell is of human origin.

In a preferred embodiment of the recombinant nucleic acid construct according to each and any aspect, including any embodiment thereof, the recombinant nucleic acid construct is a recombinant nucleic acid molecule.

It is within the present invention defined in the claims and as defined by each and any aspect, including any embodiment thereof, that the nucleotides forming the recombinant nucleic acid molecule may be modified. Suitable modifications include alterations in one or more nucleotides of a codon such that the codon encodes the same amino acid but is more stable than the codon found in the wild-type version of the nucleic acid. For example, an inverse relationship between the stability of RNA and a higher number cytidines (C's) and/or uridines (U's) residues has been demonstrated, and RNA devoid of C and U residues have been found to be stable to most RNases (Heidenreich, et al. J Biol Chem 269, 2131-8 (1994)). In some embodiments, the number of C and/or U residues in an mRNA sequence is reduced. In another embodiment, the number of C and/or U residues is reduced by substitution of one codon encoding a particular amino acid for another codon encoding the same or a related amino acid. Contemplated modifications to the mRNA nucleic acids of the present invention also include the incorporation of pseudouridine, N1-methyl-pseudouridine, 5-methoxyuridine and 5'-Methylcytidine. The incorporation of such modified nucleotides into the recombinant nucleic acid construct, preferably the mRNA of the present invention may enhance stability and translational capacity, as well as diminishing immunogenicity in vivo. (See, e.g., Kariko, K., et al., Molecular Therapy 16 (11): 1833-1840 (2008)). Substitutions and modifications to the recombinant nucleic acid construct of the present invention, particularly at the level of the individual nucleotide of the recombinant nucleic acid construct, may be performed by methods readily known to one of ordinary skill in the art.

The constraints on reducing the number of C and U residues in a sequence will likely be greater within the coding region of an mRNA, compared to an untranslated region, (i.e., it will likely not be possible to eliminate all of the C and U residues present in the message while still retaining the ability of the message to encode the desired amino acid sequence). The degeneracy of the genetic code, however presents an opportunity to allow the number of C and/or U residues that are present in the sequence to be reduced, while maintaining the same coding capacity (i.e., depending on which amino acid is encoded by a codon, several different possibilities for modification of RNA sequences may be possible). For example, the codons for Gly can be altered to GGA or GGG instead of GGU or GGC.

The term modification also includes, for example, the incorporation of non-nucleotide linkages or modified nucleotides into the nucleic acid sequences of the present invention (e.g., modifications to one or both the 3' and 5' ends of an mRNA molecule encoding a functional protein or enzyme). Such modifications include the addition of bases to a nucleic acid sequence (e.g., the inclusion of a poly A tail or a longer poly A tail), the alteration of the 3' UTR or the 5' UTR, complexing the nucleic acid with an agent (e.g., a protein or a complementary nucleic acid molecule), and inclusion of elements which change the structure of a nucleic acid molecule (e.g., which form secondary structures).

The delivery vehicle according to the fourth aspect is preferably based on a composition of one or more suitable lipids (e.g. liposomes) which in combination with the recombinant nucleic acid construct according to the present invention result in overall cationic-lipid-nucleic acid particles (cLNPs). Such cLNPs can be used for functionally transferring a nucleic acid, particularly an mRNA, into endothelial cells of the mammalian vasculature.

Lipid-nucleic acid nanoparticles (LNPs) formed by complexation of nucleic acids such as the recombinant nucleic acid construct of the present invention with cationic lipids in combination with other lipidic components, such as zwitterionic phospholipids, cholesterol and PEGylated lipids, have already been used to block degradation of RNAs in plasma and to facilitate the functional cellular uptake of the RNAs.

However, in a therapeutic context, particularly in view of parenteral therapeutic applications, the overall charge of the lipid-nucleic acid nanoparticles, characterized by the Zeta-potential, is essential in directing the organ distribution of the nanoparticles. Someone skilled in the art will appreciate that overall neutrally charged LNPs are almost exclusively taken up by organs of the reticuloendothelial system, particularly by the liver and spleen. On the other hand, it is also known to those skilled in the art, that overall positively charged lipid nanoparticles are particularly useful to functionally deliver a pharmaceutical payload into cells of the mammalian vasculature.

Those skilled in the art will also appreciate that free nucleic acids, particularly mRNAs, are immune stimulatory if applied intravenously.

Thus, in view of parenteral therapeutic applications a thorough and stable complexation of the nucleic acid inside the lipidic nanoparticle will be essential in order to prevent undesired immunogenic side reactions. Furthermore, it is also known to those skilled in the art that the size of the lipid-nucleic acid nanoparticle is crucial in suppressing the reticuloendothelial clearance of the particles.

It has recently been described that highly potent limit sized siRNA LNPs (~30 nm mean particle size) can be prepared using a microfluidic mixing process based on a staggered herringbone micromixer (SHM) device (Belliveau et al.; Molecular Therapy-Nucleic Acids (2012) 1(8)).

Due to the millisecond mixing of the lipids and the nucleic acid such as the recombinant nucleic acid construct of the invention on a nanoliter scale, the resulting particles are characterized by a very tight packaging of the nucleic acid inside a dense, solid particle core. On the contrary, those skilled in the art will appreciate that macrofluidic mixing processes using T-type connectors, generally result in larger nanoparticles with a more heterogeneous, multilamellar morphology.

In an embodiment, the present invention provides novel positively charged lipid-nucleic acid nanoparticles comprising a nucleic acid such as recombinant nucleic acid construct of the invention for the functional delivery of said recombinant nucleic acid construct of the invention, particularly mRNA, into cells of the mammalian vasculature, particularly into endothelial cells of the lung vasculature.

In an embodiment of the present invention as defined in the claims and as defined by each and any aspect, including any embodiment thereof, a cationic lipid selected from the group comprising β-(L-Arginyl)-L-2,3-diamino propionic acid-N-palmityl-N-oleyl-amide, L-Arginyl-β-alanine-N-palmityl-N-oleyl-amide, DOTAP (N-[1-(2,3-Dioleoyloxy)propyl]-N,N,N-trimethylammonium methyl-chloride), DOTMA (1,2-di-O-octadecenyl-3-trimethylammonium propane (chloride salt)) or DC-cholesterol is combined with neutral lipids and further shielding lipid components bearing a polyethylene glycol (PEG) modification. The neutral lipid can be either a zwitterionic phospholipid selected from a group comprising Diphytanoyl-PE (1,2-Diphytanoyl-sn-glycero-3-phosphoethanolamine), DOPE (1,2-Dioleoyl-sn-glycero-3-phosphoethanolamine), DLPE (1,2-Lauroyl-sn-glycero-3-phosphoethanolamine), DMPE, POPE, DSPE or an uncharged sterol lipid selected from a group comprising cholesterol and stigmasterol. The PEGylated lipid component is selected from a group comprising methoxyPEG-DSPE, methoxyPEG-DLG, methoxyPEG-DMG, methoxyPEG-DPG, methoxyPEG-DSG, methoxyPEG-c-DMA, methoxyPEG-C8-Ceramide, methoxyPEG-C16-Ceramide. In a particular embodiment the chain length of the PEG-chain corresponds to a molecular weight in the range of 750 Da to 5000 Da, preferably in a range of 1500 Da to 3000 Da.

In another embodiment of the present invention as defined in the claims and as defined by each and any aspect, including any embodiment thereof, the molar ratio of the lipids in the lipid mixture is in a range of 20 - 80 mol% cationic lipid, 10 - 70 mol% neutral lipid and 1 - 10 mol% PEGylated lipid, preferably in a range of 35-65 mol% cationic lipid, 35-65 mol% neutral lipid and 1-5 mol% PEGylated lipid (with the overall lipid content being set as 100 %).

In a preferred embodiment of the invention as defined in the claims and as defined by each and any aspect, including any embodiment thereof, the cationic lipid is β-(L-Arginyl)-L-2,3-diamino propionic acid-N-palmityl-N-oleyl-amide or L-Arginyl-β-alanine-N-palmityl-N-oleyl-amide, the neutral lipid is the phospholipid Diphytanoyl-PE and the PEGylated lipid is methoxyPEG2000-DSPE. The molar ratio is 50 mol% cationic lipid, 49 mol% Diphytanoyl-PE and 1 mol% mPEG2000-DSPE (1,2-distearoyl-sn-glycero-3-phosphoethanolamine-N-[methoxy(polyethylene glycol)-2000] (sodium salt)).

In yet another embodiment of the invention as defined in the claims and as defined by each and any aspect, including any embodiment thereof, the mixture of above mentioned lipids is dissolved in a water miscible solvent selected from a group comprising ethanol, acetone, 1-butanol, 2-butanol, tert.-butanol, 3-methyl-1-butanol, 2-methyl-1-propanol, 1-propanol, 2-propanol, dimethylsulfoxide, preferably from a group comprising ethanol, tert.-butanol and 1-butanol.

The dissolved lipid mixture is subsequently rapidly mixed with a dissolution of a nucleic acid such as the recombinant nucleic acid construct of the invention in an aqueous solvent, resulting in the formation of lipid-nucleic acid nanoparticles (LNPs). In a preferred embodiment of the present invention, the mixing is performed using a microfluidic mixing device, particularly using a staggered herringbone-type mixing chamber, e.g. NanoAssemblr^{™} (Precision NanoSystems Inc.; Vancouver, Canada) device.

In yet another embodiment of the invention as defined in the claims and as defined by each and any aspect, including any embodiment thereof, the mass ratio of total lipids to nucleic acid nucleic acid such as the recombinant nucleic acid construct of the invention is in a range of 5 to 60, preferably in a range of 15 to 40 and the volumetric mixing ratio of aqueous nucleic acid solution to organic total lipid solution is in the range of 1:1 to 6:1, preferably in the range of 2:1 to 4:1. The mixing flow-rate of the solutions is in the range of 5 ml/min to 25 ml/min, preferably in a range of 10 ml/min to 20 ml/min.

In a certain embodiment of the invention as defined in the claims and as defined by each and any aspect, including any embodiment thereof, the organic solvent of the resulting mixture is removed after the mixing step by dialysis or by tangential flow filtration using ultrafiltration membranes, whereby the ultrafiltration membranes can be either hollow fiber membranes or flat-screen membranes. The pore size of the ultrafiltration membrane corresponds to a molecular weight cutoff in the range of 1500 Da to 500.000 Da, preferably in a range of 1500 Da to 100.000 Da and even more preferably in a range of 1500 Da to 30.000 Da. Within the tangential flow filtration step it is also possible to concentrate the LNPs to a desired concentration.

The mean particle size of the resulting LNPs can be measured using dynamic light scattering (DLS) technologies and is preferably in the range of 15 nm to 400 nm, more preferably in a range of 25 nm to 200 nm and even more preferable the particles have a size in the range of 25 nm to 100 nm. The overall particle charge of the LNPs is positive and the Zeta-potential of the particles is preferably in a range between +5 mV to +60 mV, more preferably in a range of +25 mV to +60mV.

In certain embodiments of the invention as defined in the claims and as defined by each and any aspect, including any embodiment thereof, the delivery vehicle is a liposomal transfer vehicle, e.g. a lipid nanoparticle or a lipidoid nanoparticle. In one embodiment, the transfer vehicle may be selected and/or prepared to optimize delivery of the recombinant nucleic acid construct of the invention to a target cell. For example, if the target cell is an endothelial cell, the properties of the transfer vehicle (e.g., size, charge and/or pH) may be optimized to effectively deliver such transfer vehicle to the target cell, reduce immune clearance and/or promote retention in that target cell. Alternatively, if the target cell is in the central nervous system (e.g., mRNA such as the recombinant nucleic acid construct of the invention administered for the treatment of neurodegenerative diseases may specifically target brain or spinal tissue), selection and preparation of the transfer vehicle must consider penetration of, and retention within, the blood brain barrier and/or the use of alternate means of directly delivering such transfer vehicle to such target cell. In one embodiment, the compositions of the present invention may be combined with agents that facilitate the transfer of exogenous mRNA (e.g., agents which disrupt or improve the permeability of the blood brain barrier and thereby enhance the transfer of exogenous mRNA to the target cells).

Liposomes (e.g., liposomal lipid nanoparticles) are known to be particularly suitable for their use as transfer vehicles of diagnostic or therapeutic compounds in vivo (Lasic, Trends Biotechnol, 16: 307-321, 1998; Drummond et al, Pharmacol. Rev., 51: 691-743, 1999) and are usually characterized as microscopic vesicles having an interior aqua space sequestered from an outer medium by a membrane of one or more bilayers. Bilayer membranes of liposomes are typically formed by amphiphilic molecules, such as lipids of synthetic or natural origin that comprise spatially separated hydrophilic and hydrophobic domains (Lasic, Trends Biotechnol, 16: 307- 321, 1998). Bilayer membranes of the liposomes can also be formed by amphiphilic polymers and surfactants (e.g., polymerosomes, niosomes, etc.). Such liposomes may also be used as the lipid moiety in the delivery vehicle of the present invention comprising a recombinant nucleic acid construct of the present invention of the invention as defined in the claims and as defined by each and any aspect, including any embodiment thereof.

It is to be acknowledged that any feature related to the recombinant nucleic acid construct as disclosed in connection with a particular aspect of the present invention is equally an embodiment of the recombinant nucleic acid construct of each and any other aspect of the present invention, including any embodiment thereof. More specifically, any embodiment of the recombinant nucleic acid construct disclosed in connection with the first aspect is also an embodiment of the recombinant nucleic acid construct of the second, third and fourth aspect; similarly, any embodiment of the recombinant nucleic acid construct disclosed in connection with the second, third and fourth aspect is also an embodiment of the recombinant nucleic acid construct of the first aspect.

It is generally acknowledged in the art that the UTRs flanking a coding region do not interfere with the coding region and, more specifically, that the UTRs may, in principle, be exchanged without interfering with the expression of the coding region (see, e.g. WO 2017/100551 A1; Trepotec et al.; Tissue Engineering Part A, Vol 0 Nr ja (Apr. 2018) (https://doi.org/10.1089/ten.TEA.2017.0485)

The present invention as defined in the claims is further illustrated by the following Figs. and Examples form which further features, embodiments and advantages of the present invention may be taken.
Fig. 1 is a schematic representation of conventional mRNA molecule structure.
Fig. 2 is a schematic representation of selected examples for mRNA constructs with the coding sequence with an open reading frame being the one of Nano-luciferase, namely PAN02, PAN05, PAN07, PAN08, PAN09, PAN10, PAN11, PAN12, 13, PAN28, PAN29, PAN30, PAN31, PAN32, PAN33, PAN34, PAN35, PAN36, PAN37, PAN38, PAN39, PAN40, PAN41, PAN42, PAN43, PAN44, PAN45, PAN46, PAN47, PAN48 and PAN49.
Fig. 3 is a restriction map of plasmid pcDNA3.1(-) containing construct PAN11.
Fig. 4 is a restriction digest map of plasmid pdDNA3.1(-) of Fig. 3.
Fig. 5 is an 1% agarose gel stained with EtBr showing the non-linearized (uncut, supercoiled, right lanes) and linearization product (left lanes) of pcDNA3.1(-) plasmids containing constructs PAN28 ("28"), PAN13 ("13"), PAN12 ("12"), PAN11 ("11"), PAN10 ("10"), PAN09 ("09"), PAN08 ("08"), PAN07 ("07") and PAN05 ("05") upon BamHI restriction.
Fig. 6 shows 1% agarose gels stained with EtBr showing PCR products of Poly-A tailing PCRs for the addition of 120 nt of Poly-A tail.
Fig. 7A shows the 5' primers used for the 5' UTRs of the indicated gene; the first column indicates in connection with which construct such primer is to be used, the second column indicates the origin of the 5' UTR, and the third column indicates the position where the primer hybridizes to the nucleotide sequence.
Fig. 7B shows the 3' primers used for the 3' UTRs of the indicated gene; the first column indicates in connection with which construct such primer is to be used, the second column indicates the origin of the 3' UTR, and the third column indicates the position where the primer hybridizes to the nucleotide sequence.
Fig. 8 is an image of a non-denaturing agarose gel after EtBr staining showing the *in vitro* mRNA transcripts of various construct.

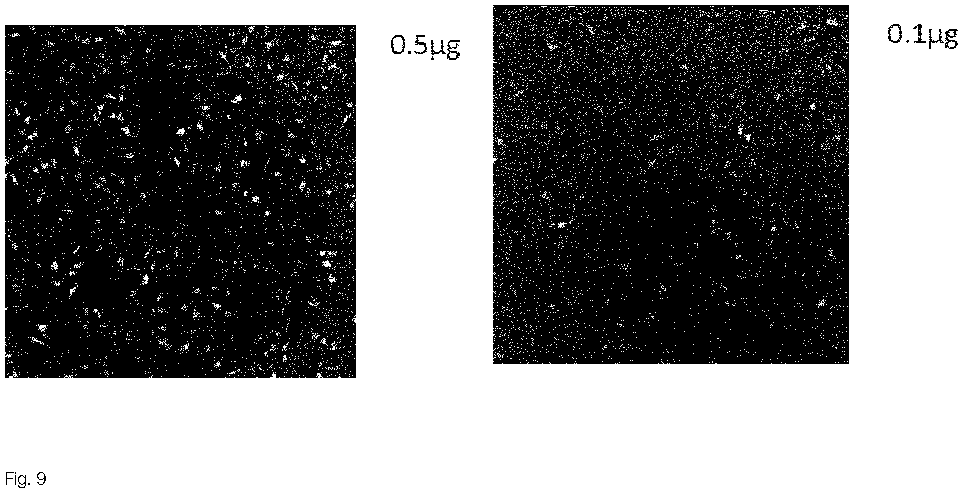

Fig. 9 are photographs of fluorescence microscopy 24 h post transfection of HeLa cells transfected with 0.5 µg (left) and 0.1 µg (right) of a commercially, enhanced mRNA version of the producing green fluorescent protein (TriLINK Biotechnology).
Fig. 10 is a bar diagram showing expression of luciferase, indicated as RLU (relative light units) of luciferase in medium, by the indicated recombinant nucleic acid constructs in HPMEC where luciferase is used as the effector molecule after 2 hours (left column), 6 hours (middle column) and 24 hours (right column) post transfection.
Fig. 11 is a bar diagram showing expression of luciferase, indicated as RLU (relative light units) of luciferase in whole cell lysates of HPMEC, by the indicated recombinant nucleic acid constructs in HPMEC, where luciferase is used as the effector molecule after, 6 hours (left column) and 24 hours (right column) post transfection.
Fig. 12 is a bar diagram showing expression of luciferase, indicated as RLU (relative light units) of luciferase in medium, by the indicated recombinant nucleic acid constructs in HUVEC where luciferase is used as the effector molecule after 2 hours (left column), 6 hours (middle column) and 24 hours (right column) post transfection.
Fig. 13 is a bar diagram showing expression of luciferase, indicated as RLU (relative light units)] of luciferase in whole cell lysates of HUVEC, by the indicated recombinant nucleic acid constructs after lysis of HUVEC, where luciferase is used as the effector molecule after 2 hours (left column), 6 hours (middle column) and 24 hours (right column) post transfection.
Fig. 14 is a bar diagram showing expression of luciferase, indicated as RLU [(relative light units) of luciferase in medium, by the indicated recombinant nucleic acid constructs in HeLa cells where luciferase is used as the effector molecule after 2 hours (left column), 6 hours (middle column) and 24 hours (right column) post transfection.

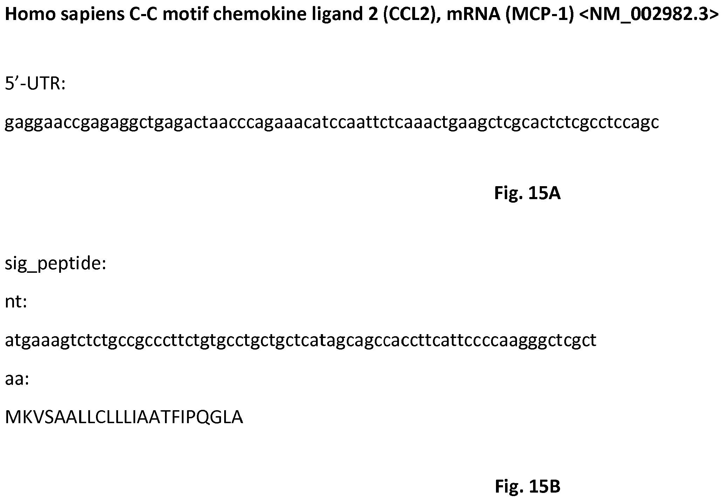

Fig. 15A shows the 5' UTR nucleotide sequence of the mRNA of human MCP-1 which is also referred to as homo sapiens C-C motif chemokine ligand 2 (CCL2) (GenBank entry NM_002982.3).
Fig. 15B shows both the nucleotide sequence and the amino acid sequence of the signal peptide sequence of human MCP-1.

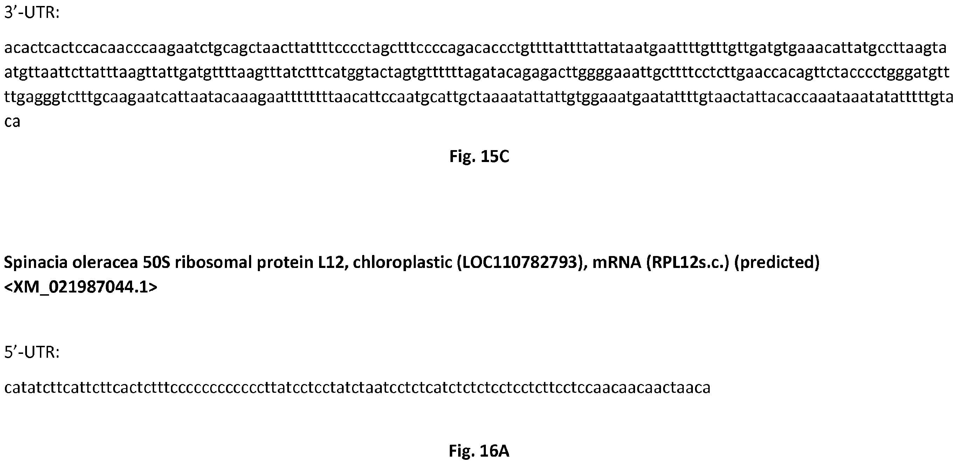

Fig. 15C shows the 3' UTR nucleotide sequence of the mRNA of human MCP-1.
Fig. 16A shows the 5' UTR nucleotide sequence of the mRNA of the 50S ribosomal protein L12, chloroplastic (LOC110782793), of Spinacia oleracea which is also referred to as RPL12s.c. (GenBank entry XM_021987044.1).

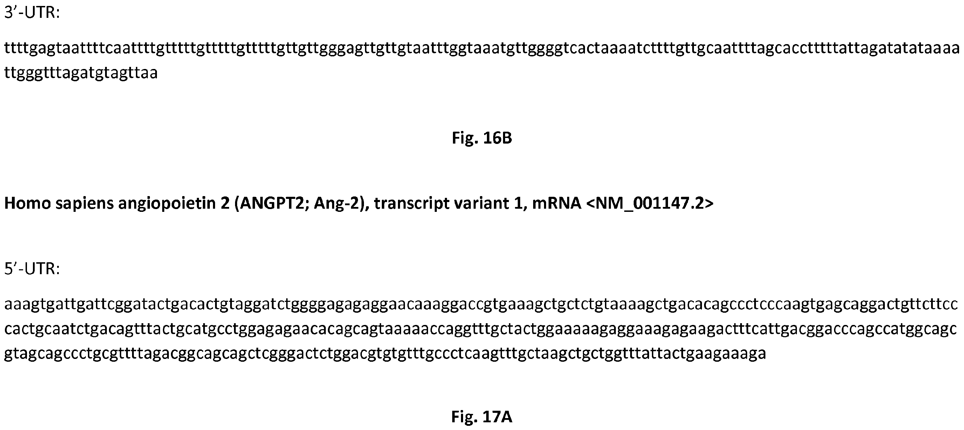

Fig. 16B shows the 3' UTR from the mRNA of the 50S ribosomal protein L12, chloroplastic (LOC110782793), of Spinacia oleracea.
Fig. 17A shows the 5' UTR nucleotide sequence of the mRNA of human angiopoietin 2 which is also referred to as ANGPT2 or Ang-2, transcript variant 1 (GenBank entry NM_001147.2).

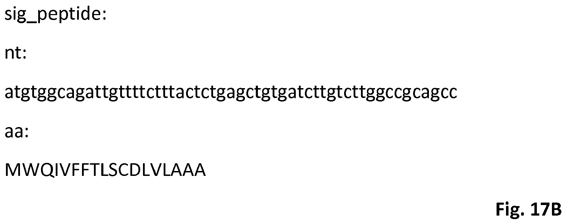

Fig. 17B shows both the nucleotide sequence and the amino acid sequence of the signal sequence of the mRNA of human Ang-2.

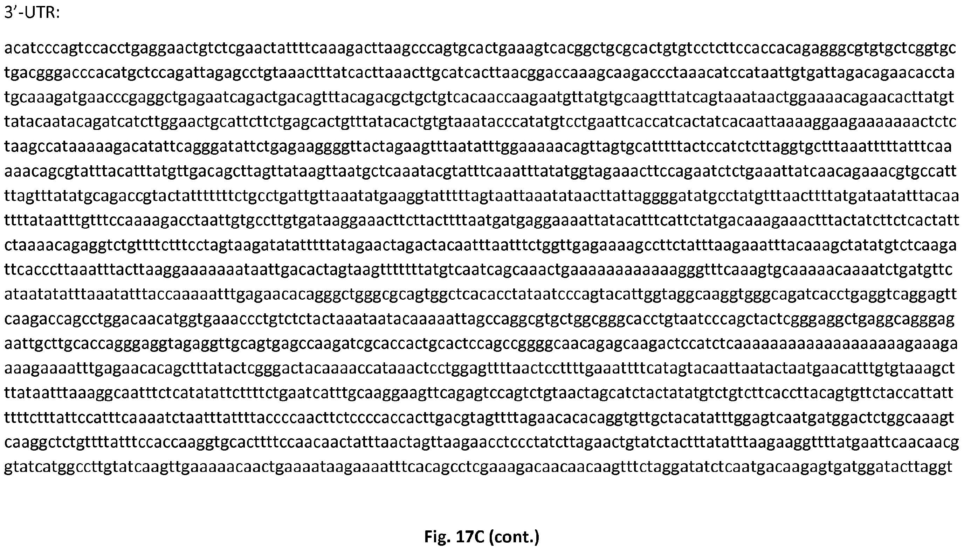

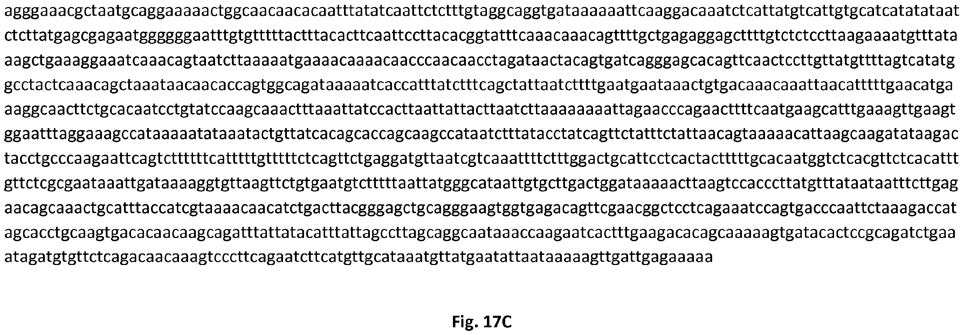

Fig. 17C shows the 3' UTR nucleotide sequence of the mRNA of human Ang-2.

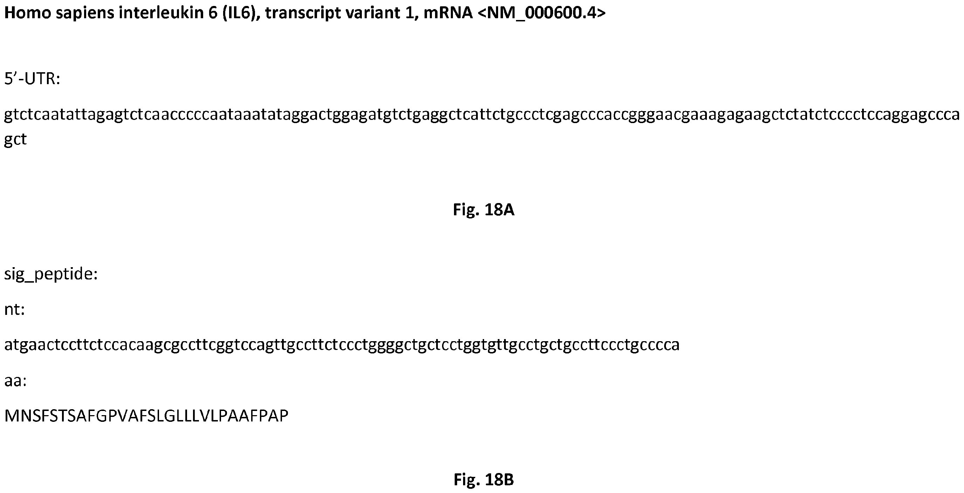

Fig. 18A shows the 5' UTR nucleotide sequence of the mRNA of human interleukin 6 (IL-6), transcript variant 1 (GenBank entry NM_000600.4).
Fig. 18B shows both the nucleotide sequence and the amino acid sequence of the signal sequence of the mRNA of human IL-6.

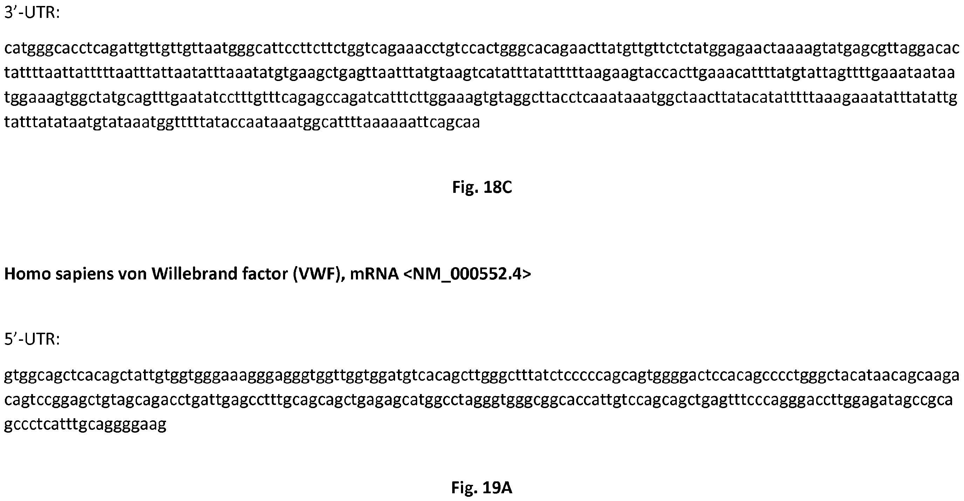

Fig. 18C shows the 3' UTR nucleotide sequence of the mRNA of human IL-6.
Fig. 19A shows the 5' UTR nucleotide sequence of the mRNA of human von Willebrand factor (vWF) (GenBank entry NM_000552.4).

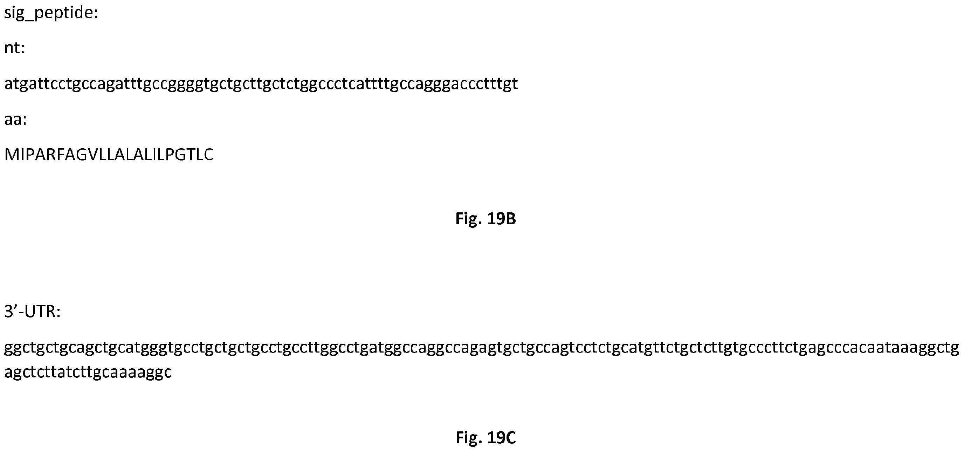

Fig. 19B shows both the nucleotide sequence and the amino acid sequence of the signal sequence of the mRNA of von Willebrand factor (vWF).
Fig. 19C shows the 3' UTR nucleotide sequence of the mRNA of von Willebrand factor (vWF).

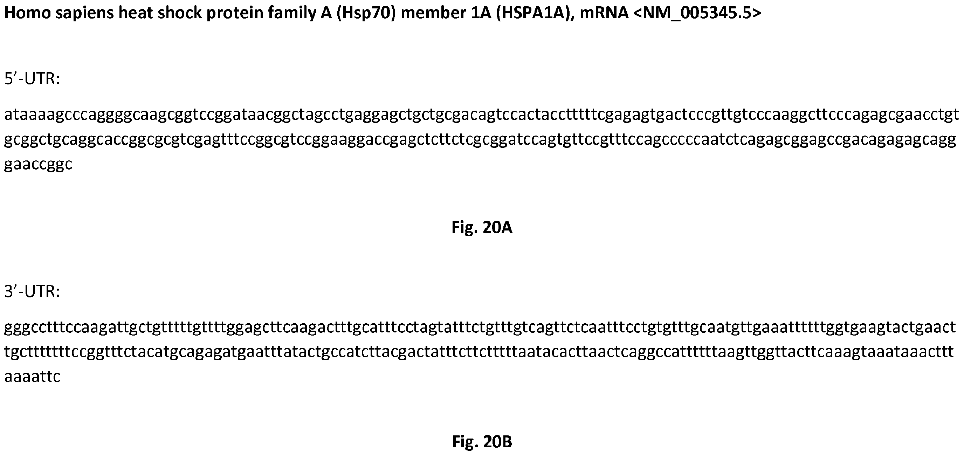

Fig. 20A shows the 5' UTR nucleotide sequence of the mRNA of human heat shock protein family A (Hsp70) member 1A, also referred to as HSPA1A (GenBank entry NM_005345.5).
Fig. 20B shows the 3' UTR nucleotide sequence of the mRNA of human HSPA1A.
Fig. 21A shows the 5' UTR nucleotide sequence of the mRNA of human heat shock protein family A (Hsp70) member 5, also referred to as HSPA5, (GenBank entry NM_005347.4).
Fig. 21B shows both the nucleotide sequence and the amino acid sequence of the signal sequence of the mRNA of human HSPA5.

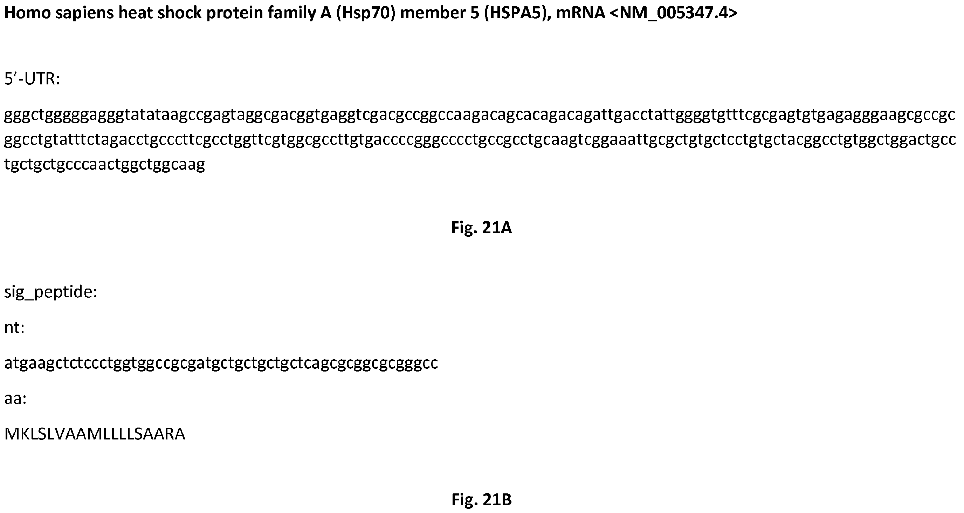

Fig. 21B shows both the nucleotide sequence and the amino acid sequence of the signal sequence of the mRNA of human HSPA5.

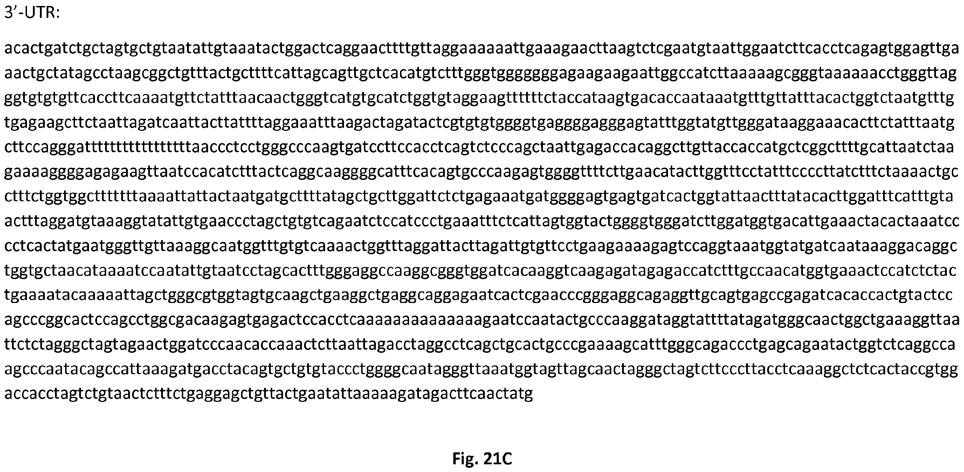

Fig. 21C shows the 3' UTR nucleotide sequence of the mRNA of human HSPA1A.

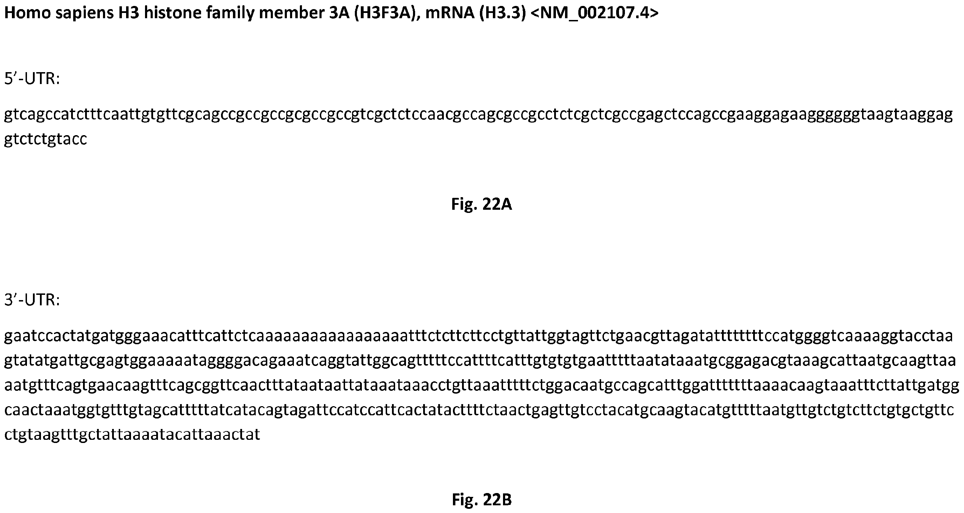

Fig. 22A shows the 5' UTR nucleotide sequence of the mRNA of human H3 histone family member 3A (H3F3A), also referred to as H3.3, (GenBank entry NM_002107.4).
Fig. 22B shows the 3' UTR nucleotide sequence of the mRNA of human H3.3.

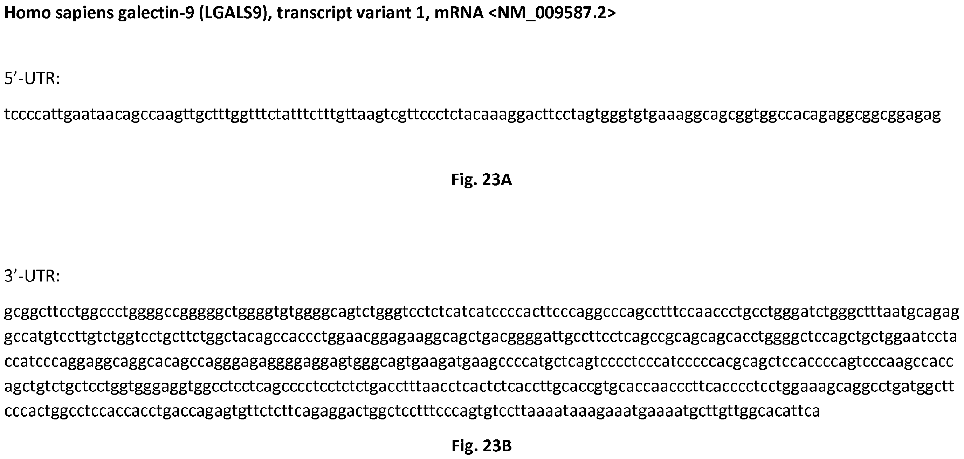

Fig. 23A shows the 5' UTR nucleotide sequence of the mRNA of human galectin-9 (LGALS9), transcript variant 1 (GenBank entry NM_009587.2).
Fig. 23B shows the 3' UTR nucleotide sequence of the mRNA of human galectin-9.

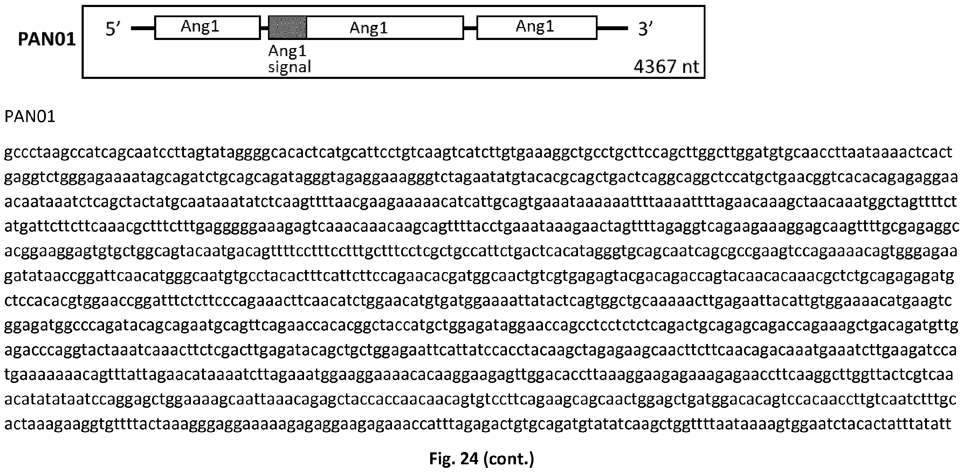

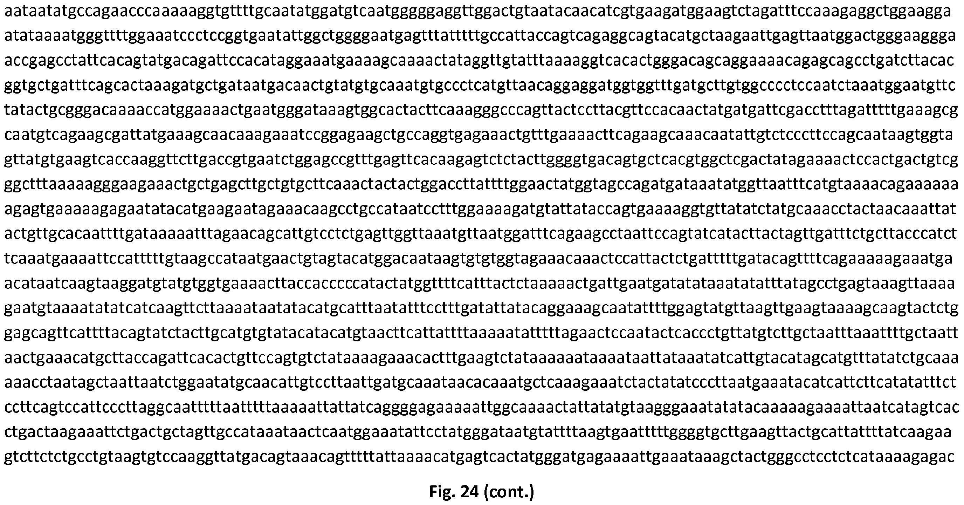

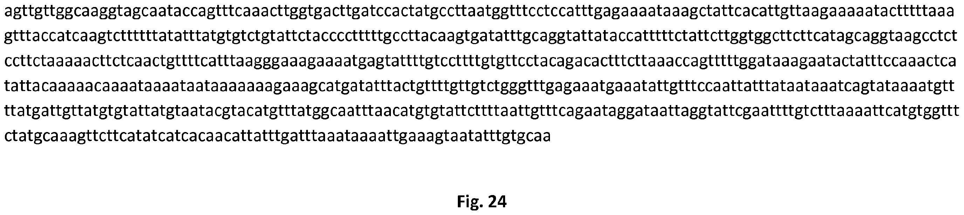

Fig. 24 shows the basic structure and nucleotide sequence of recombinant nucleic acid construct PAN01.

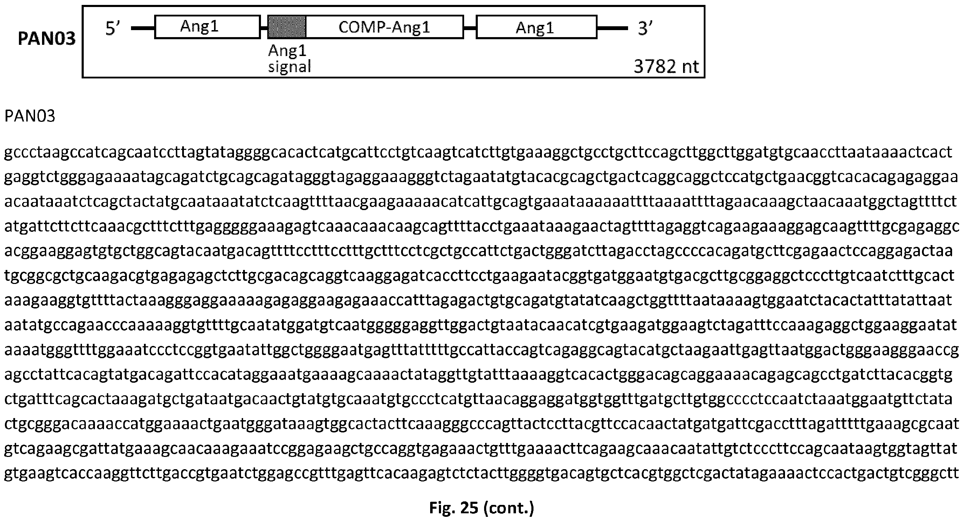

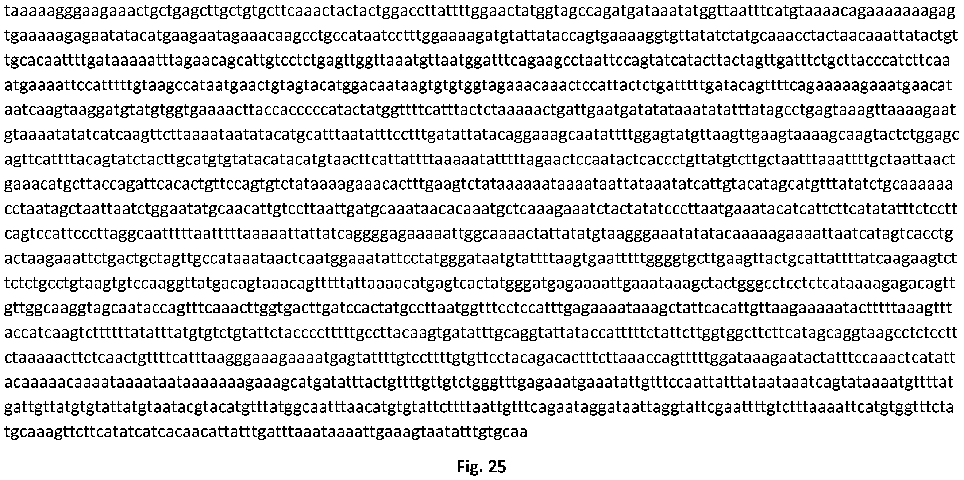

Fig. 25 shows the basic structure and nucleotide sequence of recombinant nucleic acid construct PAN03.

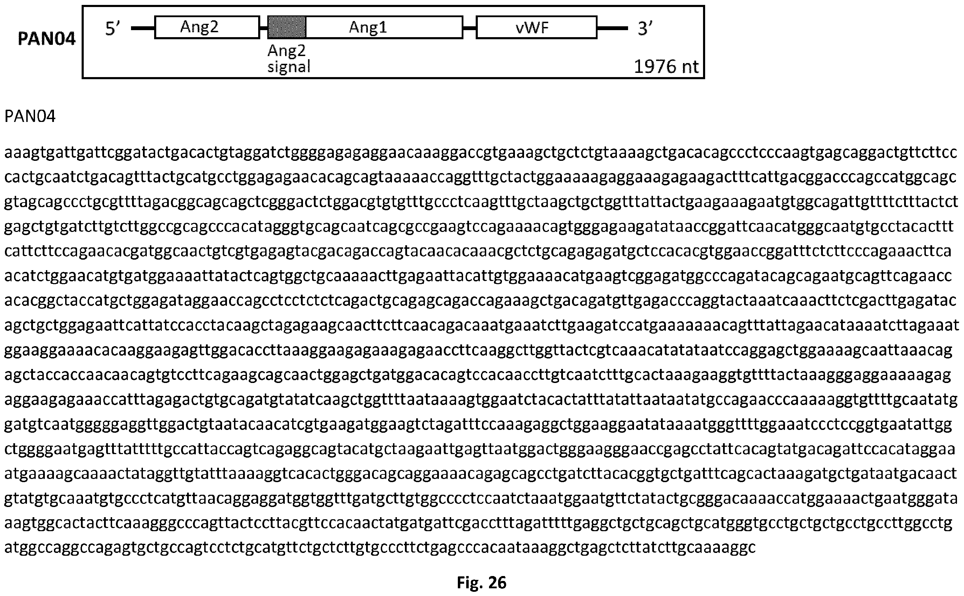

Fig. 26 shows the basic structure and nucleotide sequence of recombinant nucleic acid construct PAN04.

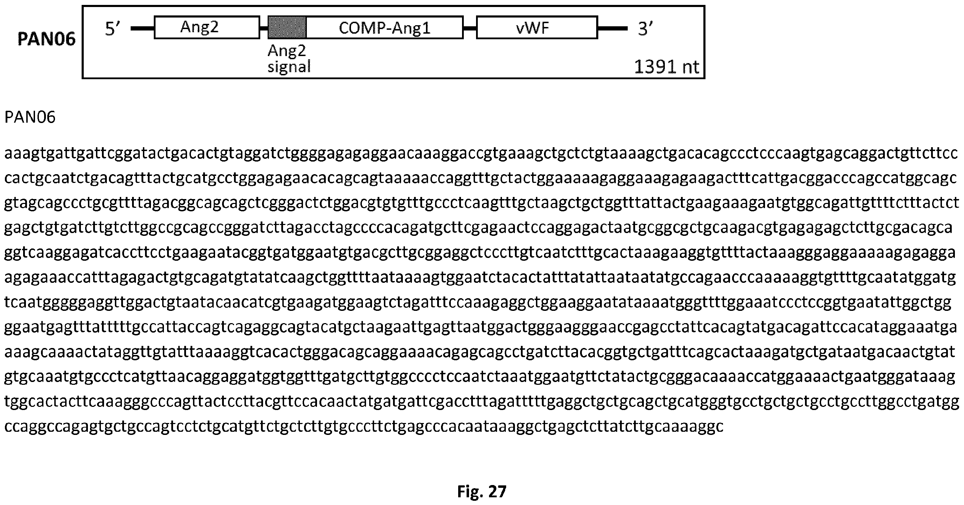

Fig. 27 shows the basic structure and nucleotide sequence of recombinant nucleic acid construct PAN06.

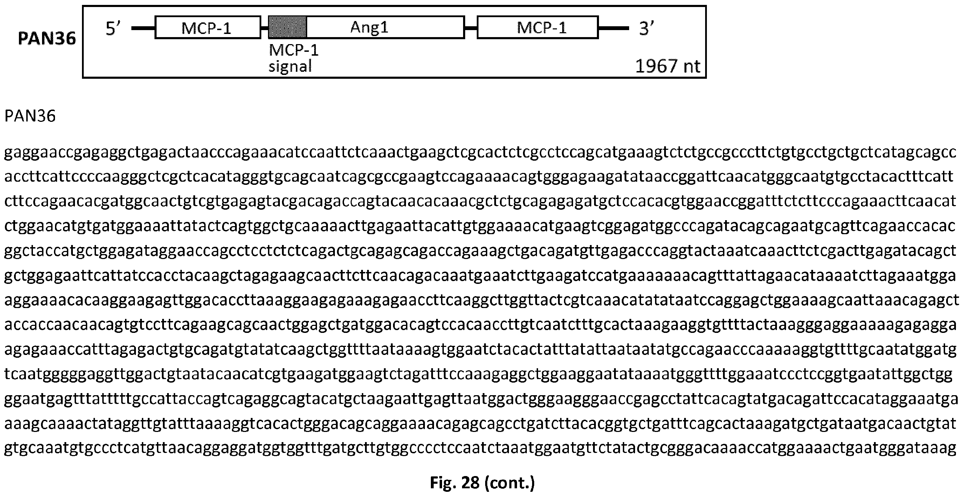

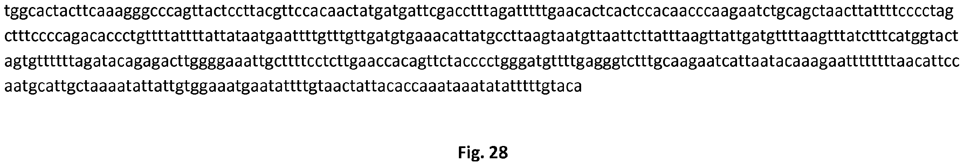

Fig. 28 shows the basic structure and nucleotide sequence of recombinant nucleic acid construct PAN36.

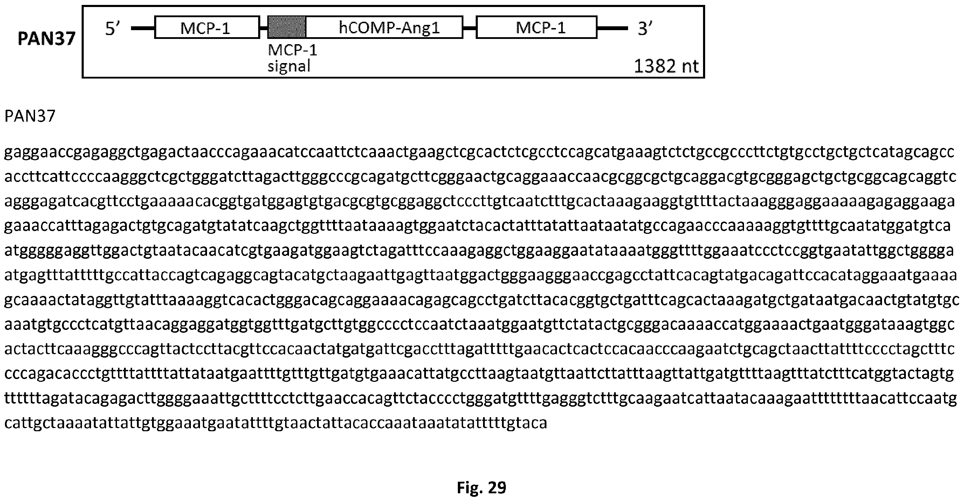

Fig. 29 shows the basic structure and nucleotide sequence of recombinant nucleic acid construct PAN37.

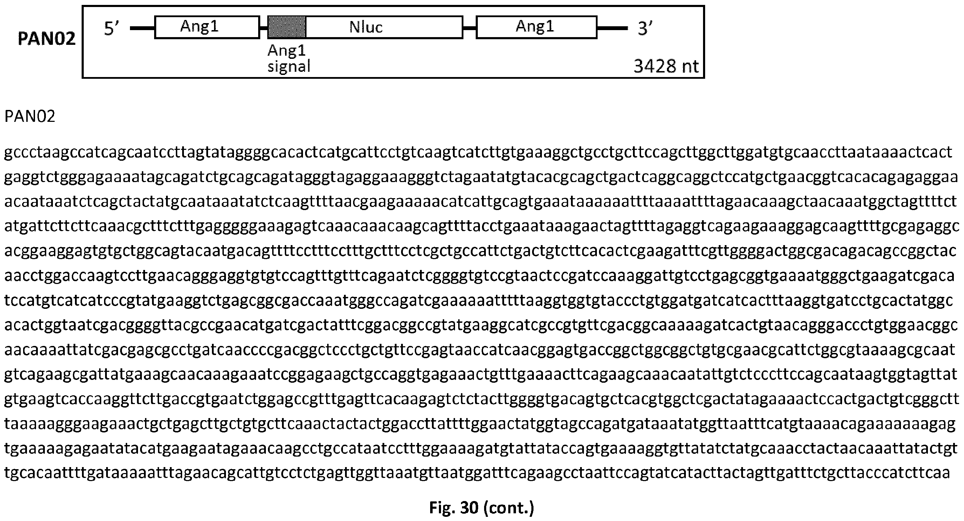

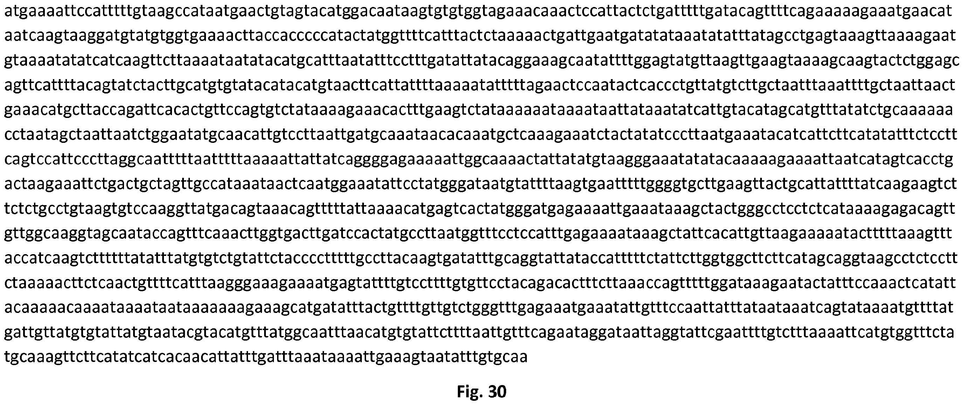

Fig. 30 shows the basic structure and nucleotide sequence of recombinant nucleic acid construct PAN02.

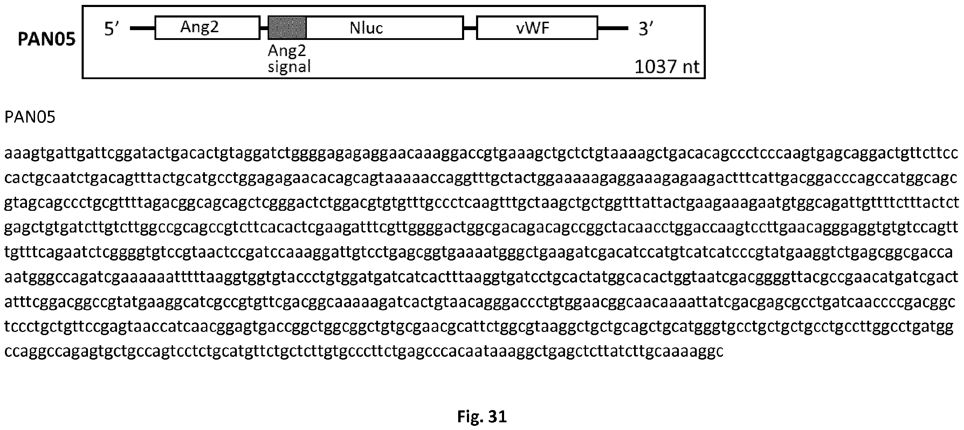

Fig. 31 shows the basic structure and nucleotide sequence of recombinant nucleic acid construct PAN05.

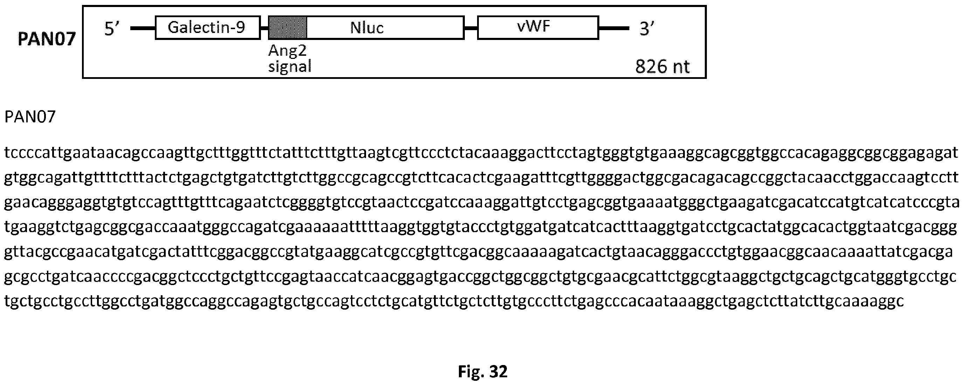

Fig. 32 shows the basic structure and nucleotide sequence of recombinant nucleic acid construct PAN07.

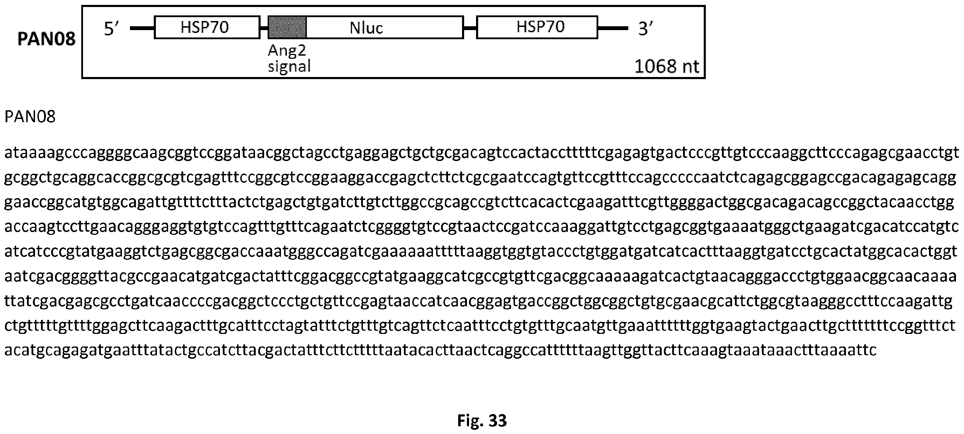

Fig. 33 shows the basic structure and nucleotide sequence of recombinant nucleic acid construct PAN08.

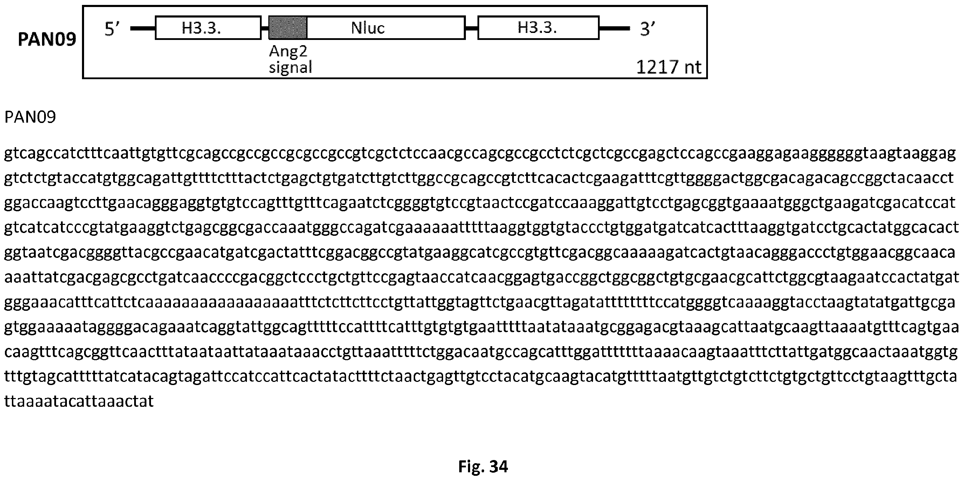

Fig. 34 shows the basic structure and nucleotide sequence of recombinant nucleic acid construct PAN09.

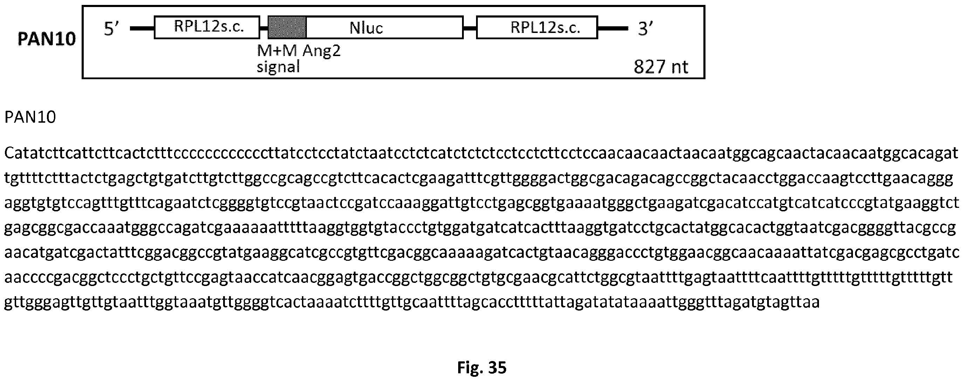

Fig. 35 shows the basic structure and nucleotide sequence of recombinant nucleic acid construct PAN10.

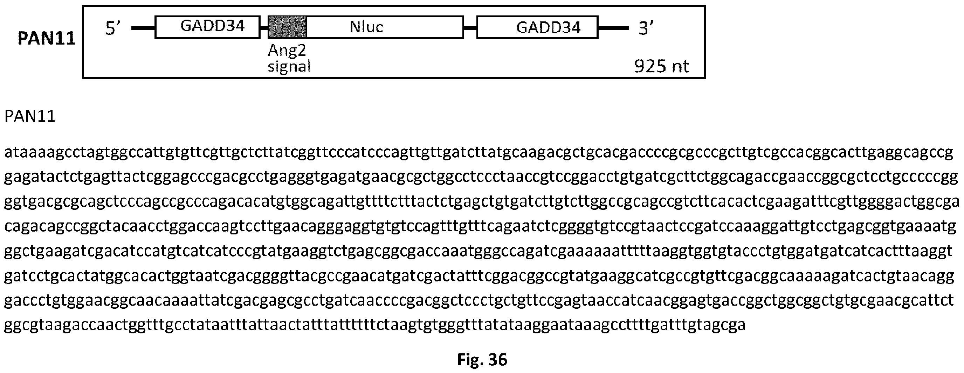

Fig. 36 shows the basic structure and nucleotide sequence of recombinant nucleic acid construct PAN11.

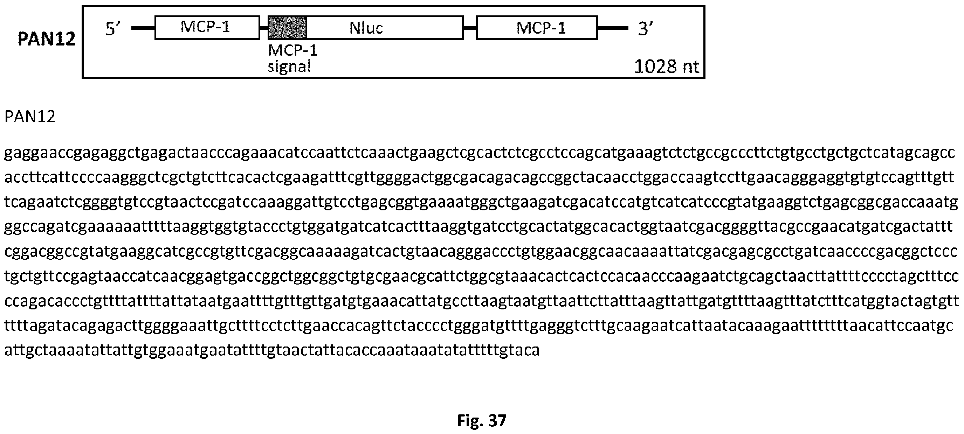

Fig. 37 shows the basic structure and nucleotide sequence of recombinant nucleic acid construct PAN12.

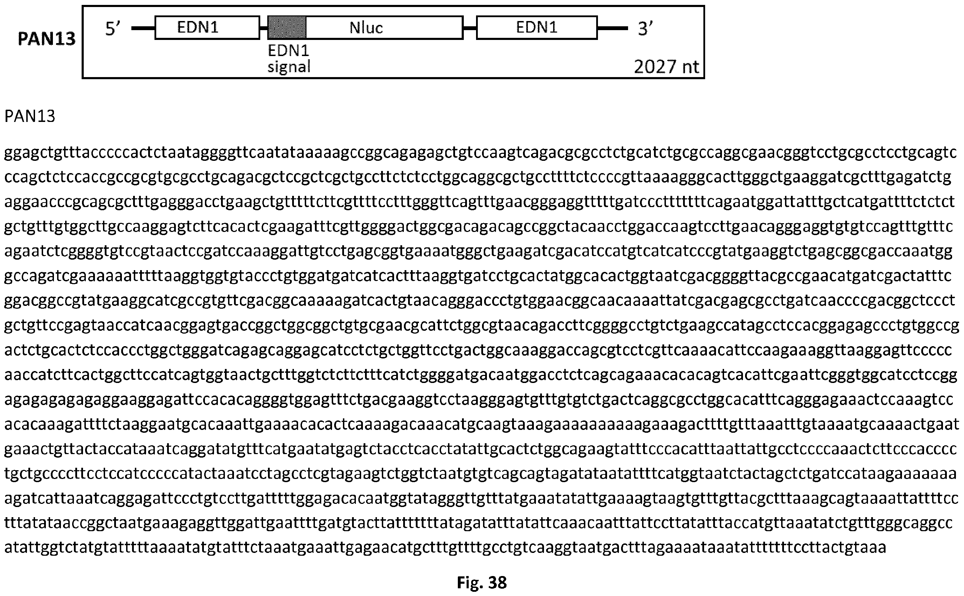

Fig. 38 shows the basic structure and nucleotide sequence of recombinant nucleic acid construct PAN13.

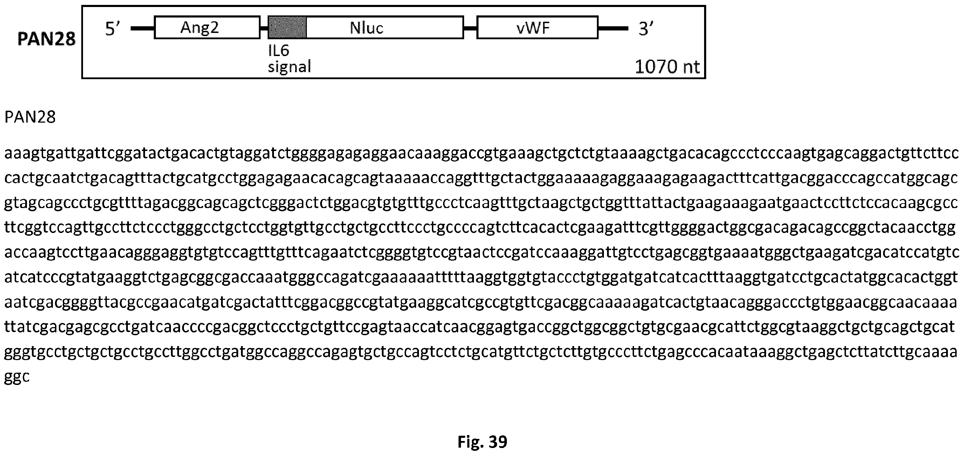

Fig. 39 shows the basic structure and nucleotide sequence of recombinant nucleic acid construct PAN28.
Fig. 40 shows the basic structure and nucleotide sequence of recombinant nucleic acid construct PAN29.

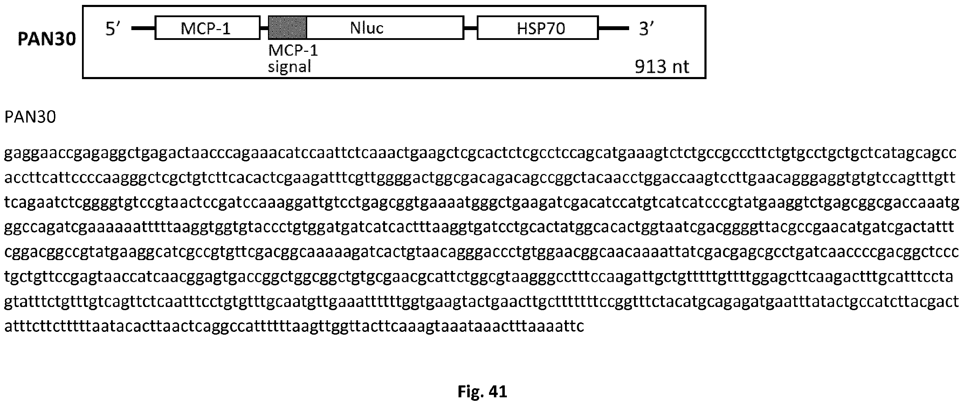

Fig. 41 shows the basic structure and nucleotide sequence of recombinant nucleic acid construct PAN30.

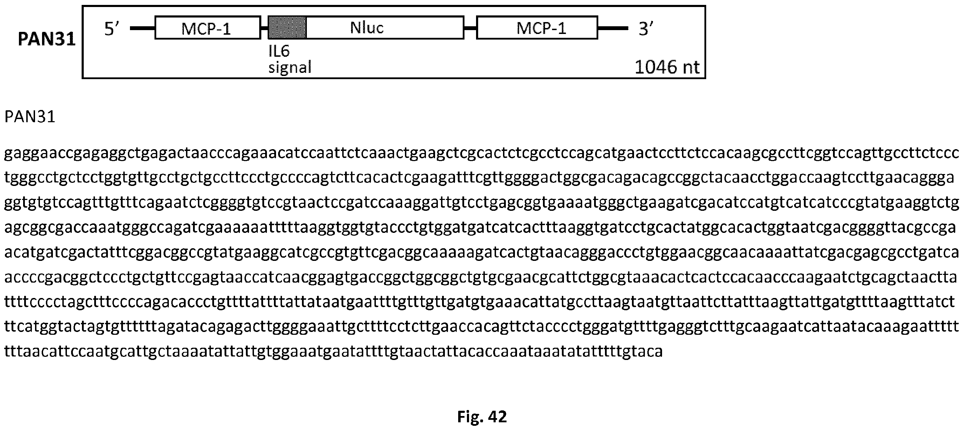

Fig. 42 shows the basic structure and nucleotide sequence of recombinant nucleic acid construct PAN31.

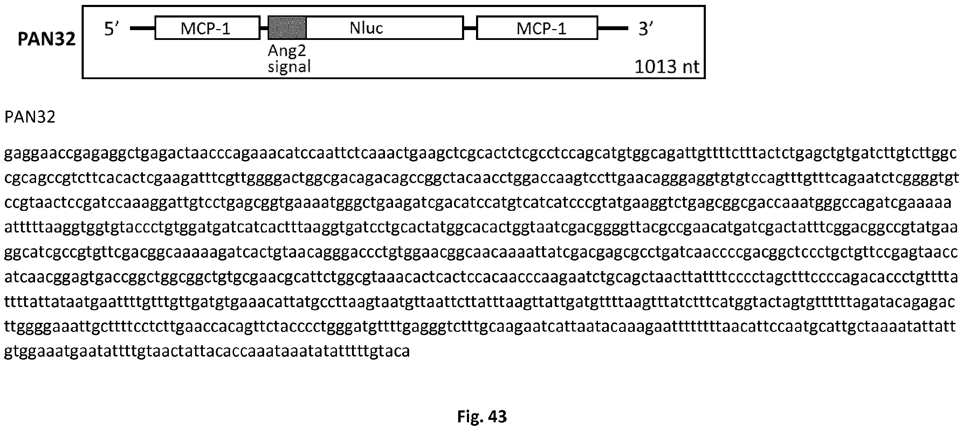

Fig. 43 shows the basic structure and nucleotide sequence of recombinant nucleic acid construct PAN32.

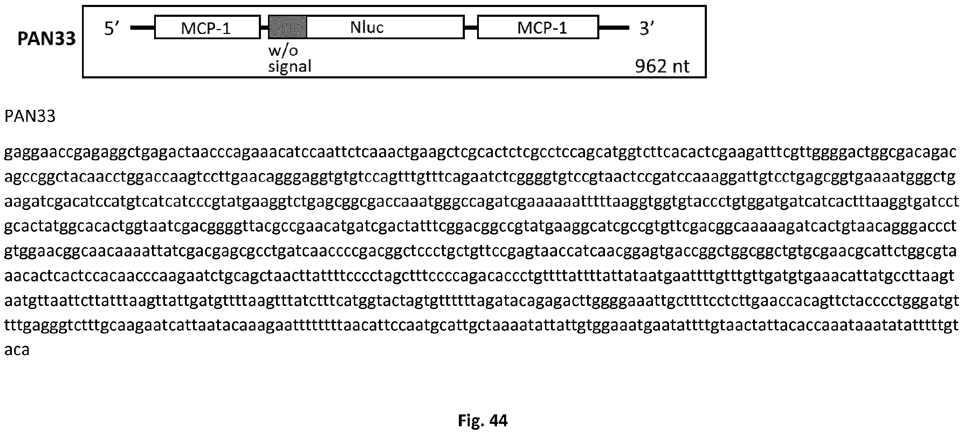

Fig. 44 shows the basic structure and nucleotide sequence of recombinant nucleic acid construct PAN33.

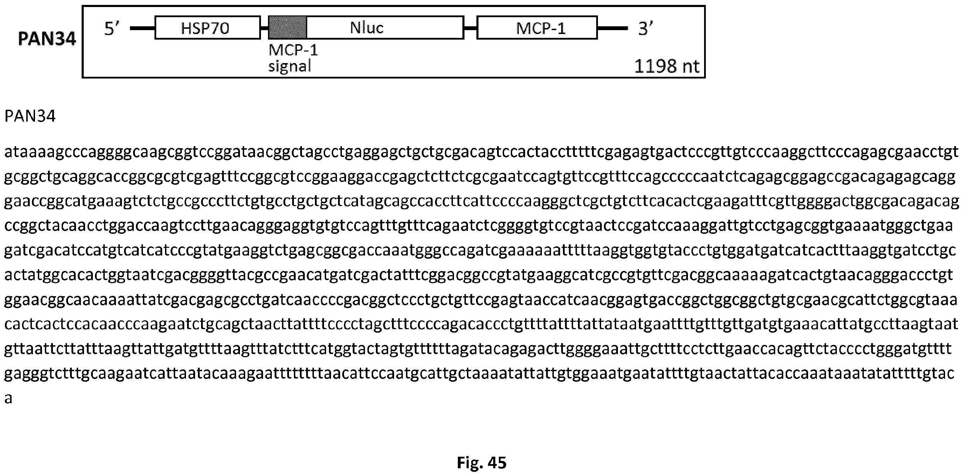

Fig. 45 shows the basic structure and nucleotide sequence of recombinant nucleic acid construct PAN34.

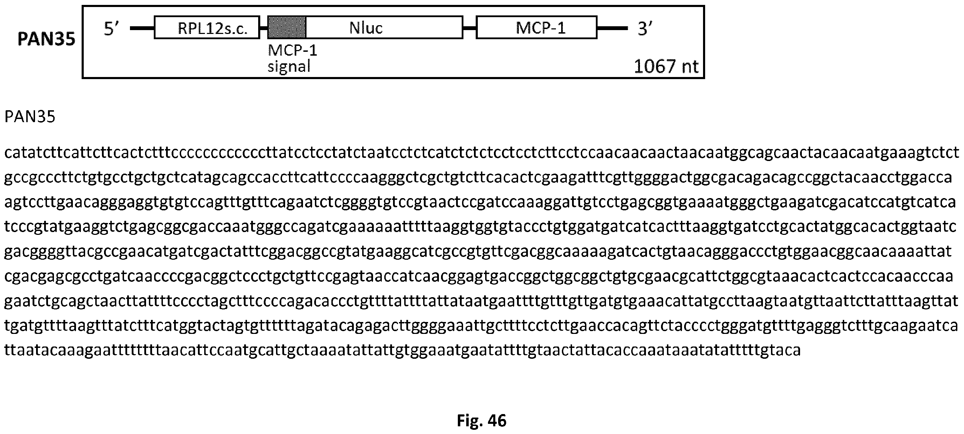

Fig. 46 shows the basic structure and nucleotide sequence of recombinant nucleic acid construct PAN35.

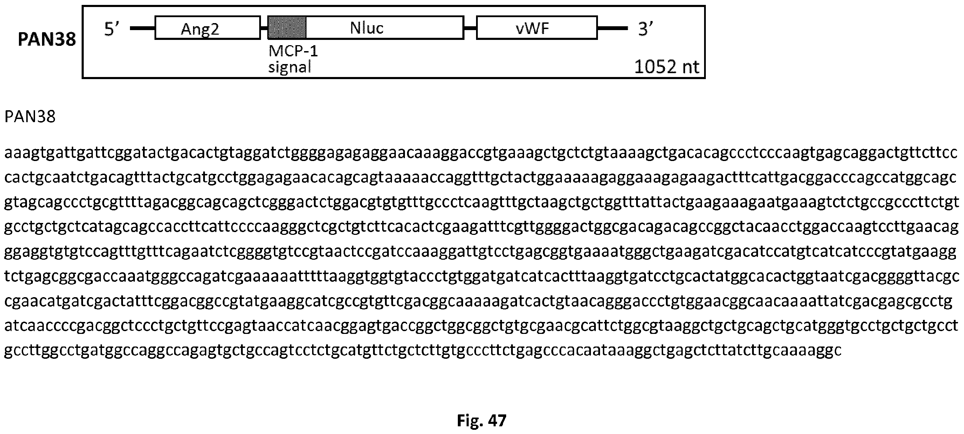

Fig. 47 shows the basic structure and nucleotide sequence of recombinant nucleic acid construct PAN38.

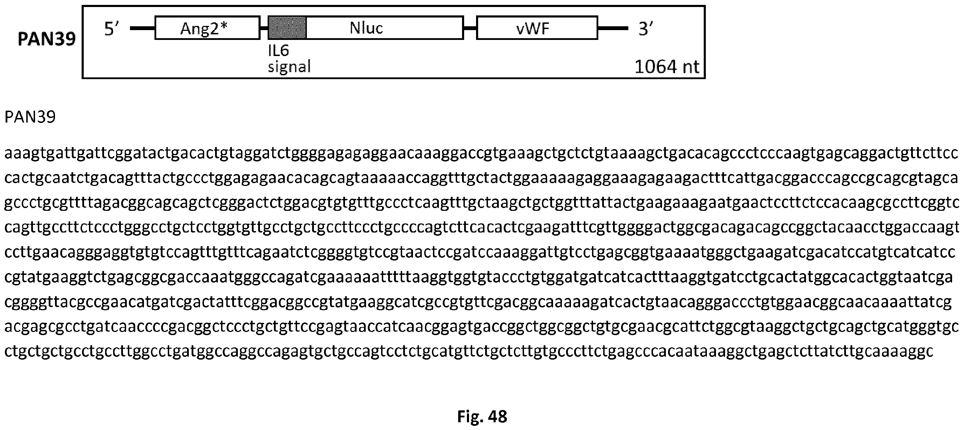

Fig. 48 shows the basic structure and nucleotide sequence of recombinant nucleic acid construct PAN39.

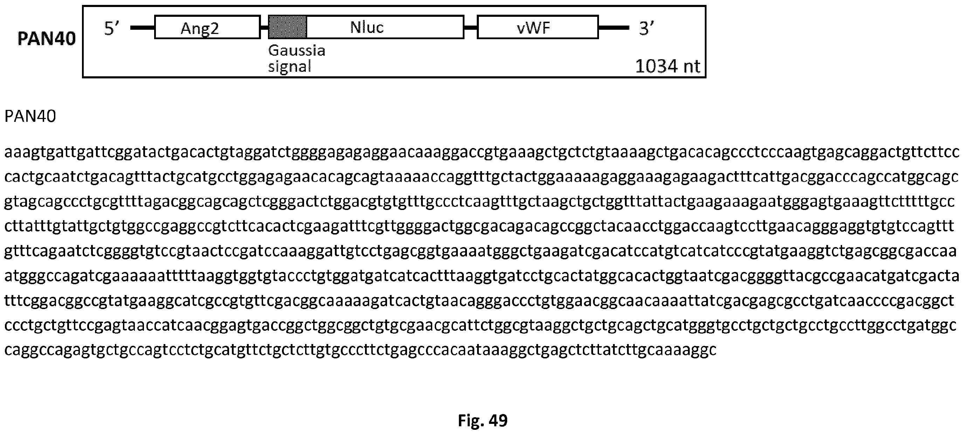

Fig. 49 shows the basic structure and nucleotide sequence of recombinant nucleic acid construct PAN40.

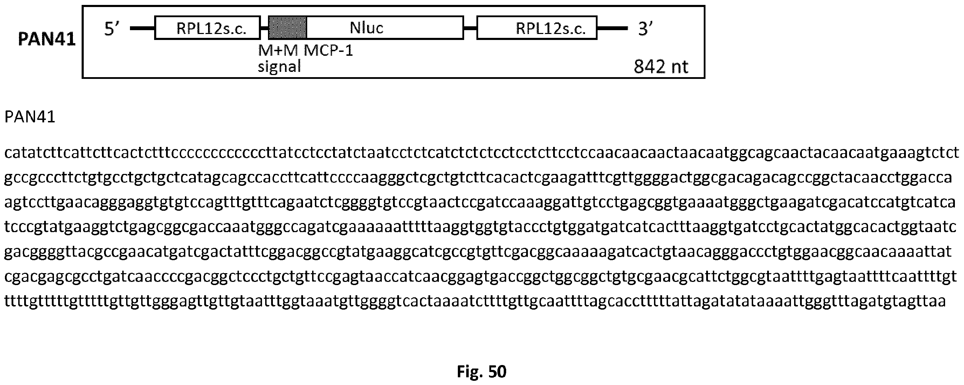

Fig. 50 shows the basic structure and nucleotide sequence of recombinant nucleic acid construct PAN41.

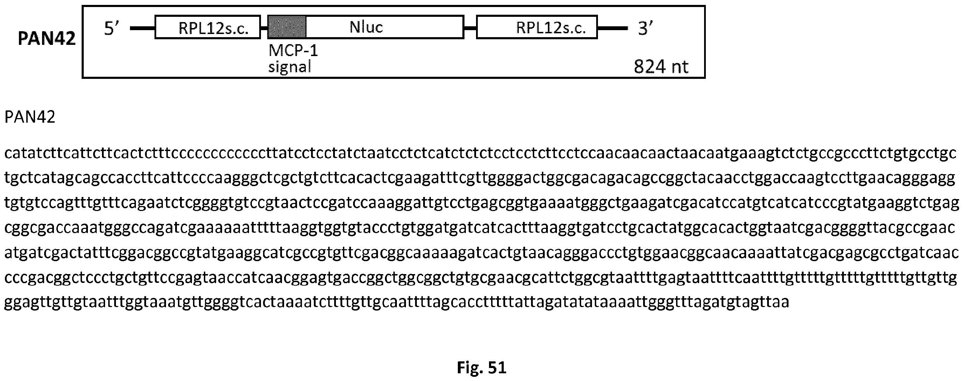

Fig. 51 shows the basic structure and nucleotide sequence of recombinant nucleic acid construct PAN42.

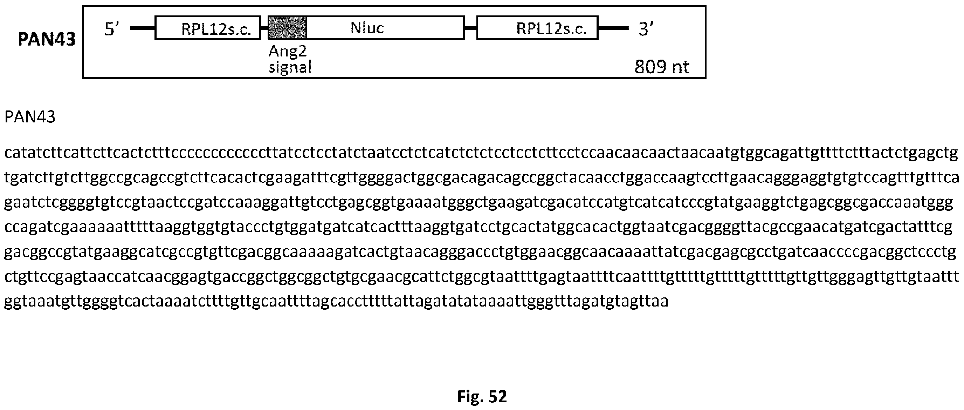

Fig. 52 shows the basic structure and nucleotide sequence of recombinant nucleic acid construct PAN43.

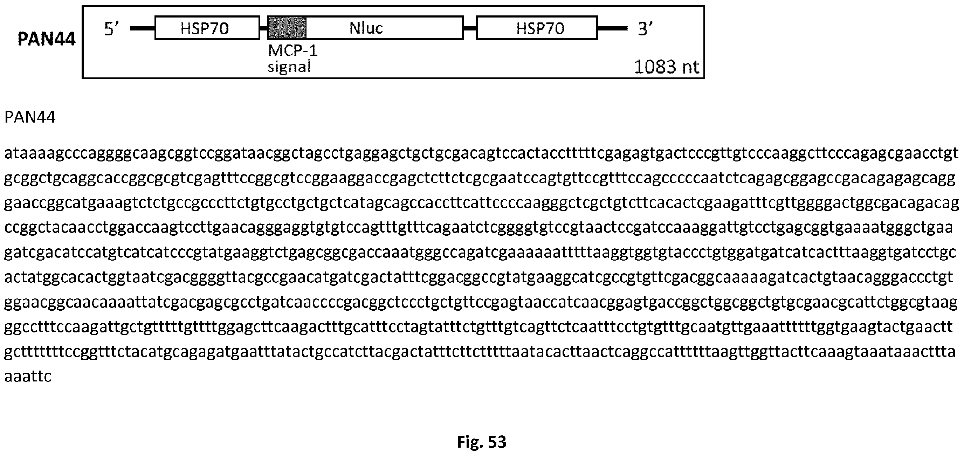

Fig. 53 shows the basic structure and nucleotide sequence of recombinant nucleic acid construct PAN44.

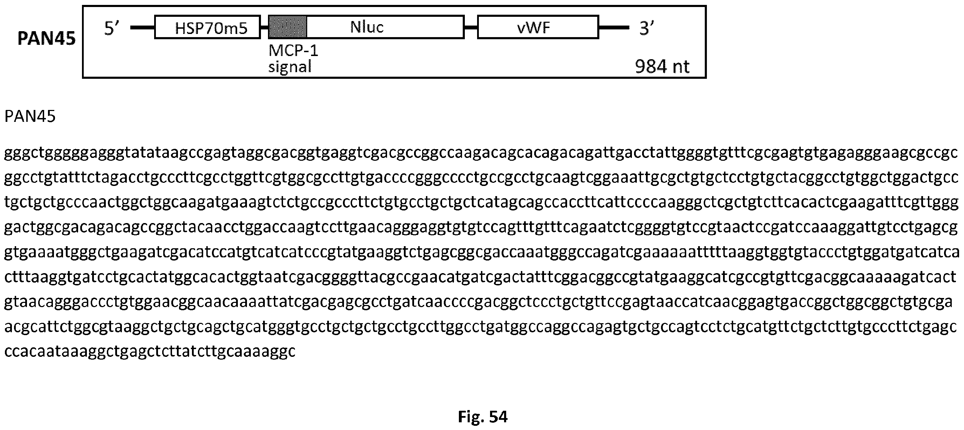

Fig. 54 shows the basic structure and nucleotide sequence of recombinant nucleic acid construct PAN45.

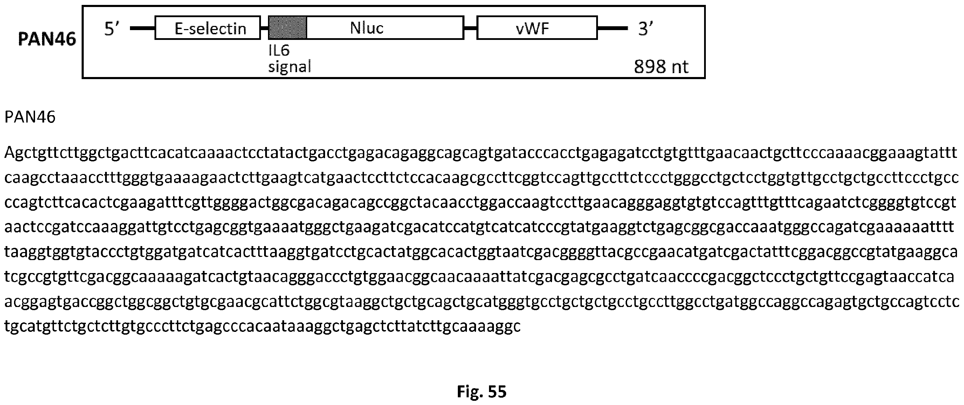

Fig. 55 shows the basic structure and nucleotide sequence of recombinant nucleic acid construct PAN46.

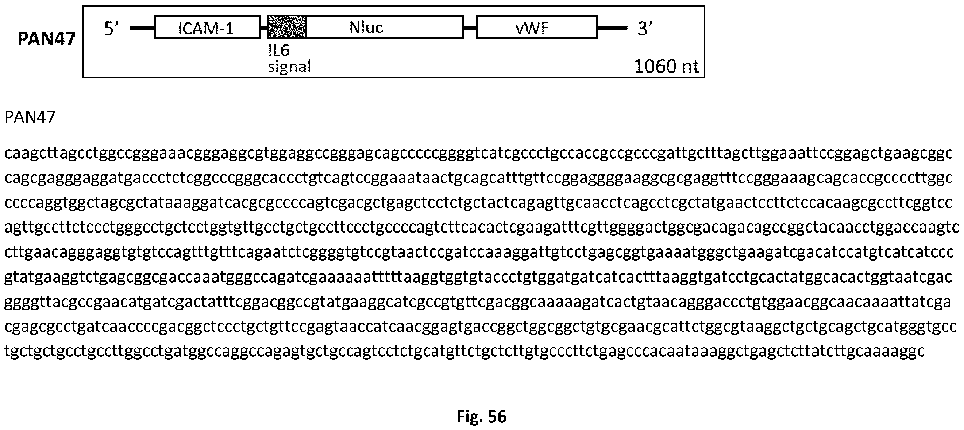

Fig. 56 shows the basic structure and nucleotide sequence of recombinant nucleic acid construct PAN47.

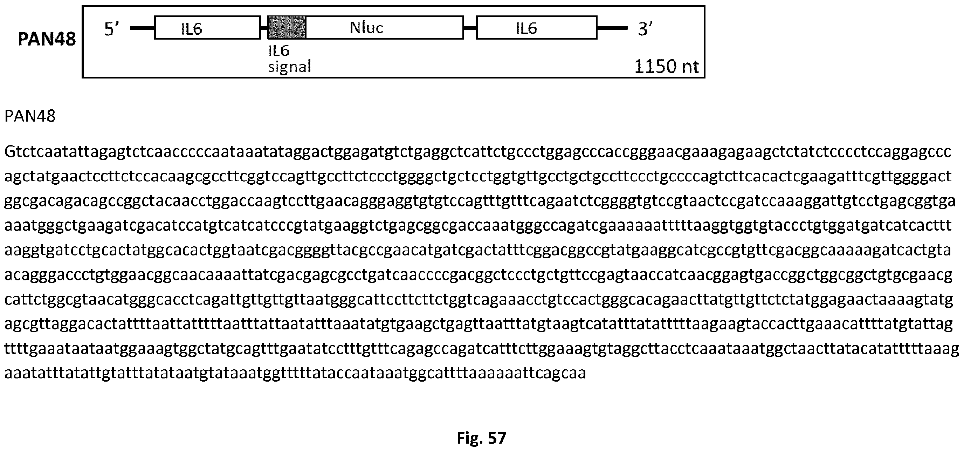

Fig. 57 shows the basic structure and nucleotide sequence of recombinant nucleic acid construct PAN48.

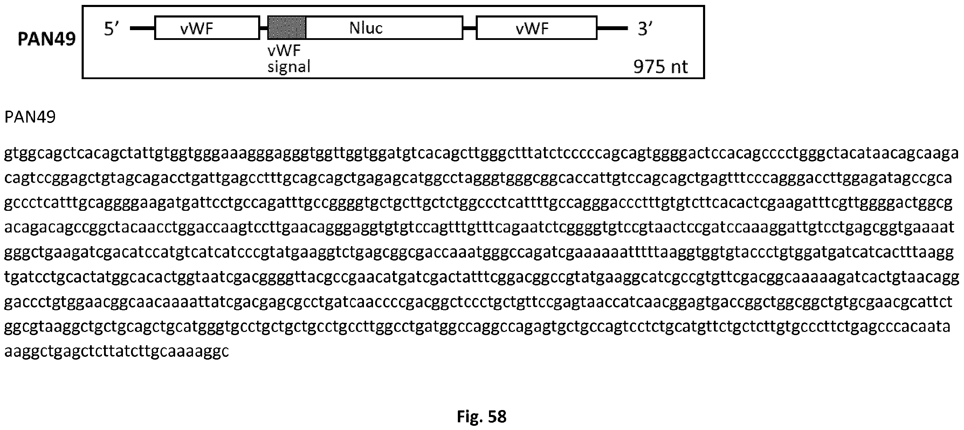

Fig. 58 shows the basic structure and nucleotide sequence of recombinant nucleic acid construct PAN49.

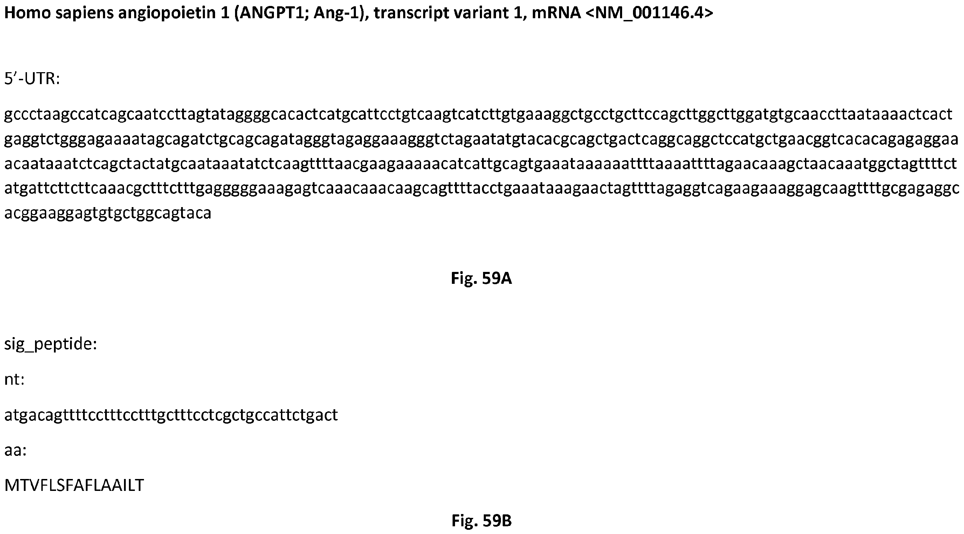

Fig. 59A shows the nucleotide sequence of mRNA of human angiopoietin 1, also referred to as ANGPT1 or Ang-1, transcript variant 1, GenBank entry NM_001146.4.
Fig. 59B shows both the nucleotide sequence and the amino acid sequence of the mRNA of human Ang-1.

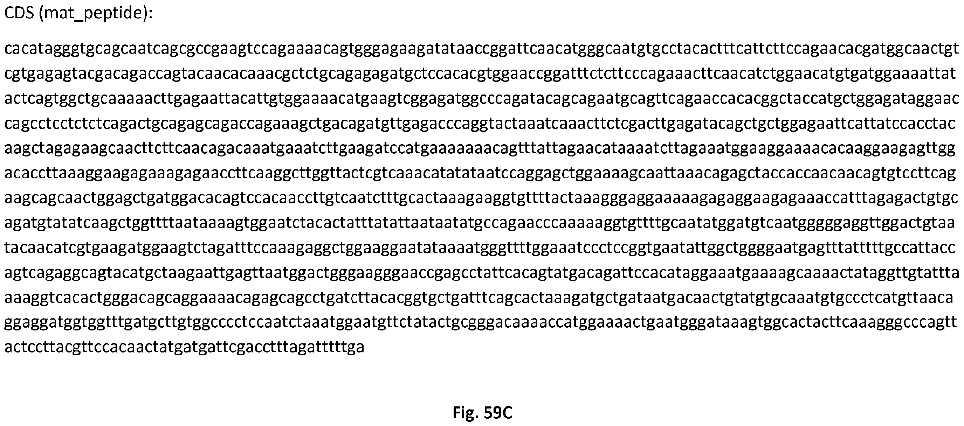

Fig. 59C shows the coding sequence (CDS) of the mature peptide of mRNA of human Ang-1.

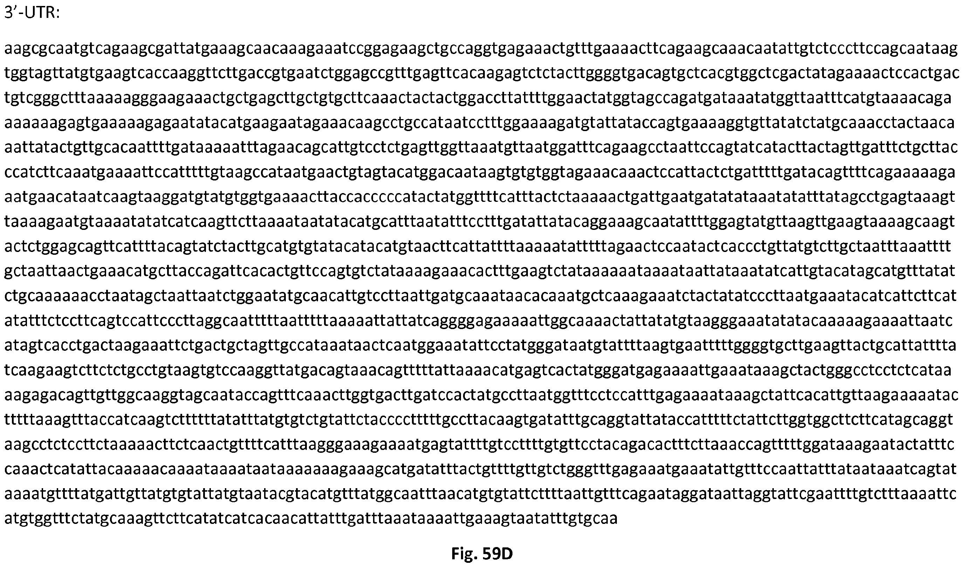

Fig. 59D shows the 3' UTR nucleotide sequence of the mRNA of human Ang-1.

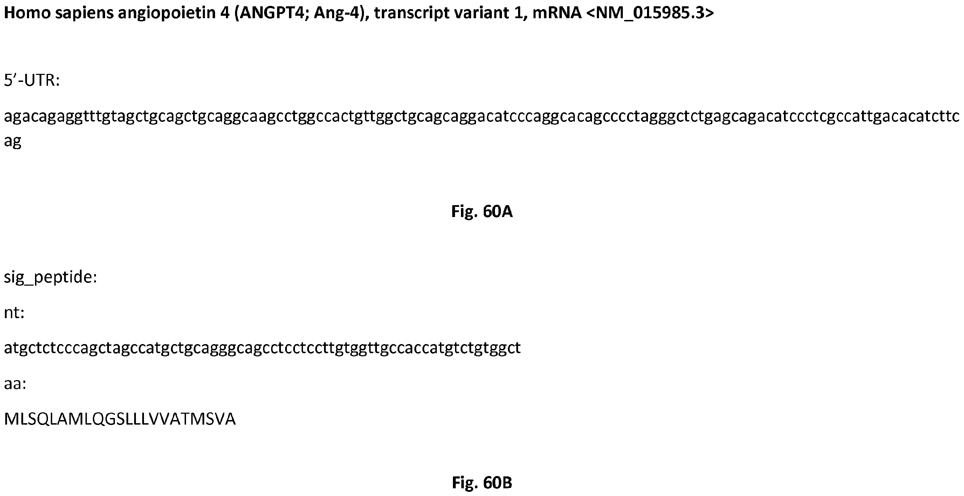

Fig. 60A shows the nucleotide sequence of mRNA of human angiopoietin 4, also referred to as ANGPT4 or Ang-4, transcript variant 1, GenBank entry NM_015985.3.
Fig. 60B shows both the nucleotide sequence and the amino acid sequence of the mRNA of human Ang-4.

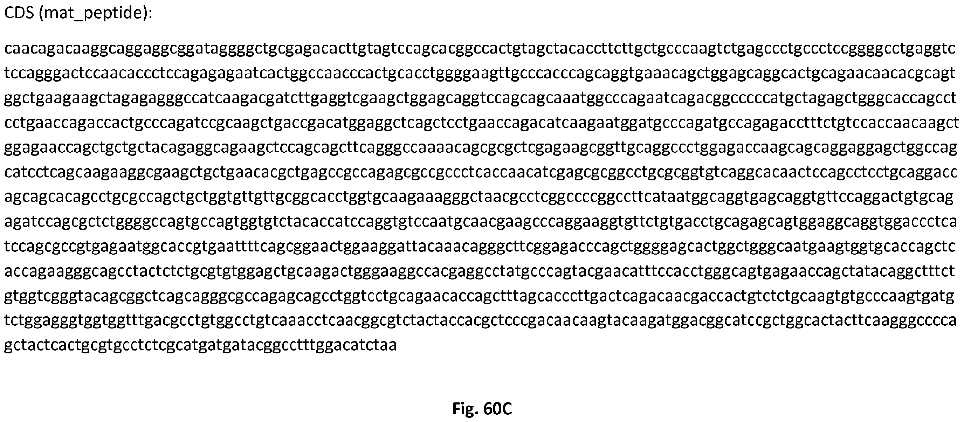

Fig. 60C shows the coding sequence (CDS) of the mature peptide of mRNA of human Ang-4.

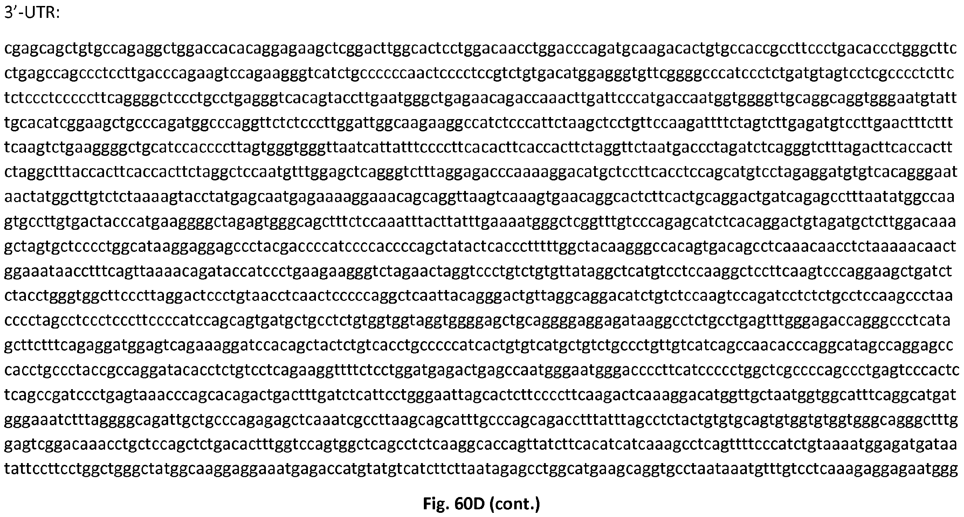

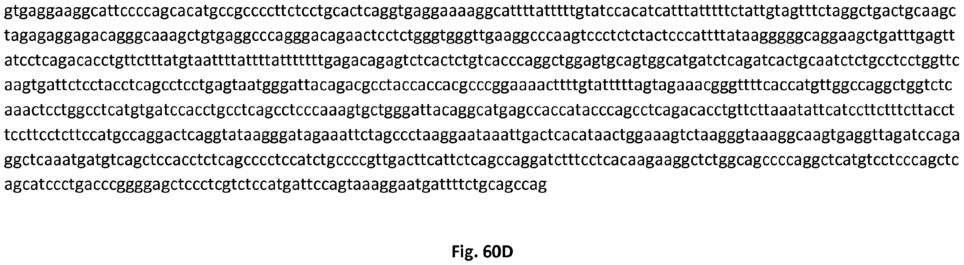

Fig. 60D shows the 3' UTR nucleotide sequence of the mRNA of human Ang-4.

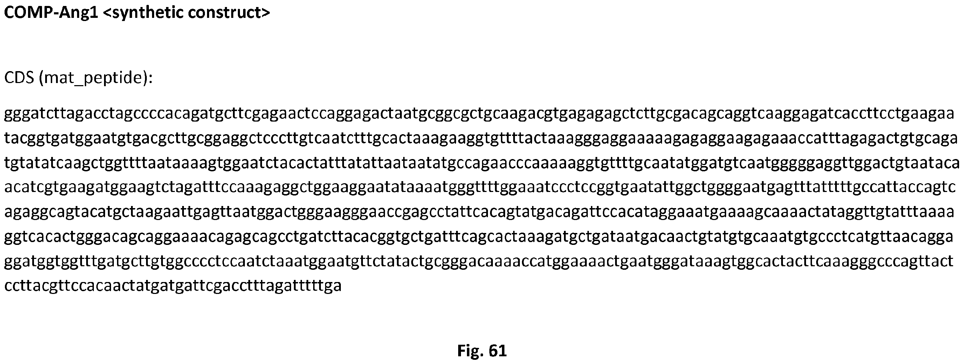

Fig. 61 shows the nucleotide sequence coding for COMP-Ang1 which is a synthetic construct.

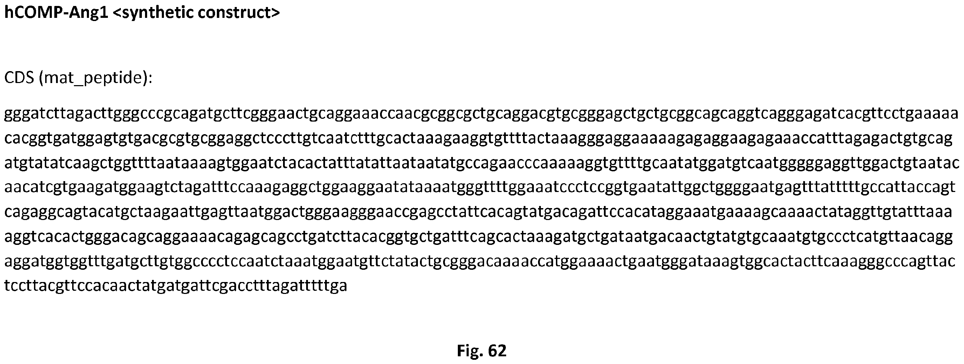

Fig. 62 shows the nucleotide sequence coding for hCOMP-Ang1 which is a synthetic construct.

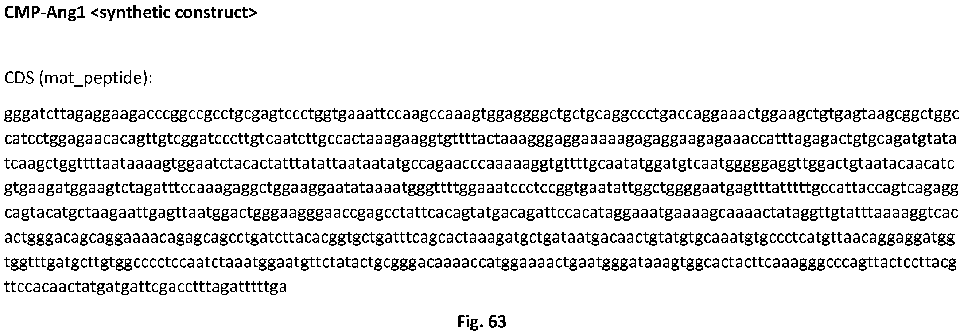

Fig. 63 shows the nucleotide sequence coding for CMP-Ang1 which is a synthetic construct.

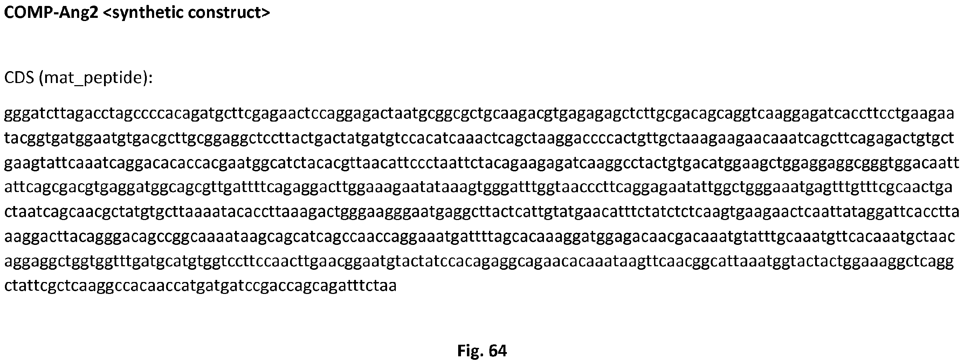

Fig. 64 shows the nucleotide sequence coding for COMP-Ang2 which is a synthetic construct.

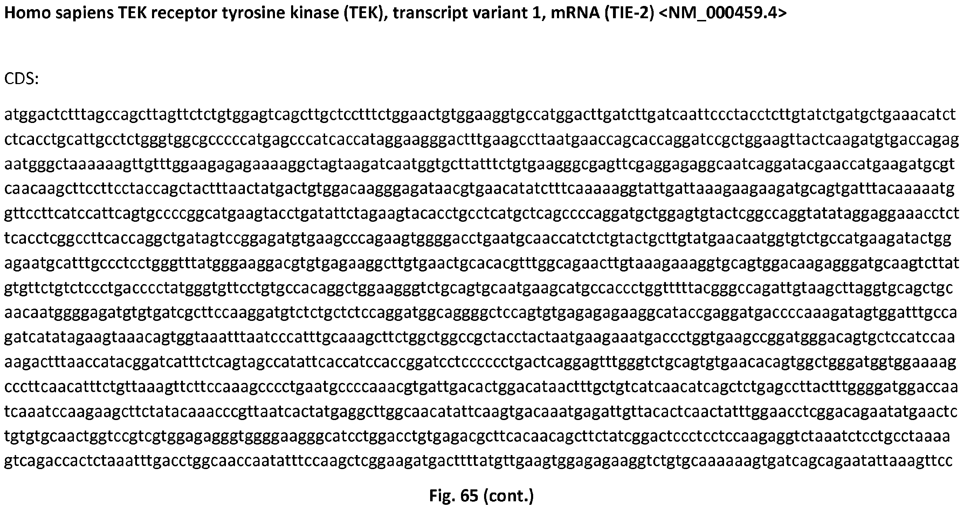

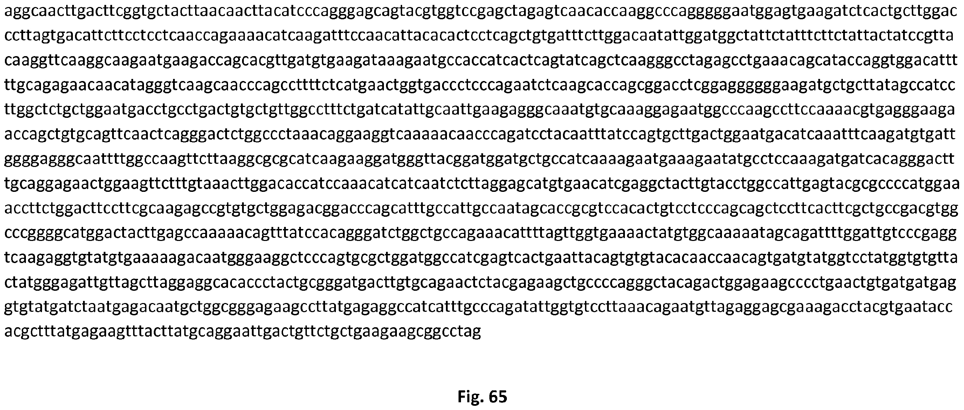

Fig. 65 shows the coding nucleotide sequence of mRNA of human TEK receptor tyrosine kinase (TEK), transcript variant 1, also referred to as Tie-2, GenBank entry.

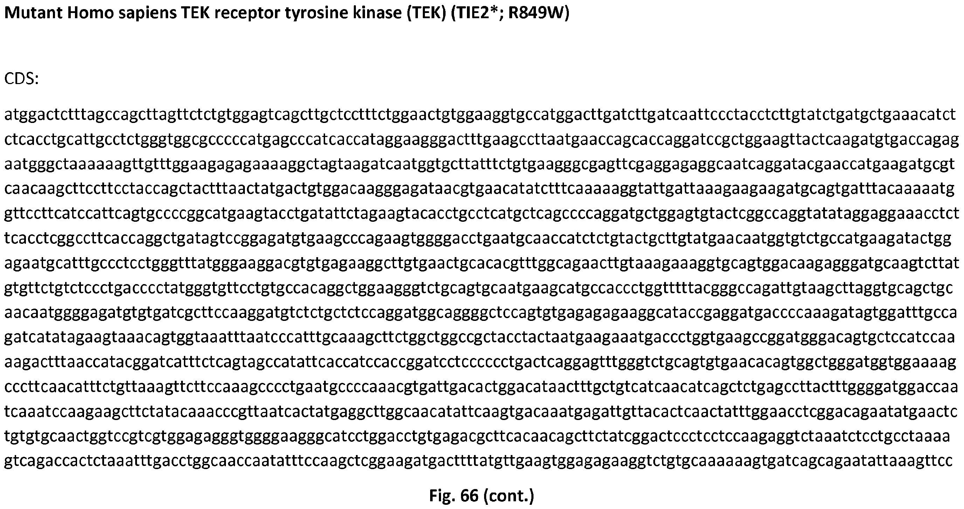

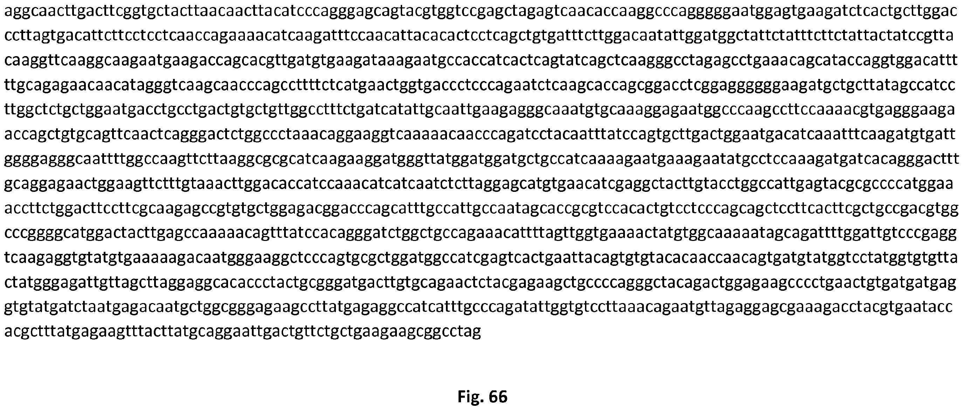

Fig. 66 shows the coding nucleotide sequence of mRNA of human mutant TEK receptor tyrosine kinase (TEK), which is also referred to as TIE* having a R849W mutation.
Fig. 67 shows the basic structure and nucleotide sequence of recombinant nucleic acid construct PAN50.

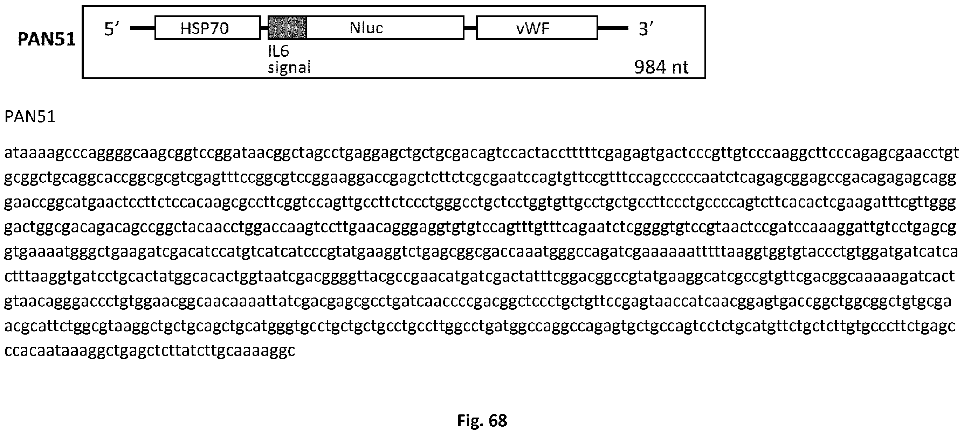

Fig. 68 shows the basic structure and nucleotide sequence of recombinant nucleic acid construct PAN51.

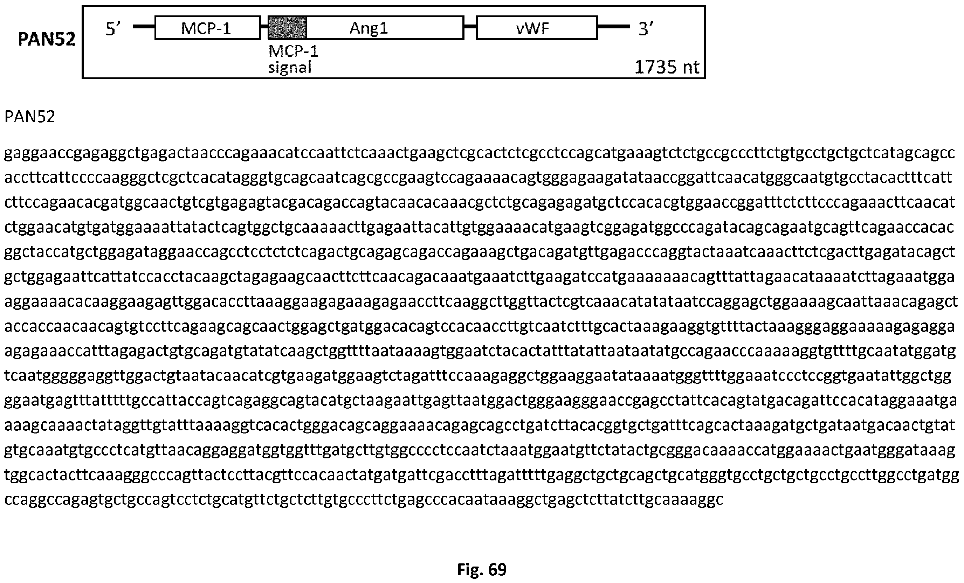

Fig. 69 shows the basic structure and nucleotide sequence of recombinant nucleic acid construct PAN52.

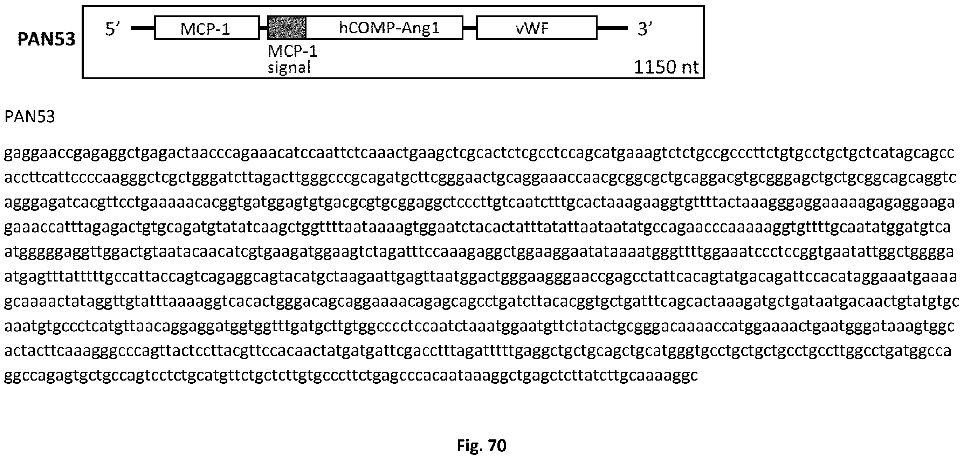

Fig. 70 shows the basic structure and nucleotide sequence of recombinant nucleic acid construct PAN53.

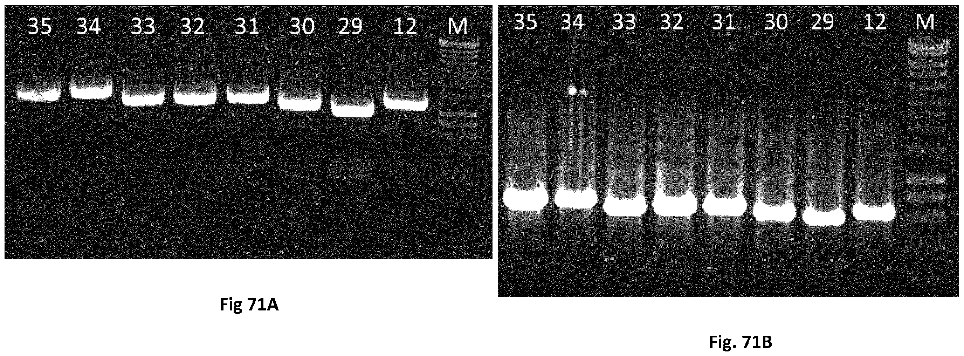

Fig. 71A is an image of a non-denaturing agarose gel after EtBr staining showing the tail-PCR products of various construct.
Fig. 71B is an image of a non-denaturing agarose gel after EtBr staining showing the *in vitro* mRNA transcripts of various construct.

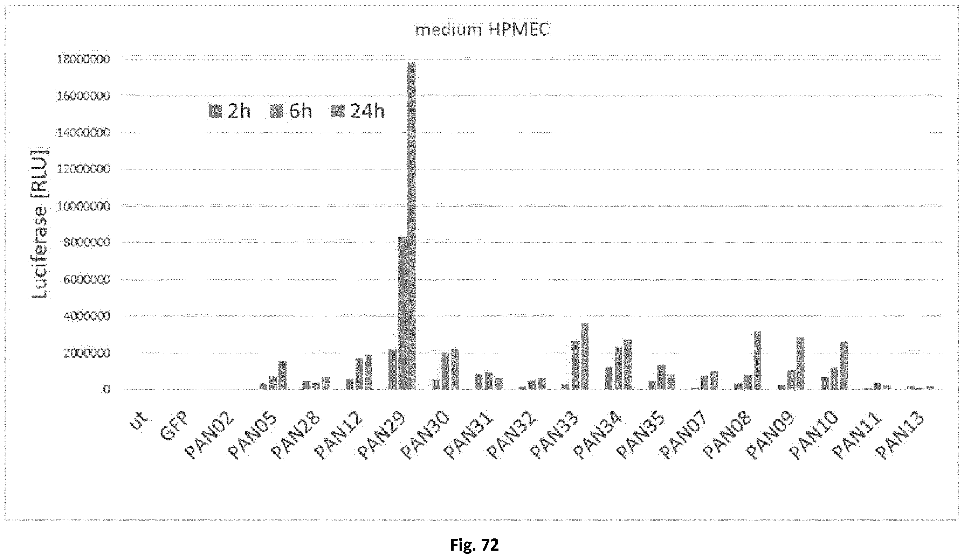

Fig. 72 is a bar diagram showing expression of luciferase, indicated as RLU (relative light units) of luciferase in medium, by the indicated recombinant nucleic acid constructs in HPMEC where luciferase is used as the effector molecule after 2 hours (left column), 6 hours (middle column) and 24 hours (right column) post transfection.

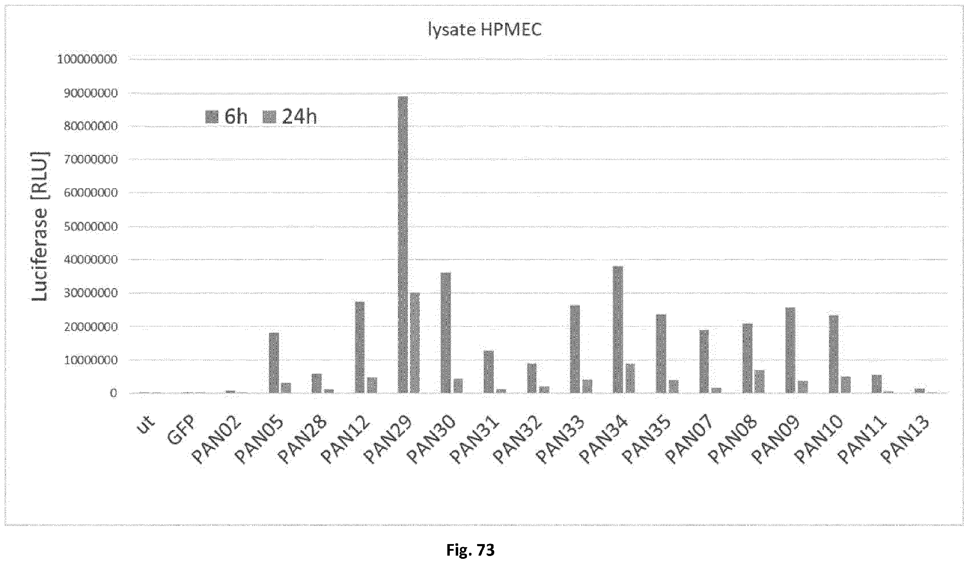

Fig. 73 is a bar diagram showing expression of luciferase, indicated as RLU (relative light units) of luciferase in whole cell lysates of HPMEC, by the indicated recombinant nucleic acid constructs in HPMEC, where luciferase is used as the effector molecule after 6 hours (left column) and 24 hours (right column) post transfection.

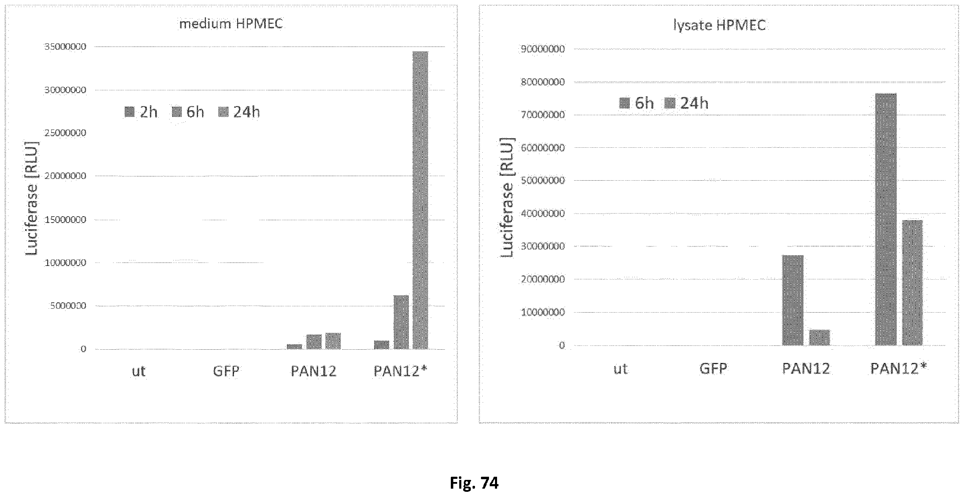

Fig. 74A (left) is a bar diagram showing expression of luciferase, indicated as RLU (relative light units) of luciferase in medium, by the indicated recombinant nucleic acid constructs in HPMEC (transfected with GFP mRNA, PAN12 mRNA and PAN12* mRNA) where luciferase is used as the effector molecule after 2 hours (left column), 6 hours (middle column) and 24 hours (right column) post transfection. In PAN12* 100% of uridine and 100% cytidine nucleotides are replaced by pseudo-uridine and 5-methyl-cytidine, respectively.
Fig. 74B (right) is a bar diagram showing expression of luciferase, indicated as RLU (relative light units) of luciferase in cell lysates, by the indicated recombinant nucleic acid constructs in HPMEC (transfected with GFP mRNA, PAN12 mRNA and PAN12* mRNA) where luciferase is used as the effector molecule after 6 hours (left column) and 24 hours (right column) post transfection. In PAN12* 100% of uridine and 100% cytidine nucleotides are replaced by pseudo-uridine and 5-methyl-cytidine, respectively.

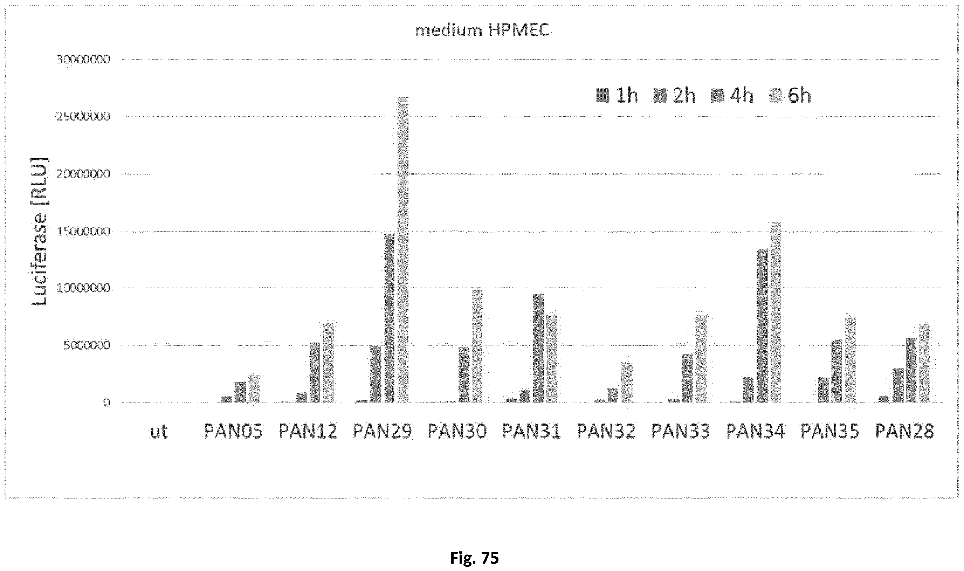

Fig. 75 is a bar diagram showing expression of luciferase, indicated as RLU (relative light units) of luciferase in medium, by the indicated recombinant nucleic acid constructs in HPMEC where luciferase is used as the effector molecule after 1 hour, 2 hours, 4 hours and 24 hours post transfection (from left to right, with 1 hour results being shown to the utmost left side and 24 hours result being shown to the utmost right side).

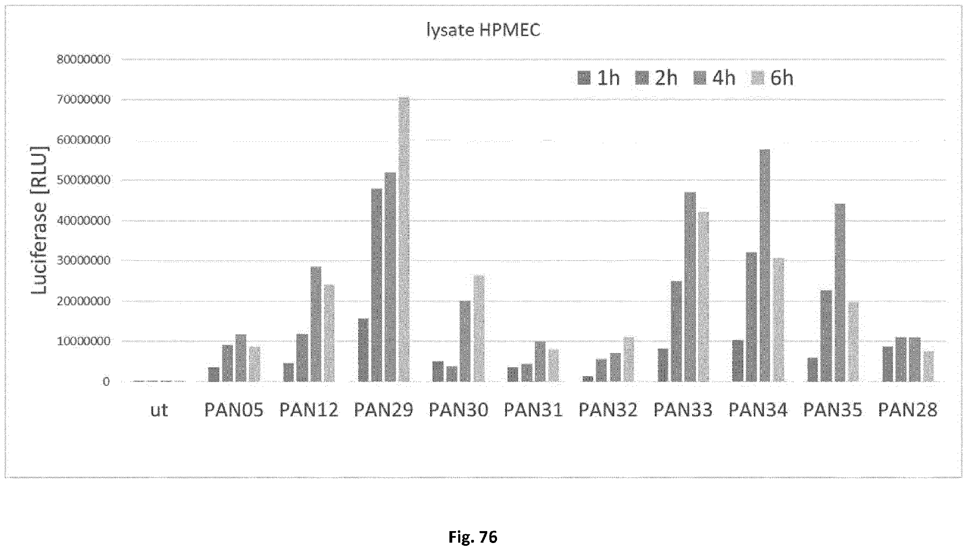

Fig. 76 is a bar diagram showing expression of luciferase, indicated as RLU (relative light units) of luciferase in whole cell lysates of HPMEC, by the indicated recombinant nucleic acid constructs in HPMEC, where luciferase is used as the effector molecule after 1 hour, 2 hours, 4 hours and 24 hours post transfection (from left to right, with 1 hour results being shown to the utmost left side and 24 hours result being shown to the utmost right side).

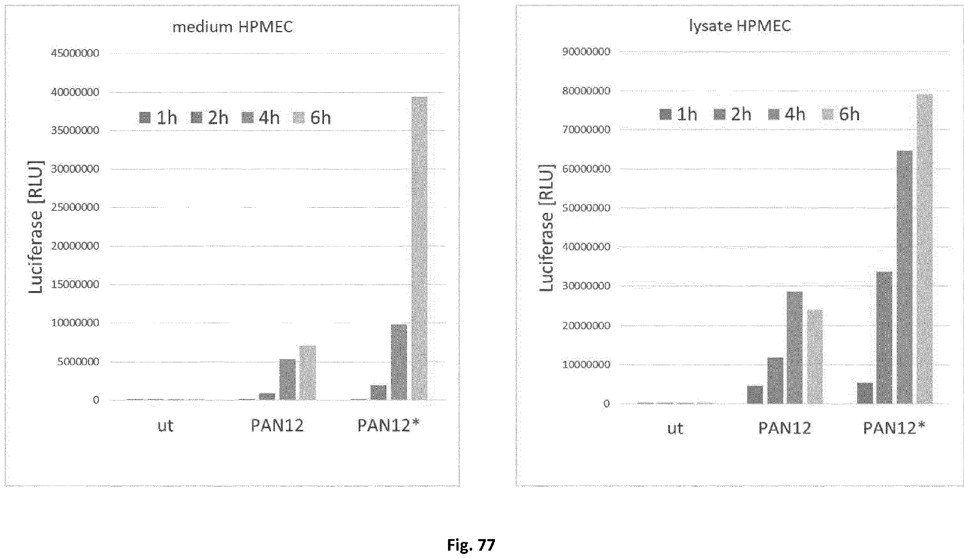

Fig. 77A (left) is a bar diagram showing expression of luciferase, indicated as RLU (relative light units) of luciferase in medium, by the indicated recombinant nucleic acid constructs in HPMEC (transfected with GFP mRNA, PAN12 mRNA and PAN12* mRNA) where luciferase is used as the effector molecule after 1 hour, 2 hours, 4 hours and 24 hours post transfection (from left to right, with 1 hour results being shown to the utmost left side and 24 hours result being shown to the utmost right side). In PAN12* 100% of uridine and 100% cytidine nucleotides are replaced by pseudo-uridine and 5-methyl-cytidine, respectively.
Fig. 77B (right) is a bar diagram showing expression of luciferase, indicated as RLU (relative light units) of luciferase in cell lysates, by the indicated recombinant nucleic acid constructs in HPMEC (transfected with GFP mRNA, PAN12 mRNA and PAN12* mRNA) where luciferase is used as the effector molecule after 1 hour, 2 hours, 4 hours and 24 hours post transfection (from left to right, with 1 hour results being shown to the utmost left side and 24 hours result being shown to the utmost right side). In PAN12* 100% of uridine and 100% cytidine nucleotides are replaced by pseudo-uridine and 5-methyl-cytidine, respectively.

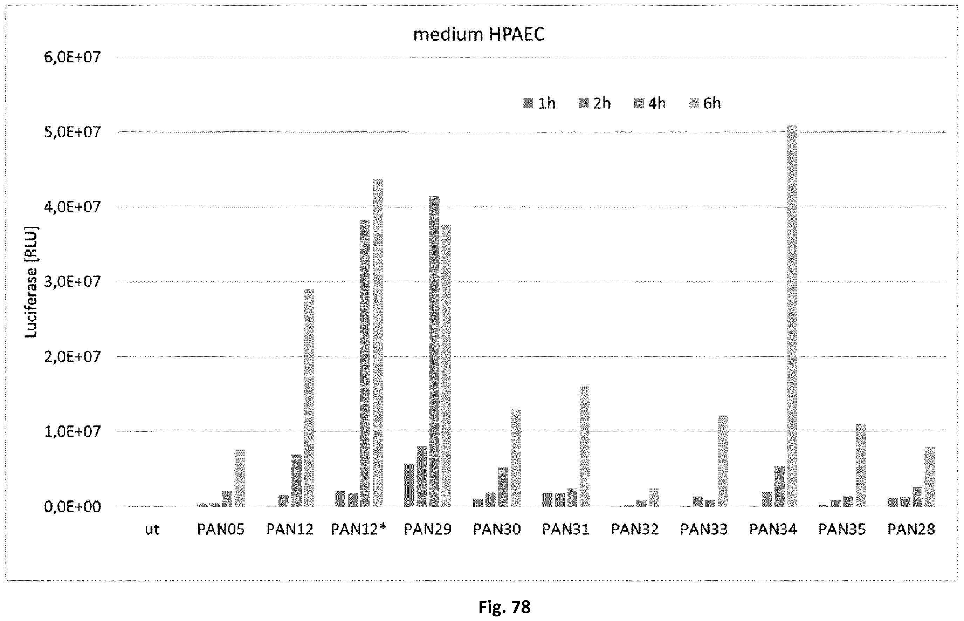

Fig. 78 is a bar diagram showing expression of luciferase, indicated as RLU (relative light units) of luciferase in medium, by the indicated recombinant nucleic acid constructs in HPAEC where luciferase is used as the effector molecule after 1 hour, 2 hours, 4 hours and 24 hours post transfection (from left to right, with 1 hour results being shown to the utmost left side and 24 hours result being shown to the utmost right side).

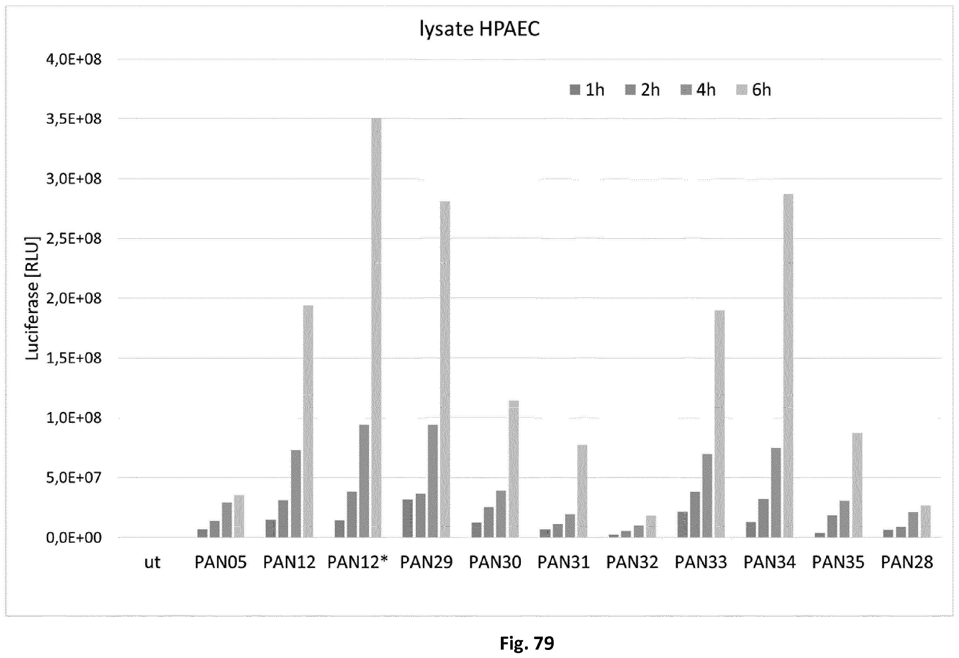

Fig. 79 is a bar diagram showing expression of luciferase, indicated as RLU (relative light units) of luciferase in whole cell lysates of HPAEC, by the indicated recombinant nucleic acid constructs in HPAEC, where luciferase is used as the effector molecule after 1 hour, 2 hours, 4 hours and 24 hours post transfection (from left to right, with 1 hour results being shown to the utmost left side and 24 hours result being shown to the utmost right side).

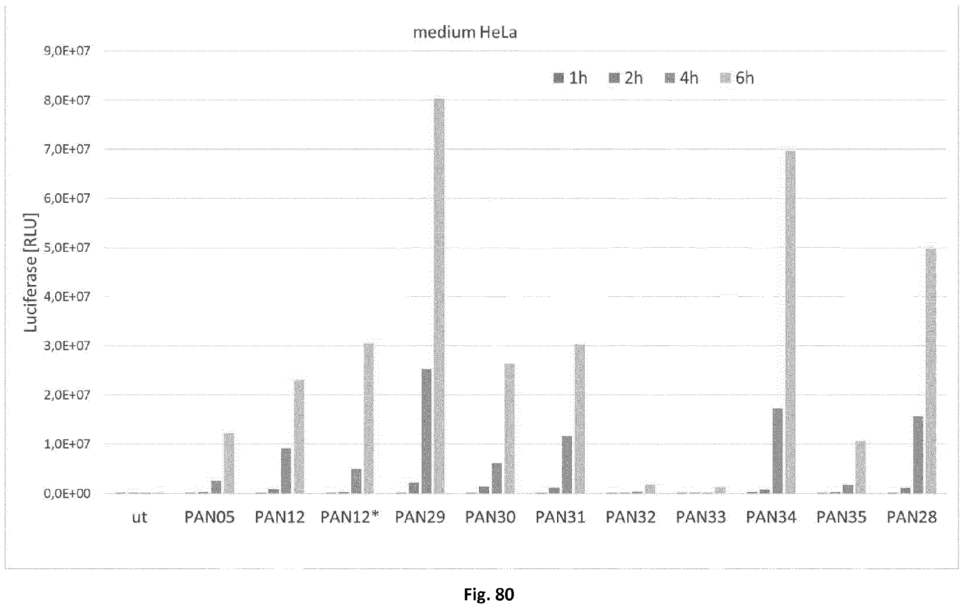

Fig. 80 is a bar diagram showing expression of luciferase, indicated as RLU (relative light units) of luciferase in medium, by the indicated recombinant nucleic acid constructs in HeLa cells where luciferase is used as the effector molecule after 1 hour, 2 hours, 4 hours and 24 hours post transfection (from left to right, with 1 hour results being shown to the utmost left side and 24 hours result being shown to the utmost right side).

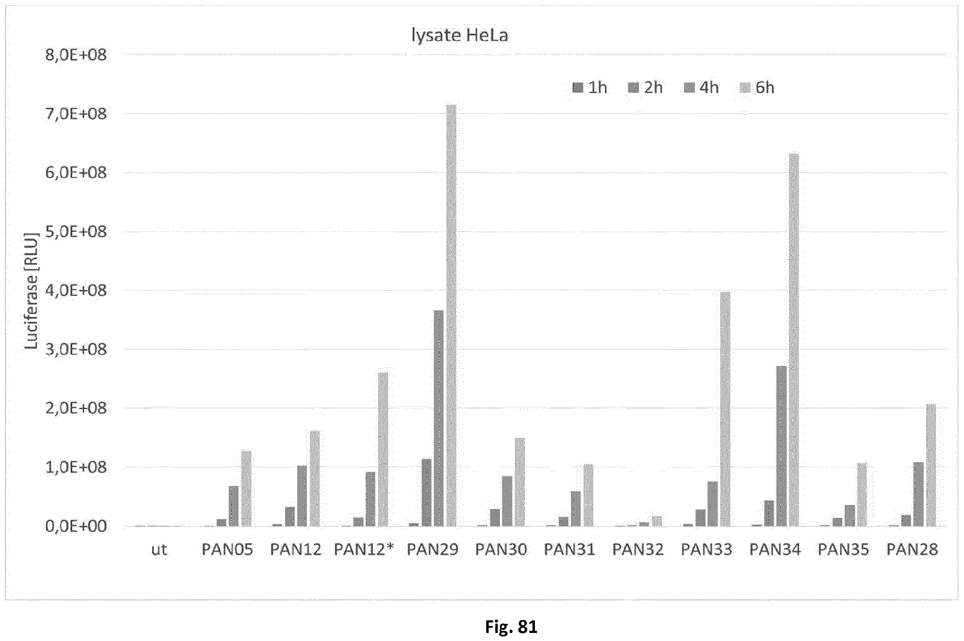

Fig. 81 is a bar diagram showing expression of luciferase, indicated as RLU (relative light units) of luciferase in whole cell lysates of HeLa cells, by the indicated recombinant nucleic acid constructs in HeLA cells, where luciferase is used as the effector molecule after 1 hour, 2 hours, 4 hours and 24 hours post transfection (from left to right, with 1 hour results being shown to the utmost left side and 24 hours result being shown to the utmost right side).

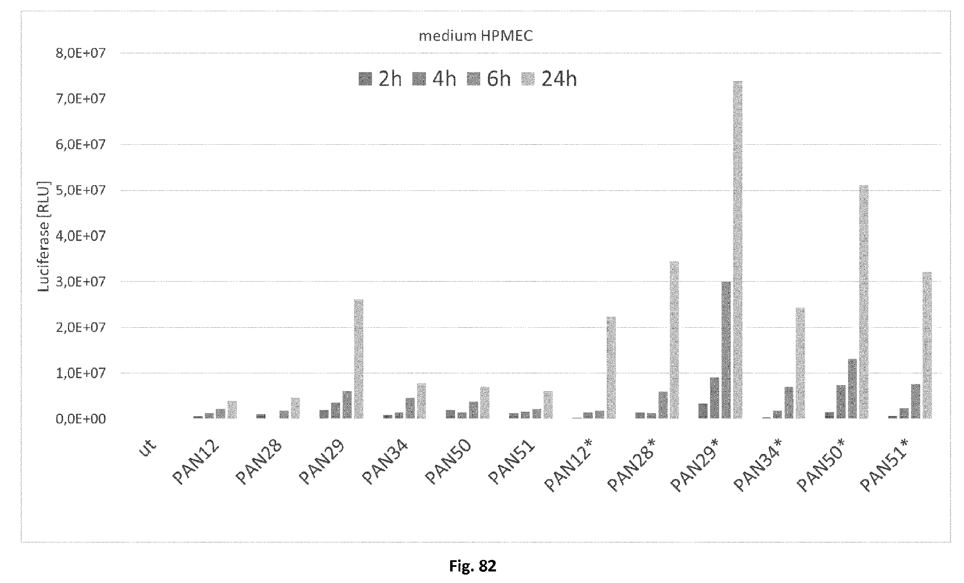

Fig. 82 is a bar diagram showing expression of luciferase, indicated as RLU (relative light units) of luciferase in medium, by the indicated recombinant nucleic acid constructs in HPMEC where luciferase is used as the effector molecule after 2 hours, 4 hours, 6 hours and 24 hours post transfection (from left to right, with 1 hour results being shown to the utmost left side and 24 hours result being shown to the utmost right side). In constructs PAN12*, PAN28*, PAN29*, PAN34*, PAN50* and PAN51*100% of uridine and 100% cytidine nucleotides are replaced by pseudo-uridine and 5-methyl-cytidine, respectively.

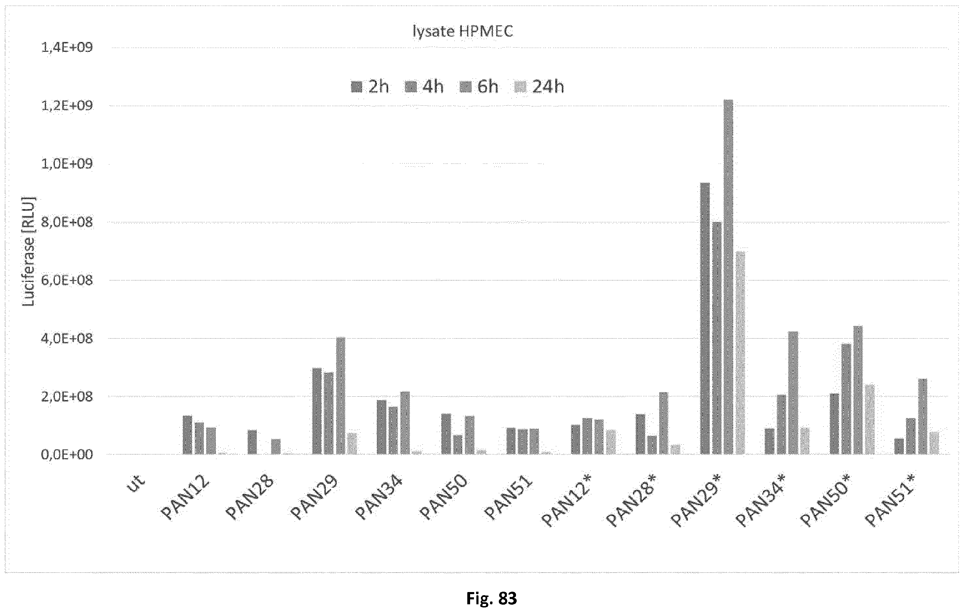

Fig. 83 is a bar diagram showing expression of luciferase, indicated as RLU (relative light units) of luciferase in whole cell lysates of HPMEC, by the indicated recombinant nucleic acid constructs in HPMEC, where luciferase is used as the effector molecule after 2 hours, 4 hours, 6 hours and 24 hours post transfection (from left to right, with 1 hour results being shown to the utmost left side and 24 hours result being shown to the utmost right side). In constructs PAN12*, PAN28*, PAN29*, PAN34*, PAN50* and PAN51*100% of uridine and 100% cytidine nucleotides are replaced by pseudo-uridine and 5-methyl-cytidine, respectively.

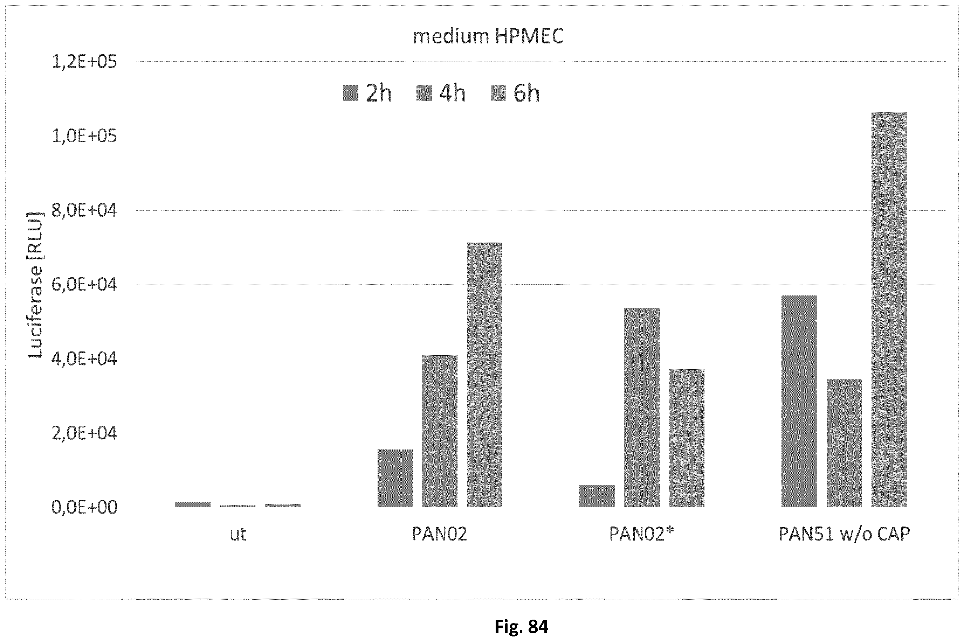

Fig. 84 is a bar diagram showing expression of luciferase, indicated as RLU (relative light units) of luciferase in medium, by the indicated recombinant nucleic acid constructs in HPMEC where luciferase is used as the effector molecule after 2 hours (left column), 4 hours (middle column) and 6 hours (right column) post transfection. In construct PAN02*, 100% of uridine and 100% cytidine nucleotides are replaced by pseudo-uridine and 5-methyl-cytidine, respectively; and in construct PAN51 w/o CAP the mRNA sequence is the one of construct PAN51, but without any 5'-capping structure.

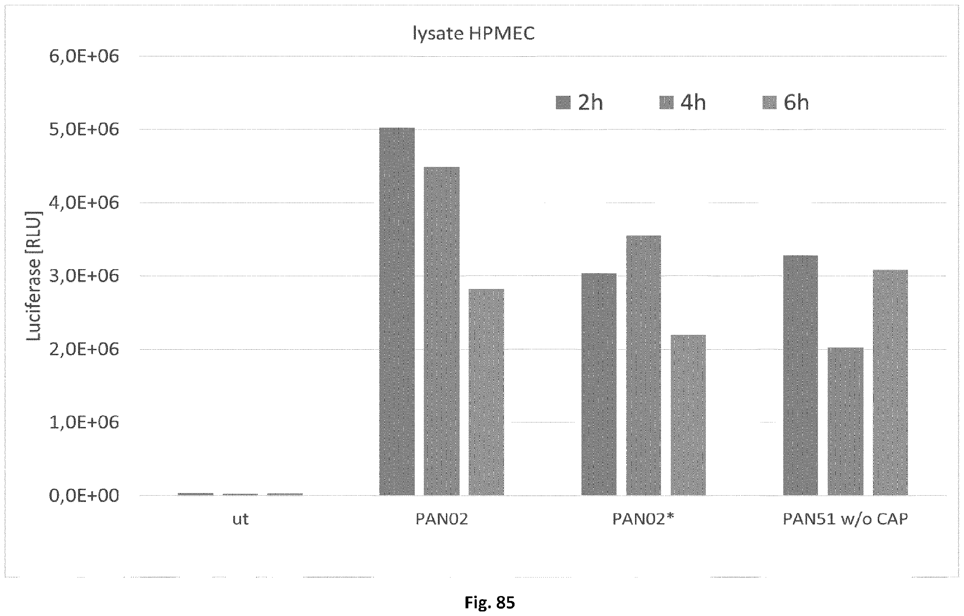

Fig. 85 is a bar diagram showing expression of luciferase, indicated as RLU (relative light units) of luciferase in whole cell lysates of HPMEC, by the indicated recombinant nucleic acid constructs in HPMEC, where luciferase is used as the effector molecule after 2 hours (left column) 4 hours (middle column) and 6 hours (right column) post transfection. In construct PAN02*, 100% of uridine and 100% cytidine nucleotides are replaced by pseudo-uridine and 5-methyl-cytidine, respectively; and in construct PAN51 w/o CAP the mRNA sequence is the one of construct PAN51, but without any 5'-capping structure.

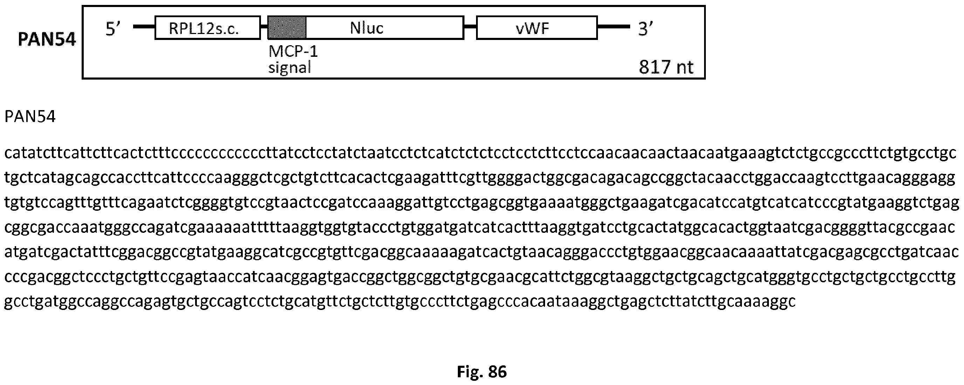

Fig. 86 shows the basic structure and nucleotide sequence of recombinant nucleic acid construct PAN54.

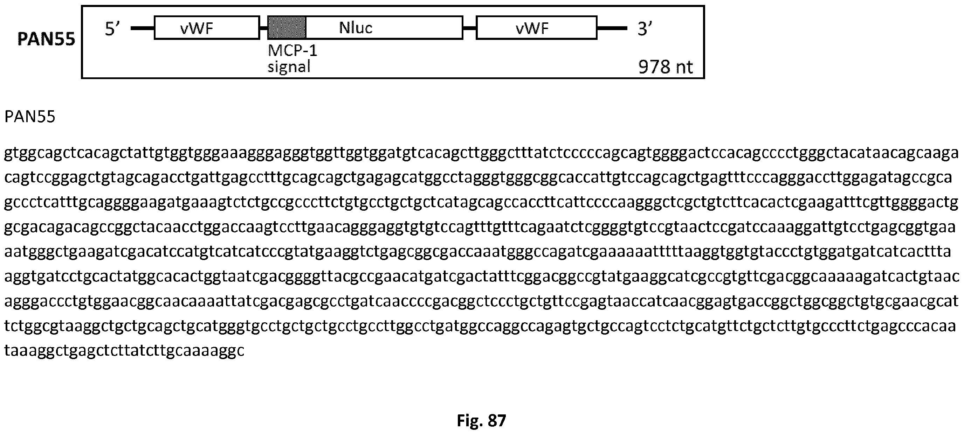

Fig. 87 shows the basic structure and nucleotide sequence of recombinant nucleic acid construct PAN55.

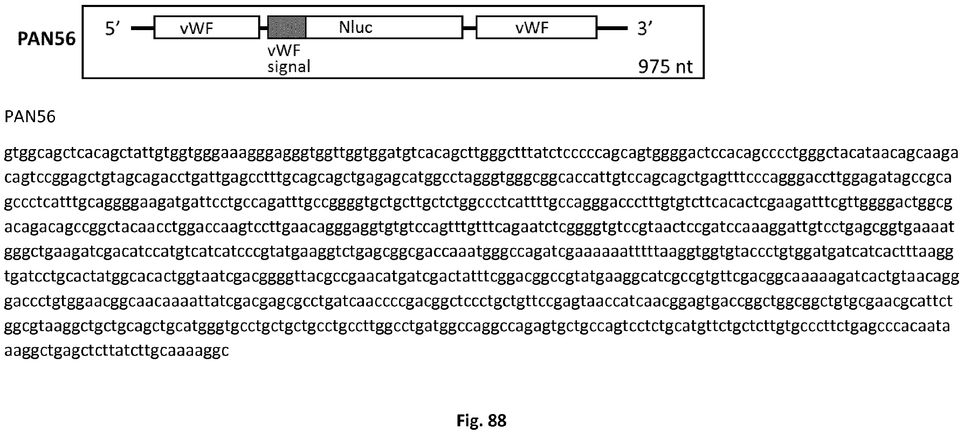

Fig. 88 shows the basic structure and nucleotide sequence of recombinant nucleic acid construct PAN56.

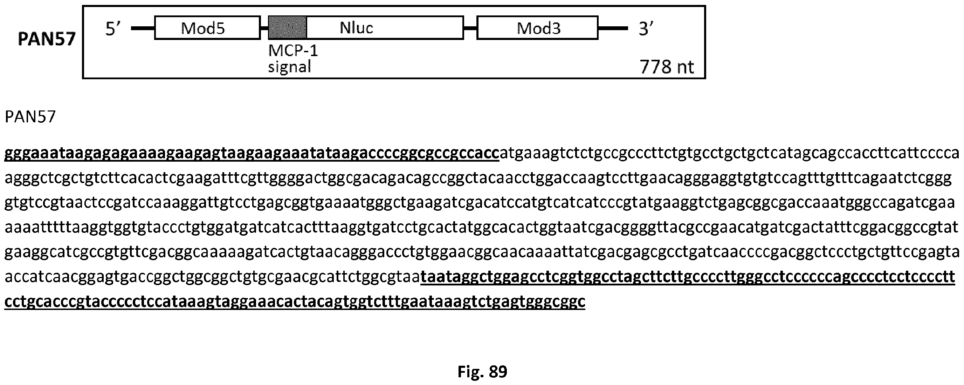

Fig. 89 shows the basic structure and nucleotide sequence of recombinant nucleic acid construct PAN57.
Fig. 90 shows the basic structure and nucleotide sequence of recombinant nucleic acid construct PAN58.

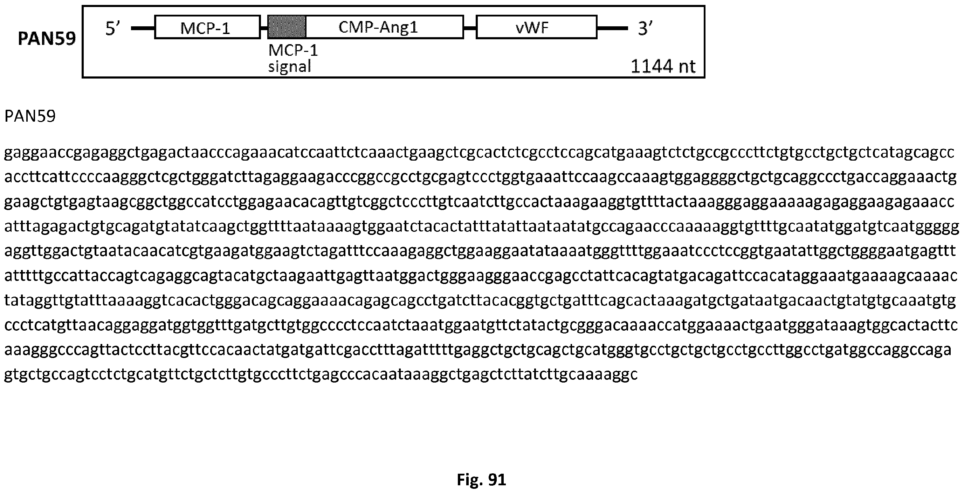

Fig. 91 shows the basic structure and nucleotide sequence of recombinant nucleic acid construct PAN59.

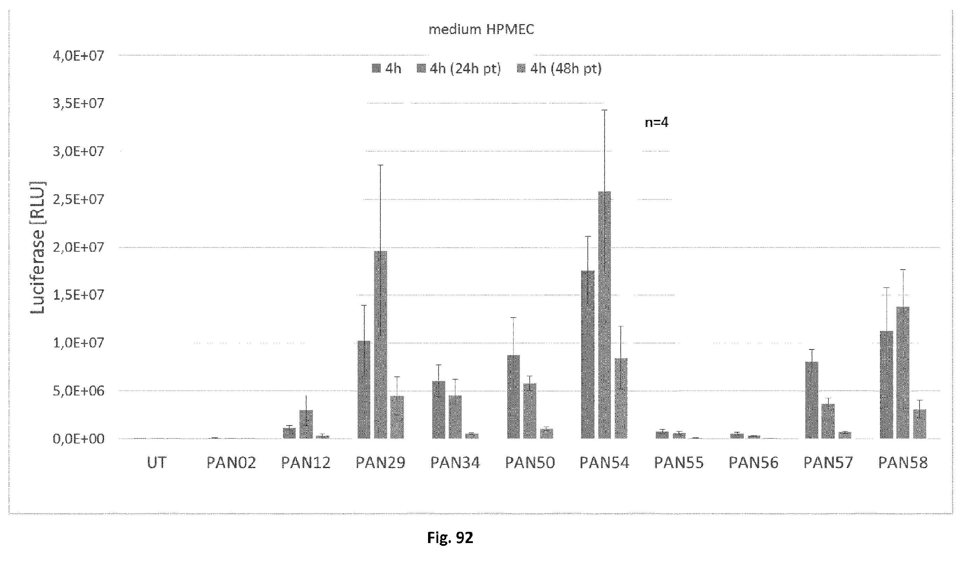

Fig. 92 is a bar diagram showing expression of luciferase, indicated as RLU (relative light units) of luciferase in medium, by the indicated recombinant nucleic acid constructs in HPMEC, where luciferase is used as the effector molecule after 4 hours (left column). Additional 4-hours luciferase activity values are assessed 24 hours (middle column) and 48 hours (right column) post transfection, after washing of the cells and supplementing the cells with fresh medium.

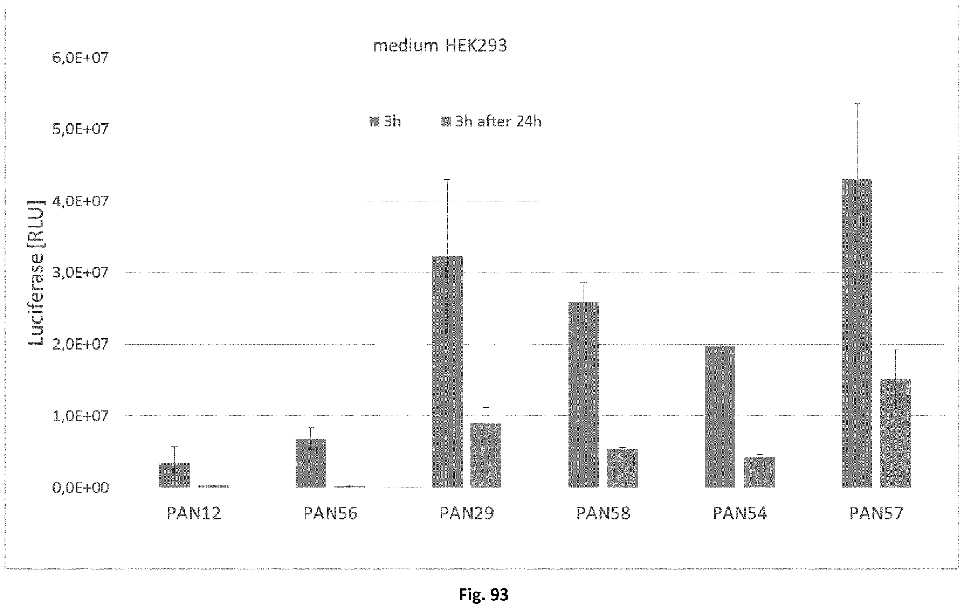

Fig. 93 is a bar diagram showing expression of luciferase, indicated as RLU (relative light units) of luciferase in medium, by the indicated recombinant nucleic acid constructs in HEK293 cells, where luciferase is used as the effector molecule after 3 hours (left column). An additional 3-hours luciferase activity value is assessed 24 hours (right column) post transfection, after washing of the cells and supplementing the cells with fresh medium. The human embryonic kidney 293 cell line (HEK293) is grown in EMEM (EBSS) + 2mM Glutamine + 1% Non Essential Amino Acids (NEAA) + 10% FCS culture medium. Subculture Routine is to split sub-confluent cultures (70-80%) 1:2 to 1:6 i.e. seeding at 2-5x10,000 cells/cm² using 0.25% trypsin or trypsin/EDTA; 5% CO₂; 37°C. mRNA transfection are performed with the Lipofectamine^{™} MessengerMAX^{™} Transfection Reagent (Invitrogen^{™}) according to the manufactures protocol.

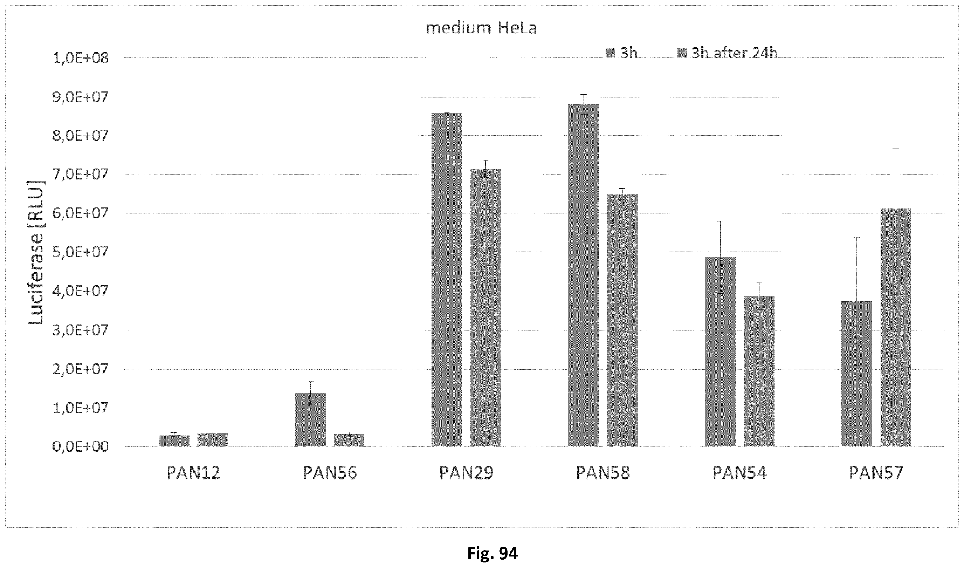

Fig. 94 is a bar diagram showing expression of luciferase, indicated as RLU (relative light units) of luciferase in medium, by the indicated recombinant nucleic acid constructs in HeLa cells, where luciferase is used as the effector molecule after 3 hours (left column). An additional 3-hours luciferase activity value is assessed 24 hours (right column) post transfection, after washing of the cells and supplementing the cells with fresh medium.

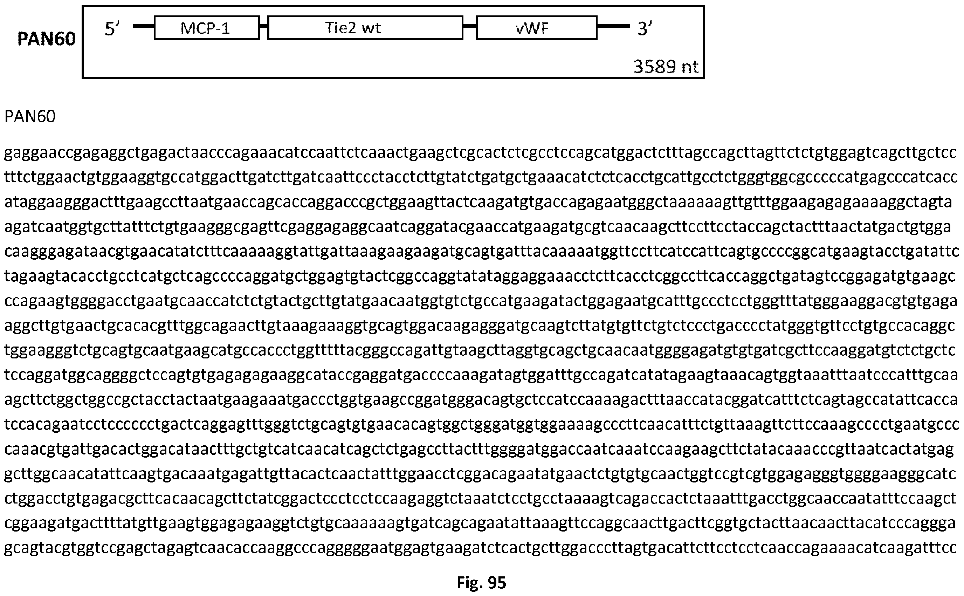

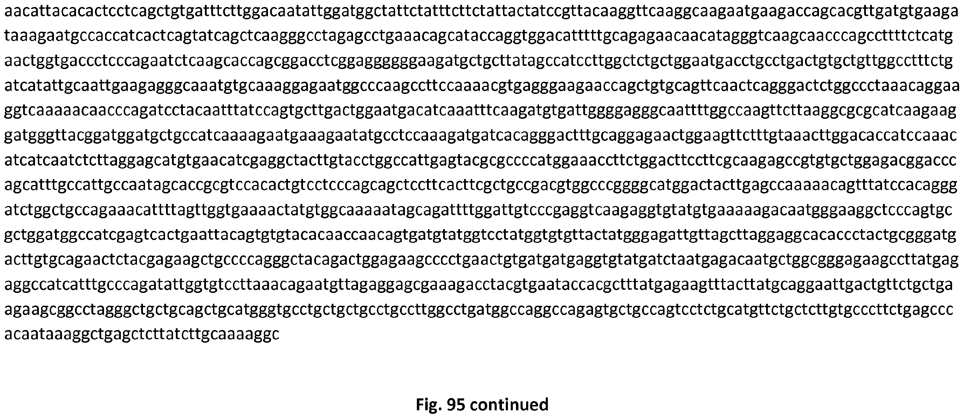

Fig. 95 shows the basic structure and nucleotide sequence of recombinant nucleic acid construct PAN60.

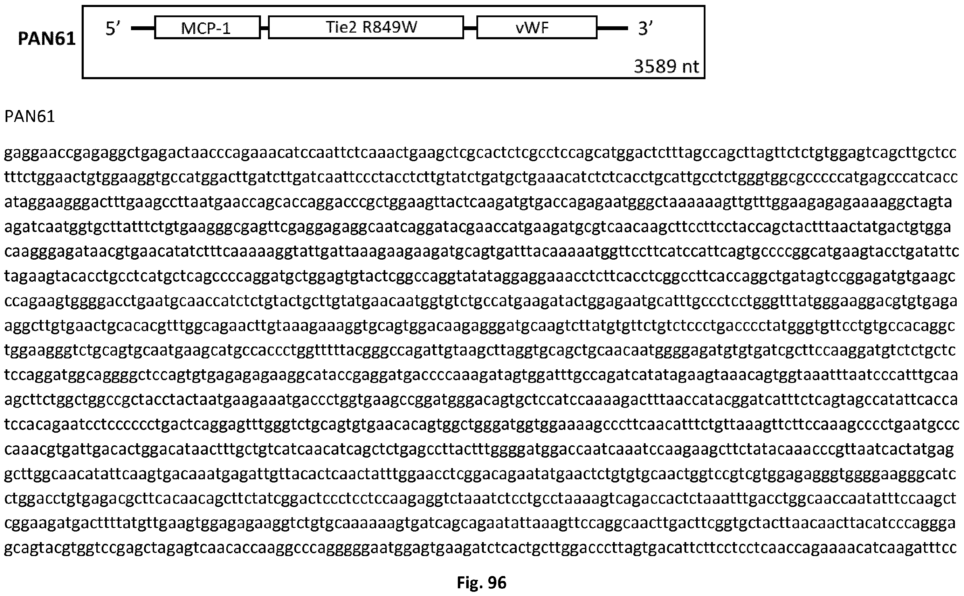

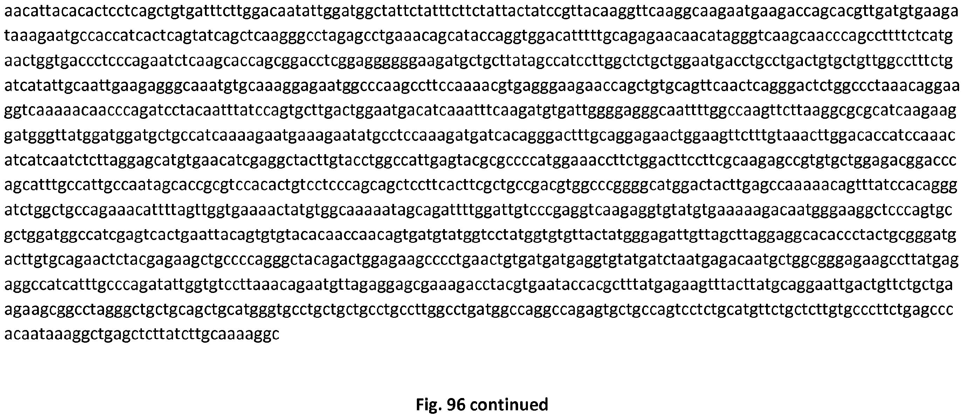

Fig. 96 shows the basic structure and nucleotide sequence of recombinant nucleic acid construct PAN61.

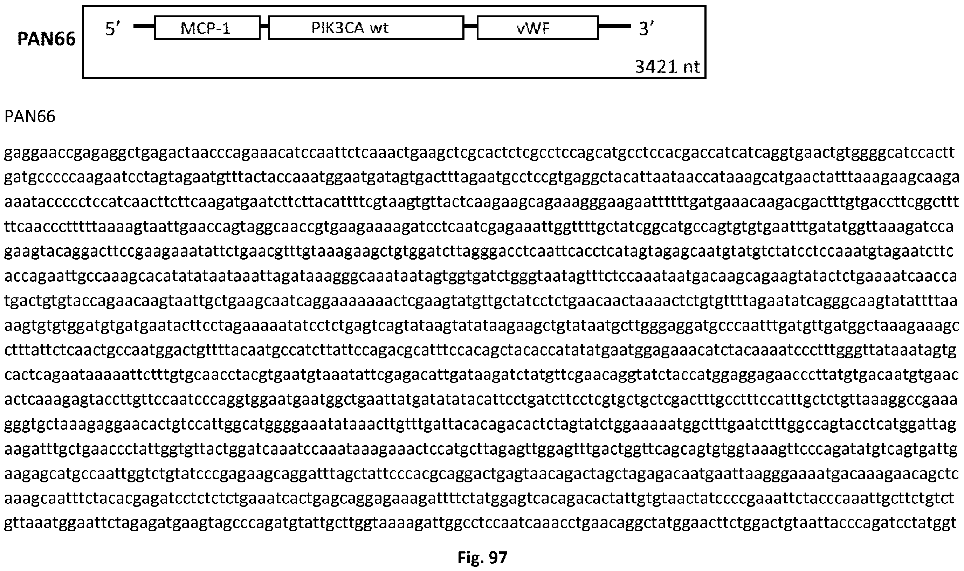

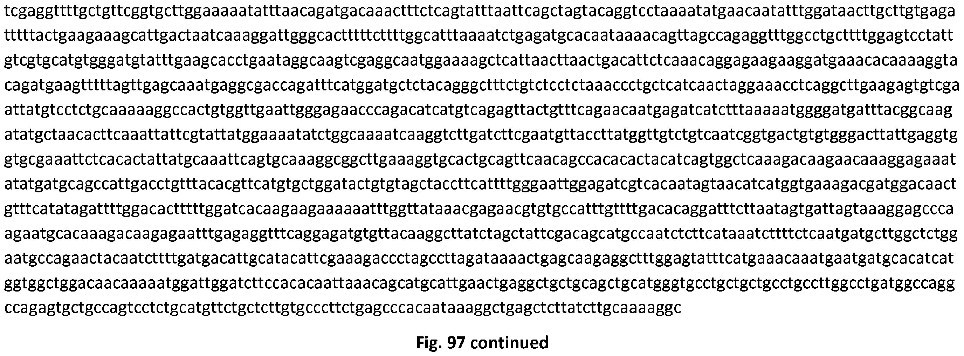

Fig. 97 shows the basic structure and nucleotide sequence of recombinant nucleic acid construct PAN66.

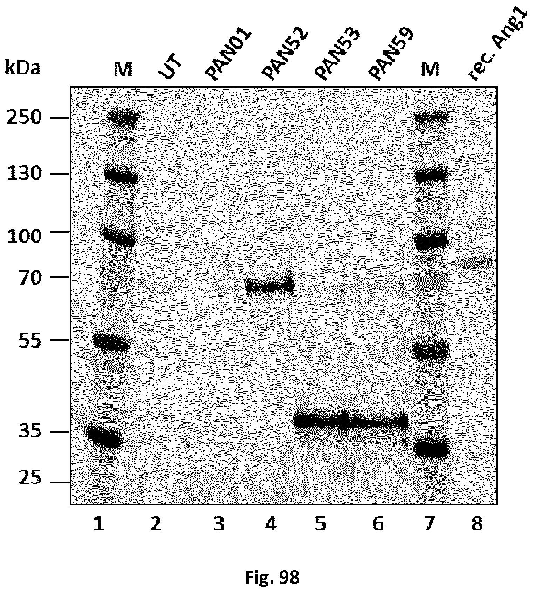

Fig. 98 shows Western Blot analysis of protein lysates from HPMEC cells transfected with indicated PAN mRNA constructs. Cells were transfected with 1µg the mRNAs and harvested after 6 hours. Total cell lysates were separated by SDS-PAGE and analyzed by immunoblot using anti-Ang1 antibody. M, Marker; UT, untreated cells, rec. Ang1, recombinant hAng 1.

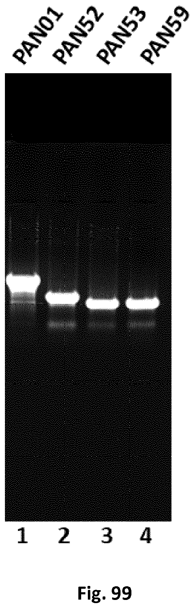

Fig. 99 shows Agarose gels of mRNAs after in vitro transcription. 2 µg of indicated mRNAs were separated by agarose gel electrophoresis and visualized by ethidium bromide staining and UV illumination.

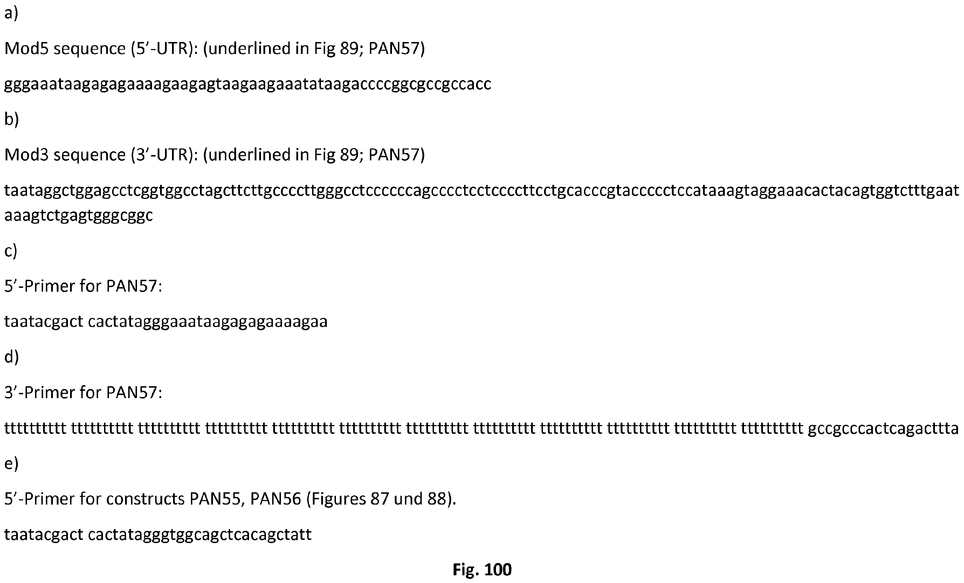

Fig. 100 shows the sequences of: a) the 5'UTR of PAN57; b) the 3'UTR of PAN57; c) the 5'PCR-Primer for PAN57; d) the 3'PCR-Primer for PAN57 and e) the 5'PCR-Primer for PAN55, PAN56.

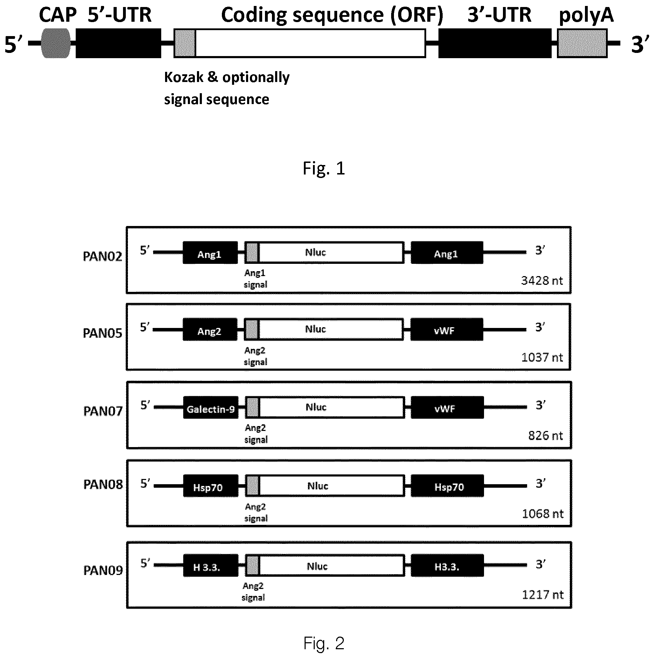

Fig. 1 is a schematic representation of conventional mRNA molecule structure. Eukaryotic including mammalian mRNAs consist of a cap region, a 5' untranslated region (UTR), the coding sequence (CDS) with an open reading frame (ORF) starting with a consensus Kozak sequence for optimal translation initiation and in case of secreted proteins followed by an signal peptide leader sequences, a 3' UTR, and a poly A-tail (>120 nt) at the 3' end.

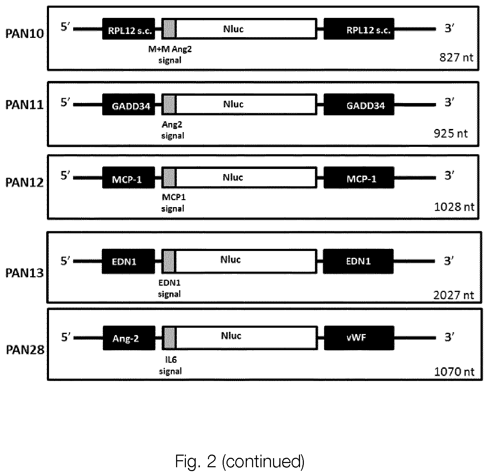

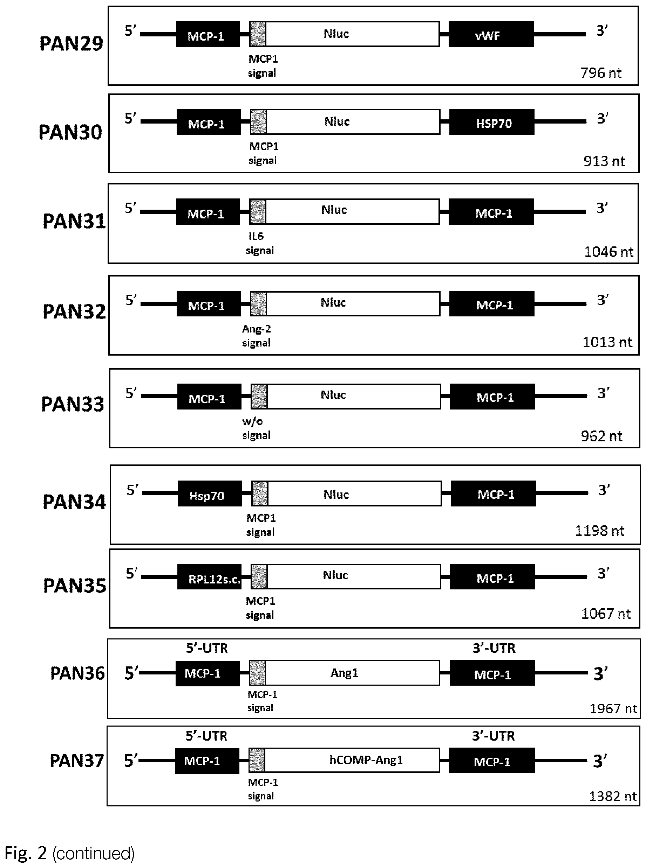

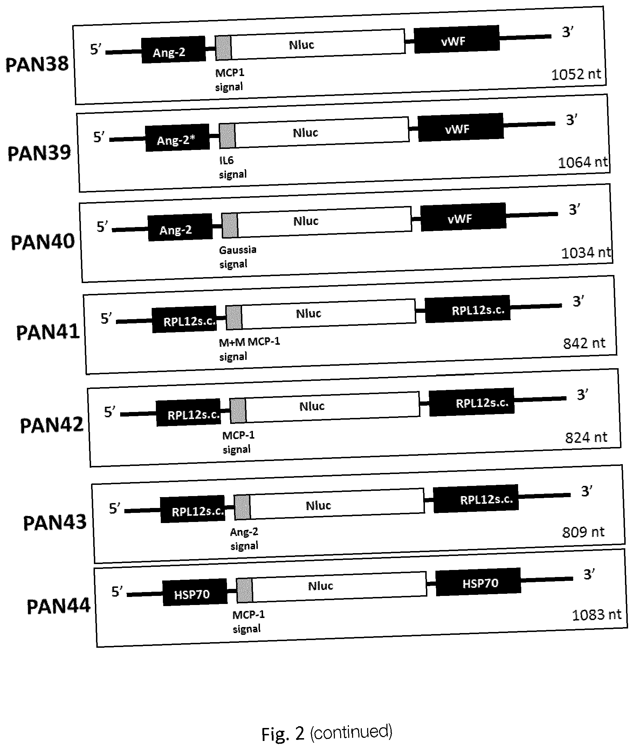

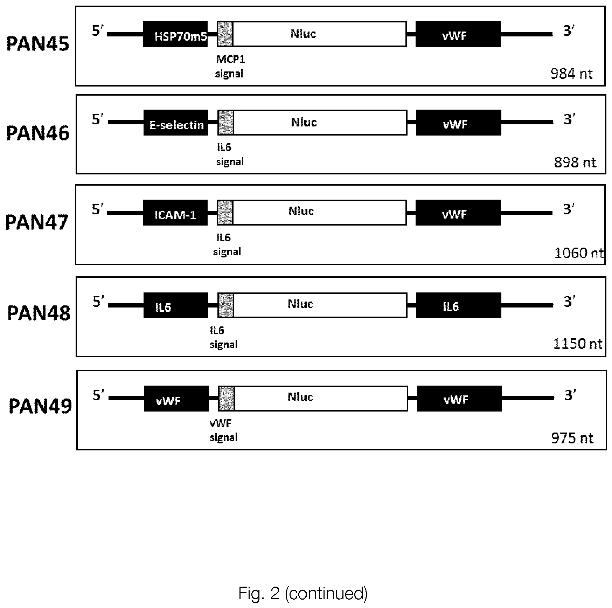

Fig. 2 is a schematic representation of selected examples for mRNA constructs with the coding sequence with an open reading frame being the one of Nano-luciferase. It is within the present invention that for each and any of the indicated mRNA constructs the coding region comprising a sequence with an open reading frame for Nano-luciferase may be replaced by a coding regions comprising a sequence coding for an effector molecule, particularly by a coding region comprising a sequence coding for an effector molecule disclosed herein; in a preferred embodiment thereof, the effector molecule is Ang1 or COMP-Ang1. In accordance with the present invention, the recombinant nucleic acid constructs are designed as follows:

Recombinant nucleic acid construct PAN02 comprises as the 5' non-translated region the 5' UTR of Ang1, as the nucleic acid sequence coding for a signal peptide the nucleic acid sequence coding for a signal peptide of Ang1, as the coding region coding for an effector molecule the coding region coding for Nano luciferase, and as the 3' non-translated region the 3' UTR of Ang1.

Recombinant nucleic acid construct PAN05 comprises as the 5' non-translated region the 5' UTR of Ang2, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of von Willebrand Factor (vWF).

Recombinant nucleic acid construct PAN07 comprises as the 5' non-translated region the 5' UTR of Galectin-9, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of von Willebrand Factor (vWF).

Recombinant nucleic acid construct PAN08 comprises as the 5' non-translated region the 5' UTR of Hsp70, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of HSP70.

Recombinant nucleic acid construct PAN09 comprises as the 5' non-translated region the 5' UTR of H3.3, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of H3.3.

Recombinant nucleic acid construct PAN10 comprises as the 5' non-translated region the 5' UTR of RPL12s.c., as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of RPL12s.c. This construct additionally comprises another start codon preceding the start codon of the coding region for Nano-luciferase in-frame.

Recombinant nucleic acid construct PAN11 comprises as the 5' non-translated region the 5' UTR of GADD34, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of GADD34.

Recombinant nucleic acid construct PAN12 comprises as the 5' non-translated region the 5' UTR of MCP-1 as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of MCP-1.

Recombinant nucleic acid construct PAN13 comprises as the 5' non-translated region the 5' UTR of EDN1, as the nucleic acid sequence coding for a signal peptide the nucleic acid sequence coding for a signal peptide of EDN1, as the coding region coding for an effector molecule the coding region coding for Nano luciferase, and as the 3' non-translated region the 3' UTR of EDN1.

Recombinant nucleic acid construct PAN28 comprises as the 5' non-translated region the 5' UTR of Ang2, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of vWF.

Recombinant nucleic acid construct PAN29 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of von Willebrand Factor (vWF).

Recombinant nucleic acid construct PAN30 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of HSP70.

Recombinant nucleic acid construct PAN31 comprises as the 5' non-translated region the 5' UTR of MCP-1, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of MCP-1.

Recombinant nucleic acid construct PAN32 comprises as the 5' non-translated region the 5' UTR of MCP-1, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of MCP-1.

Recombinant nucleic acid construct PAN33 comprises as the 5' non-translated region the 5' UTR of MCP-1, no nucleic acid sequence coding for a signal peptide, as the coding region coding for an effector molecule the coding region coding for Nano luciferase, and as the 3' non-translated region the 3' UTR of MCP-1.

Recombinant nucleic acid construct PAN34 comprises as the 5' non-translated region the 5' UTR of Hsp70, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of MCP-1.

Recombinant nucleic acid construct PAN35 comprises as the 5' non-translated region the 5' UTR of RPL12 s.c. (spinach chloroplast), as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of MCP-1.

Recombinant nucleic acid construct PAN36 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Ang1, and as the 3' non-translated region the 3' UTR of MCP-1.

Recombinant nucleic acid construct PAN37 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for hCOMP-Ang1, and as the 3' non-translated region the 3' UTR of MCP-1.

Recombinant nucleic acid construct PAN38 comprises as the 5' non-translated region the 5' UTR of Ang2, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of von Willebrand Factor (vWF).

Recombinant nucleic acid construct PAN39 comprises as the 5' non-translated region the 5' UTR of Ang2* (Ang2 5'-UTR with deleted upstream ATGs), as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of von Willebrand Factor (vWF).

Recombinant nucleic acid construct PAN40 comprises as the 5' non-translated region the 5' UTR of Ang2, as the nucleic acid sequence coding for a signal peptide the nucleic acid sequence coding for a signal peptide of Gaussia luciferase, as the coding region coding for an effector molecule the coding region coding for Nano luciferase, and as the 3' non-translated region the 3' UTR of von Willebrand Factor (vWF).

Recombinant nucleic acid construct PAN41 comprises as the 5' non-translated region the 5' UTR of RPL12 s.c., as the nucleic acid sequence coding for a signal peptide the nucleic acid sequence coding for a signal peptide of MCP-1 with an additional upstream ATG for translational start, as the coding region coding for an effector molecule the coding region coding for Nano luciferase, and as the 3' non-translated region the 3' UTR of RPL12 s.c.. This construct additionally comprises another start codon preceding the start codon of the coding region for Nano-luciferase in-frame.

Recombinant nucleic acid construct PAN42 comprises as the 5' non-translated region the 5' UTR of RPL12 s.c., as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of RPL12 s.c..

Recombinant nucleic acid construct PAN43 comprises as the 5' non-translated region the 5' UTR of RPL12 s.c., as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of RPL12 s.c..

Recombinant nucleic acid construct PAN44 comprises as the 5' non-translated region the 5' UTR of Hsp70, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of Hsp70.

Recombinant nucleic acid construct PAN45 comprises as the 5' non-translated region the 5' UTR of Hsp70m5, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of von Willebrand Factor (vWF).

Recombinant nucleic acid construct PAN46 comprises as the 5' non-translated region the 5' UTR of E-selectin, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of von Willebrand Factor (vWF).

Recombinant nucleic acid construct PAN47 comprises as the 5' non-translated region the 5' UTR of ICAM1, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of von Willebrand Factor (vWF).

Recombinant nucleic acid construct PAN48 comprises as the 5' non-translated region the 5' UTR of IL-6, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of IL-6).

Recombinant nucleic acid construct PAN49 comprises as the 5' non-translated region the 5' UTR of von Willebrand Factor (vWF), as the nucleic acid sequence coding for a signal peptide the nucleic acid sequence coding for a signal peptide of von Willebrand Factor (vWF), as the coding region coding for an effector molecule the coding region coding for Nano luciferase, and as the 3' non-translated region the 3' UTR of von Willebrand Factor (vWF).

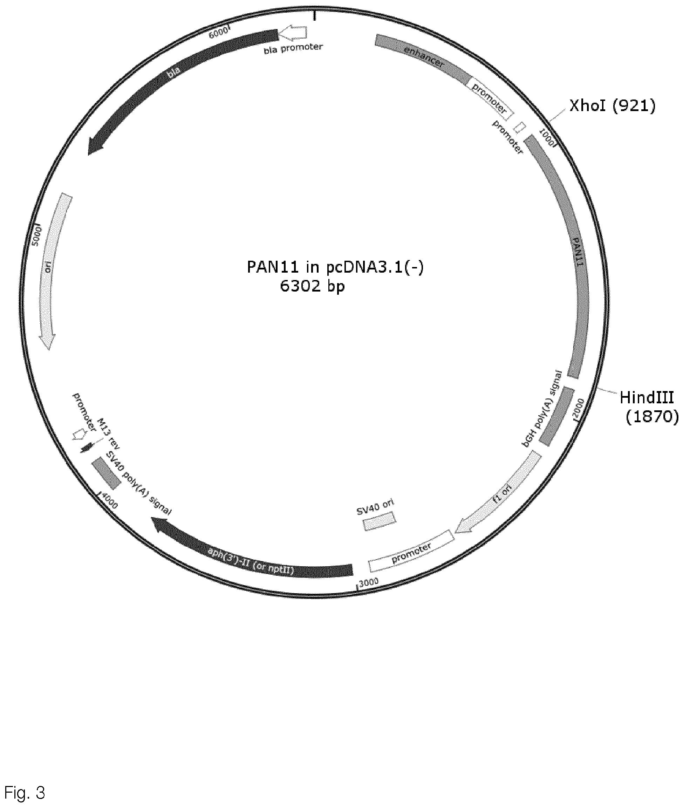

Fig. 3 shows a restriction map of a plasmid pcDNA3.1(-) used as an illustrative example for a plasmid for the expression of an exemplary recombinant nucleic acid construct of the present invention, whereby such exemplary recombinant nucleic acid construct is construct PAN 11; restriction sites XhoI/HindIII are shown.

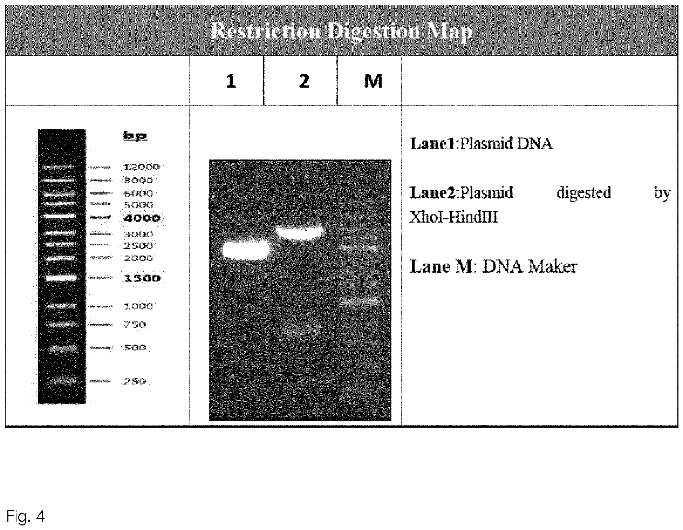

Fig. 4 shows a restriction digestion map of the PAN11 expressing pcDNA3.1- plasmid of Fig. 3; the insert represents PAN11 sequence with engineered 5' and 3' UTR, signal sequence and the coding region coding for Nano-Luciferase.

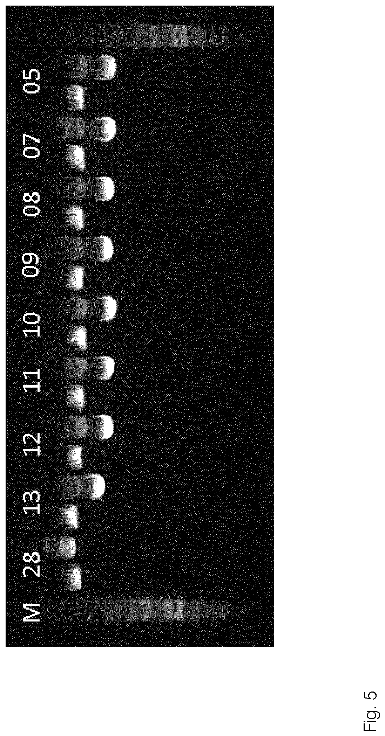

Fig. 5 is an 1% agarose gel stained with EtBr showing the non-linearized (uncut, supercoiled, right lanes) and linearization product (left lanes) of pcDNA3.1(-) plasmids upon BamHI restriction of the constructs PAN28 ("28"), PAN13 ("13"), PAN12 ("12"), PAN11 ("11"), PAN10 ("10"), PAN09 ("09"), PAN08 ("08"), PAN07 ("07") and PAN05 ("05").

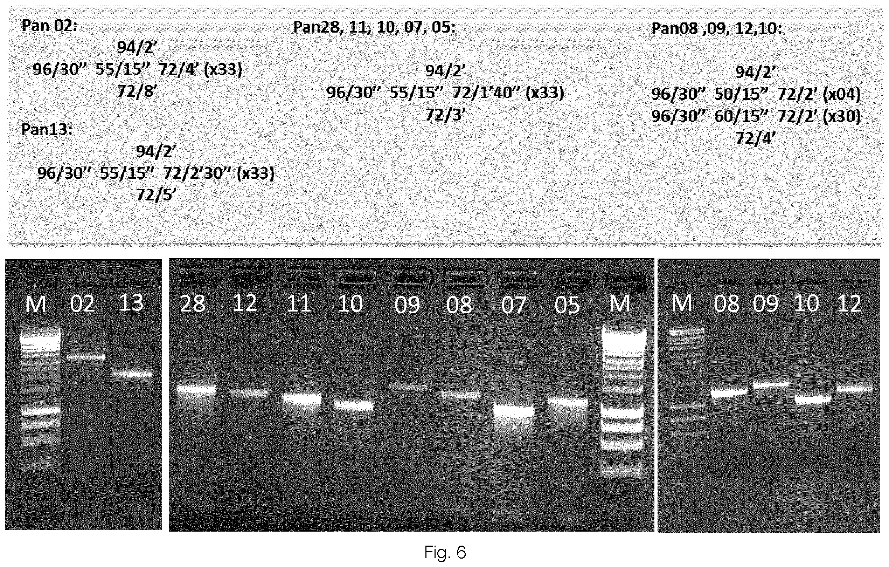

Fig. 6 shows 1% agarose gels stained with EtBr showing PCR products of Poly-A tailing PCRs for the addition of 120 nt of Poly-A tail by 120 nt long poly-T 3' primer flanking the different recombinant nucleic acid constructs. On top are the optimized PCR conditions for the indicated recombinant nucleic acid constructs. For example, for Pan02 the conditions are as follows: denaturation for 2 minutes at 94°C. 33 cycles of 30 seconds at 96°C, 15 seconds at 55°C and 4 minutes at 72°C. Final extension for 8 minutes at 72°C.

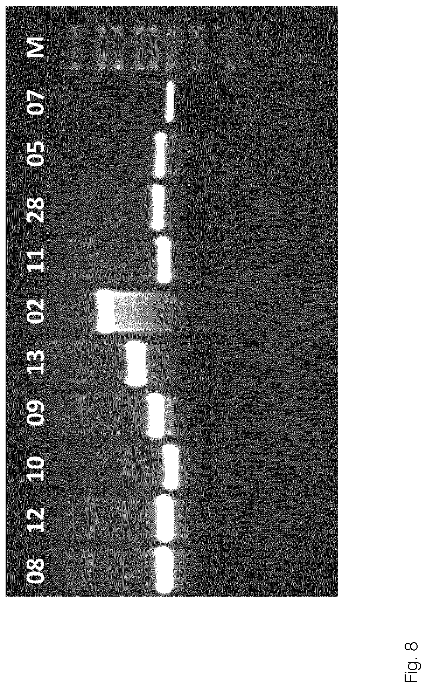

Fig. 8 is an image of a 1% non-denaturing agarose gel stained with EtBr showing the in vitro transcribed mRNAs of constructs PAN28 ("28"), PAN13 ("13"), PAN12 ("12"), PAN11 ("11"), PAN10 ("10"), PAN09 ("09"), PAN08 ("08"), PAN07 ("07") and PAN05 ("05"). M: is a lane with a high range RiboRuler RNA ladder. From this Fig. 8 may be taken that the transcribed mRNAs were intact and, more specifically, were not degraded.

Fig. 24 shows the basic structure and nucleotide sequence of recombinant nucleic acid construct PAN01. PAN1 is an embodiment of the present invention comprising as the 5' non-translated region the 5' UTR of Ang1, as the nucleic acid sequence coding for a signal peptide the nucleic acid sequence coding for a signal peptide of Ang1, as the coding region coding for an effector molecule the coding region coding for Ang1, and as the 3' non-translated region the 3' UTR of Ang1.

Fig. 25 shows the basic structure and nucleotide sequence of recombinant nucleic acid construct PAN03. PAN03 is an embodiment of the present invention comprising as the 5' non-translated region the 5' UTR of Ang1, as the nucleic acid sequence coding for a signal peptide the nucleic acid sequence coding for a signal peptide of Ang1, as the coding region coding for an effector molecule the coding region coding for COMP-Ang1, and as the 3' non-translated region the 3' UTR of Ang1.

Fig. 26 shows the basic structure and nucleotide sequence of recombinant nucleic acid construct PAN04. PAN04 is an embodiment of the present invention comprising as the 5' non-translated region the 5' UTR of Ang2, as the nucleic acid sequence coding for a signal peptide the nucleic acid sequence coding for a signal peptide of Ang2, as the coding region coding for an effector molecule the coding region coding for Ang1, and as the 3' non-translated region the 3' UTR of von Willebrand Factor (vWF).

Fig. 27 shows the basic structure and nucleotide sequence of recombinant nucleic acid construct PAN06. PAN06 is an embodiment of the present invention comprising as the 5' non-translated region the 5' UTR of Ang2, as the nucleic acid sequence coding for a signal peptide the nucleic acid sequence coding for a signal peptide of Ang2, as the coding region coding for an effector molecule the coding region coding for COMP-Ang1, and as the 3' non-translated region the 3' UTR of von Willebrand Factor (vWF).

Fig. 28 shows the basic structure and nucleotide sequence of recombinant nucleic acid construct PAN36. PAN36 is an embodiment of the present invention comprising as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Ang1, and as the 3' non-translated region the 3' UTR of MCP-1.

Fig. 67 shows recombinant nucleic acid construct PAN50 which is another embodiment of an mRNA of the present invention comprising as the 5' non-translated region the 5' UTR of MCP-1, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of von Willebrand Factor (vWF).

Fig. 68 shows recombinant nucleic acid construct PAN51 which is another embodiment of an mRNA of the present invention comprising as the 5' non-translated region the 5' UTR of HSP70, as the nucleic acid sequence coding for a signal peptide the nucleic acid sequence coding for a signal peptide of IL-6, as the coding region coding for an effector molecule the coding region coding for Nano luciferase (NLuc), and as the 3' non-translated region the 3' UTR of von Willebrand Factor (vWF).

Fig. 69 shows recombinant nucleic acid construct PAN52 which is another embodiment of an mRNA of the present invention comprising as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Ang1, and as the 3' non-translated region the 3' UTR of von Willebrand Factor (vWF).

Fig. 70 shows recombinant nucleic acid construct PAN53 which is another embodiment of an mRNA of the present invention comprising as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for hCOMP-Ang1, and as the 3' non-translated region the 3' UTR of von Willebrand Factor (vWF).

Fig. 71A is an image of a 1% non-denaturing agarose gel stained with EtBr showing the tail-PCR products of constructs PAN35 ("35"), PA34 ("34"), PAN33 ("33"), PAN32 ("32"), PAN31 ("31"), PAN30 ("30"), PAN29 ("29") and PAN12 ("12"). M: is a lane with a high range RiboRuler RNA ladder. From this Fig. 71A may be taken that the transcribed mRNAs were intact and, more specifically, were not degraded.

Fig. 71B is an image of a 1% non-denaturing agarose gel stained with EtBr showing the *in vitro* mRNA transcripts of constructs PAN35 ("35"), PA34 ("34"), PAN33 ("33"), PAN32 ("32"), PAN31 ("31"), PAN30 ("30"), PAN29 ("29") and PAN12 ("12"). M: is a lane with a high range RiboRuler RNA ladder. From this Fig. 71B may be taken that the transcribed mRNAs were intact and, more specifically, were not degraded.

Fig. 86 shows recombinant nucleic acid construct PAN54 which is another embodiment of an mRNA of the present invention comprising as the 5' non-translated region the 5' UTR of RPL12s.c. as the nucleic acid sequence coding for a signal peptide the nucleic acid sequence coding for a signal peptide of MCP-1, as the coding region coding for an effector molecule the coding region coding for Nano-Luciferase (NLuc), and as the 3' non-translated region the 3' UTR of von Willebrand Factor (vWF).

Fig. 87 shows recombinant nucleic acid construct PAN55 which is another embodiment of an mRNA of the present invention comprising as the 5' non-translated region the 5' UTR of vWF, as the nucleic acid sequence coding for a signal peptide the nucleic acid sequence coding for a signal peptide of MCP-1, as the coding region coding for an effector molecule the coding region coding for Nano Luciferase (NLuc), and as the 3' non-translated region the 3' UTR of von Willebrand Factor (vWF).

Fig. 88 shows recombinant nucleic acid construct PAN56 which is another embodiment of an mRNA of the present invention comprising as the 5' non-translated region the 5' UTR of vWF, as the nucleic acid sequence coding for a signal peptide the nucleic acid sequence coding for a signal peptide of vWF, as the coding region coding for an effector molecule the coding region coding for Nano Luciferase (NLuc), and as the 3' non-translated region the 3' UTR of von Willebrand Factor (vWF).

Fig. 89 shows recombinant nucleic acid construct PAN57 which is another embodiment of an mRNA of the present invention comprising as the 5' non-translated region a synthetic nucleic acid sequence as described in Jiang, Lei et al. (2018). Systemic messenger RNA as an etiological treatment for acute intermittent porphyria. Nature Medicine. 24, 1899-2909 (2018) , as the nucleic acid sequence coding for a signal peptide the nucleic acid sequence coding for a signal peptide of MCP-1, as the coding region coding for an effector molecule the coding region coding for Nano Luciferase (NLuc), and as the 3' non-translated region the 3' UTR a nucleic acid sequence as described in Jiang, Lei et al. (2018). Systemic messenger RNA as an etiological treatment for acute intermittent porphyria. Nature Medicine. 24; 1899-1909 (2018). The 5'-and 3' sequences are shown underlined and in bold.

Fig. 90 shows recombinant nucleic acid construct PAN58 which is another embodiment of an mRNA of the present invention comprising as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Nano Luciferase (NLuc), and as the 3' non-translated region the 3' UTR of von Willebrand Factor (vWF). In addition, PAN58 comprises a consensus Kozak sequence (GCCACC) in the six bases upstream of the start AUG.

Fig. 91 shows recombinant nucleic acid construct PAN59 which is another embodiment of an mRNA of the present invention comprising as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for CMP-Ang1, and as the 3' non-translated region the 3' UTR of von Willebrand Factor (vWF).

Fig. 95 shows recombinant nucleic acid construct PAN60 which is another embodiment of an mRNA of the present invention comprising as the 5' non-translated region the 5' UTR of MCP-1, as the coding region coding for an effector molecule the coding region coding for wild type (wt) Tie2, and as the 3' non-translated region the 3' UTR of von Willebrand Factor (vWF).

Fig. 96 shows recombinant nucleic acid construct PAN61 which is another embodiment of an mRNA of the present invention comprising as the 5' non-translated region the 5' UTR of MCP-1, as the coding region coding for an effector molecule the coding region coding for Tie2 mutant R849W , and as the 3' non-translated region the 3' UTR of von Willebrand Factor (vWF).

Fig. 97 shows recombinant nucleic acid construct PAN66 which is another embodiment of an mRNA of the present invention comprising as the 5' non-translated region the 5' UTR of MCP-1, as the coding region coding for an effector molecule the coding region coding for wild type PIK3CA which is, according to GenBank Homo sapiens phosphatidylinositol-4,5-bisphosphate 3-kinase catalytic subunit alpha (PIK3CA) with the respective reference number being 006218.4., and as the 3' non-translated region the 3' UTR of von Willebrand Factor (vWF).

The SEQ ID NOs: of the sequence listing are related to the instant disclosure as summarized in Table 1.

**Table 1:**

| **Seq ID No:** | **Subject to Fig.** | **Further information** |
|---|---|---|
| 1 | 7A | 5'Primer |
| 2 | 7A | 5'Primer |
| 3 | 7A | 5'Primer |
| 4 | 7A | 5'Primer |
| 5 | 7A | 5'Primer |
| 6 | 7A | 5'Primer |
| 7 | 7A | 5'Primer |
| 8 | 7A | 5'Primer |
| 9 | 7A | 5'Primer |
| 10 | 7A | 5'Primer |
| 11 | 7B | 3'Primer |
| 12 | 7B | 3'Primer |
| 13 | 7B | 3'Primer |
| 14 | 7B | 3'Primer |
| 15 | 7B | 3'Primer |
| 16 | 7B | 3'Primer |
| 17 | 7B | 3'Primer |
| 18 | 7B | 3'Primer |
| 19 | 7B | 3'Primer |
| 20 | 15A | 5'UTR MCP 1 |
| 21 | 15B | SP (signal peptide) nucleotide sequence of MCP1 |
| 22 | 15B | SP amino acid sequence of MCP1 |
| 23 | 15C | 3'UTR of MCP1 |
| 24 | 16A | 5'UTR of RPL12 |
| 25 | 16B | 3'UTR of RPL12 |
| 26 | 17A | 5'UTR of Ang2 |
| 27 | 17B | SP (signal peptide) nucleotide sequence of Ang2 |
| 28 | 17B | SP (signal peptide) amino acid sequence of Ang2 |
| 29 | 17C | 3'UTR of Ang2 |
| 30 | 18A | 5'UTR of IL6 |
| 31 | 18B | SP (signal peptide) nucleotide sequence of IL6 |
| 32 | 18B | SP (signal peptide) amino acid sequence of IL6 |
| 33 | 18C | 3'UTR of IL6 |
| 34 | 19A | 5'UTR of vWF |
| 35 | 19B | SP(signal peptide) nucleotide sequence of vWF |
| 36 | 19B | SP (signal peptide) amino aicd sequence of vWF |
| 37 | 19C | 3'UTR of vWF |
| 38 | 20A | 5'UTR of HSP70 A1 |
| 39 | 20B | 3'UTR of HSP70 A1 |
| 40 | 21A | 5'UTR of HSP70 A5 |
| 41 | 21B | SP (signal peptide) nucleotide sequence of HSP70 A5 |
| 42 | 21B | SP (signal peptide) amino acid sequence of HSP70 A5 |
| 43 | 21C | 3'UTR of HSP70 A5 |
| 44 | 22A | 5'UTR of H3.3 |
| 45 | 22B | 3'UTR of H3.3 |
| 46 | 23A | 5'UTR of LGALS9 |
| 47 | 23B | 3'UTR of LGALS9 |
| 48 | 24 | Construct PAN01 |
| 49 | 25 | Construct PAN03 |
| 50 | 26 | Construct PAN04 |
| 51 | 27 | Construct PAN06 |
| 52 | 28 | Construct PAN36 |
| 53 | 29 | Construct PAN37 |
| 54 | 30 | Construct PAN02 |
| 55 | 31 | Construct PAN05 |
| 56 | 32 | Construct PAN07 |
| 57 | 33 | Construct PAN08 |
| 58 | 34 | Construct PAN09 |
| 59 | 35 | Construct PAN10 |
| 60 | 36 | Construct PAN11 |
| 61 | 37 | Construct PAN12 |
| 62 | 38 | Construct PAN13 |
| 63 | 39 | Construct PAN28 |
| 64 | 40 | Construct PAN29 |
| 65 | 41 | Construct PAN30 |
| 66 | 42 | Construct PAN31 |
| 67 | 43 | Construct PAN32 |
| 68 | 44 | Construct PAN33 |
| 69 | 45 | Construct PAN34 |
| 70 | 46 | Construct PAN35 |
| 71 | 47 | Construct PAN38 |
| 72 | 48 | Construct PAN39 |
| 73 | 49 | Construct PAN40 |
| 74 | 50 | Construct PAN41 |
| 75 | 51 | Construct PAN42 |
| 76 | 52 | Construct PAN43 |
| 77 | 53 | Construct PAN44 |
| 78 | 54 | Construct PAN45 |
| 79 | 55 | Construct PAN46 |
| 80 | 56 | Construct |
| 81 | 57 | Construct PAN48 |
| 82 | 58 | Construct PAN49 |
| 83 | 59A | 5'UTR of Ang1 |
| 84 | 59B | SP (signal peptide) nucleotide sequence of Ang1 |
| 85 | 59B | SP (signal peptide) amino acid sequence of Ang1 |
| 86 | 59C | CDS of Ang1 (mature peptide+stop codon) |
| 87 | - | Amino acid sequence derived from SEQ ID No. 86 |
| 88 | 59D | 3'UTR of Ang1 |
| 89 | 60A | 5'UTR of Ang4 |
| 90 | 60B | SP (signal peptide) nucleotide sequence of Ang4 |
| 91 | 60B | SP (signal peptide) amino acid sequecne of Ang4 |
| 92 | 60C | CDS of Ang4 (mature peptide+stop codon) |
| 93 | - | Amino acid sequence derived from SEQ ID No.92 |
| 94 | 60D | 3'UTR of Ang4 |
| 95 | 61 | COMP-Ang1 CDS (mature pep+stop codon) (rat) |
| 96 | - | Amino acid sequence derived from SEQ ID No.95 |
| 97 | 62 | hCOMP-Ang1 CDS (mature pep+stop codon) (human) |
| 98 | - | Amino acid sequence derived from SEQ ID No.97 |
| 99 | 63 | CMP-Ang1 CDS (mat peptide+stop codon) |
| 100 | 64 | COMP-Ang2 CDS (mat peptide+stop codon) |
| 101 | 65 | Tie2 CDS (start+mat pep+stop codon) |
| 102 | - | Amino acid sequence derived from SEQ ID No.101 |
| 103 | 66 | Tie2* CDS (R849W)(start+mat pep+stop codon) |
| 104 | - | Amino acid sequence derived from SEQ ID No.103 |
| 105 | 67 | Construct PAN50 |
| 106 | 68 | Construct PAN51 |
| 107 | 69 | Construct PAN52 |
| 108 | 70 | Construct PAN53 |
| 109 | 86 | Construct PAN54 |
| 110 | 87 | Construct PAN55 |
| 111 | 88 | Construct PAN56 |
| 112 | 89 | Construct PAN57 (Mod) |
| 113 | 90 | Construct PAN58 |
| 114 | 91 | Construct PAN59 |
| 115 | 95 | Construct PAN60 (Tie2 wt) |
| 116 | 96 | Construct PAN61 (Tie2 R849W) |
| 117 | 97 | Construct PAN66 (PIK3CA wt) |
| 118 | | Construct CDS (start+mat pep+stop codon) PIK3CA |
| 119 | | CDS (mature peptide+stop cod) Nluc |
| 120 | 100a | 5'UTR of (PAN57) |
| 121 | 100b | 3'UTR of (PAN57) |
| 122 | 7B | 3'Primer for constructs PAN01-PAN03 |
| 123 | 100c | 5'Primer for construct PAN57 |
| 124 | 100d | 3'Primer for construct PAN57 |
| 125 | 100e | 5'Primer for construct PAN55 (Fig. 87) and PAN56 (Fig 88) |

### Example 1: Materials and Methods

### Plasmid Template generation by gene synthesis and cloning

Sequences encoding Nluc reporter protein, Ang-1 protein and derivatives thereof (e.g. COMP-Ang-1, CMP-Ang-1) with different signal peptides were flanked by different heterologous 5' and 3' UTRs and were designed and produced by gene synthesis by BioCat (Heidelberg) and cloned (Xho1-BamHI) into pcDNA3.1- (Thermo Fisher).

Sequences encoding wt Tie-2 and sequences with the specified Tie-2 mutation in the coding region flanked by different heterologous 5' and 3' UTRs were designed (see Figures 95 and 96) and produced by gene synthesis by BioCat (Heidelberg) and cloned (Xho1-BamHI) into pcDNA3.1- (Thermo Fisher).

### In vitro transcription from PCR products to generate polyadenylated mRNAs

In vitro transcription is the synthesis of RNA transcripts by RNA polymerase from a linear DNA template containing the corresponding promoter sequence (T7, T3, SP6) and the gene to be transcribed. A typical transcription reaction consists of the template DNA, RNA polymerase, ribonucleotide triphosphates, RNase inhibitor and buffer containing Mg2⁺ ions. Linearized plasmid DNA, PCR products and synthetic DNA oligonucleotides can be used as templates for transcription as long as they have the T7 promoter sequence upstream of the gene to be transcribed. In order to generate DNA templates with a poly-(A) tail a tail-PCR using 5' primers containing T7 RNA polymerase sequences and 3' primers with a 120 nt Poly-T sequence were synthesized and purified (BioSpring, Frankfurt).

The Linear DNA templates for in vitro transcription were generated by PCR from linearized plasmid. Plasmids were digested with BamHI that cut once 3-terminal in the vector backbone to produce a linearized vector that can be used as the template for the poly-(A) tail PCR. 5µg plasmid are digested for 2h at 37°C with 5U BamHI restriction enzyme in 50µl and 1x HF Buffer. Small Aliquots of digested mix were analyzed by gel electrophoresis to check for complete digestion of the plasmid (see, for example, Fig. 5 for constructs PAN28 ("28"), PAN13 ("13"), PAN12 ("12"), PAN11 ("11"), PAN10 ("10"), PAN09 ("09"), PAN08 ("08"), PAN07 ("07") and PAN05 ("05")). The restriction enzyme is heat-inactivated by incubating at 80°C for 20 min and the digested plasmid is purified by a PCR purification column following the manufacturer's protocol (NucleoSpin Gel and PCR clean-up, Macherey-Nagel). The linearized plasmid can be stored at -20°C for several months and can be used for PCR template generation. The purpose of linearization was to eliminate circular templates that could potentially generate run-on transcripts during the IVT reaction.

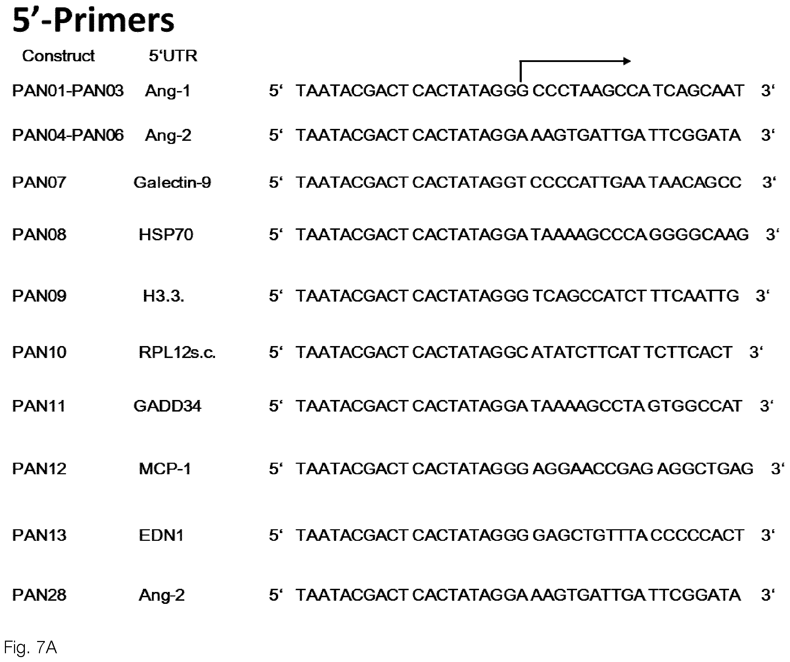

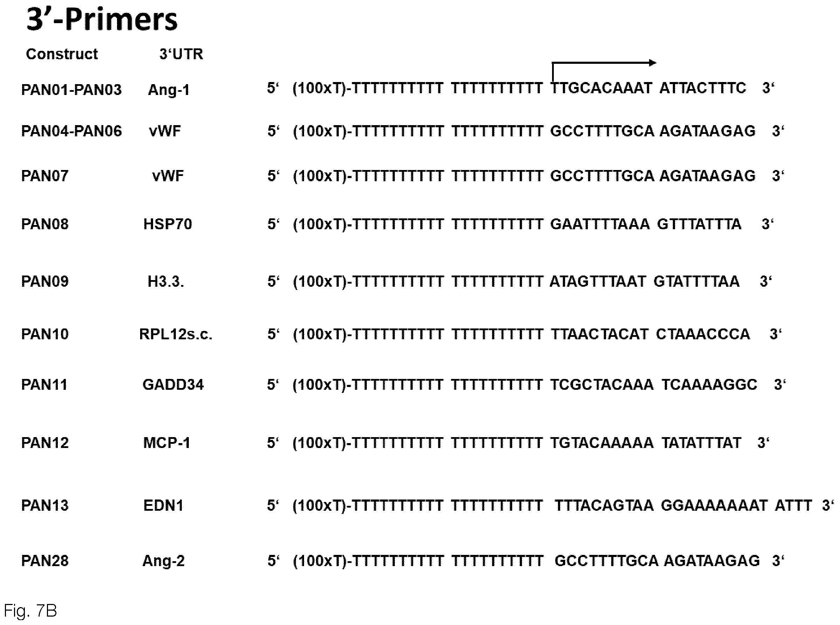

Addition of poly-(A) tail by PCR was performed by using Hot StarHiFidelity polymerase (Qiagen) or Taq DNA Polymerase (Roche) following the manufacturer's protocol. For this purpose, Adapter primers containing T7 promoter sequences and 3' Poly A tail sequences (120nt) were used to amplify linear DNA-templates. Each of the adapter primers had overlap sequences which fused regulatory sequences necessary for translation and transcription to the gene of interest (see, for example, Figures 7a and 7b). A typical 50µl PCR reaction contained final concentrations of 200µM (of each) dNTP, 0,5µM for each primer, 50 ng linearized plasmid DNA, 1x PCR reaction buffer (1,5 mM MgCl2) and 1,25 U Taq DNA Polymerase per reaction (0,25µl of 5U/µl). The specific PCR cycle programs or thermal profiles were adjusted according to the Tm of the primer pairs, according to the expected length of the PCR product and according to the used thermal cycler. The quality of the PCR products was checked by analyzing an aliquot by gel electrophoresis and the reactions are purified by commercial PCR purification kits ((NucleoSpin Gel and PCR clean-up, Macherey-Nagel).

### In vitro transcription reaction

Addition of a 5' end cap structure to the RNA is an important process in eukaryotes. It is essential for RNA stability, efficient translation, nuclear transport and splicing. The process involved addition of a 7-methylguanosine cap at the 5' triphosphate end of the RNA. RNA capping can be carried out post-transcriptionally using capping enzymes or co-transcriptionally using cap analogs such as ARCA (Jena Bioscience) or CleanCap (Trilink Biotechnologies) or EZ-Cap (ApexBio). In the enzymatic method, the mRNA may be capped using the vaccinia virus mRNA capping enzyme (NEB) as per manufacturer's protocol. The enzyme adds on a 7-methylguanosine cap at the 5' end of the RNA using GTP and S-adenosyl methionine as donors (cap-0 structure). Both methods yield functionally active capped RNA suitable for transfection or other applications. In addition, a 2'-O-Methyltransferase can be used to introduce cap-1 and cap-2 structures.

In the example described below, the T7 High Yield Transcription Kit (Thermo Scientific) was used to synthesize capped RNA transcripts (PAN 01-XX) using cap analogs co-transriptionally.

The DNA template for the transcription reaction was a linearized plasmid or a PCR product. For the capped RNA the reaction at room temperature was set up in the following order:

| **Component** | **Volume (µl)** | **Final** |
|---|---|---|
| Nuclease free water* | To 20 | |
| 5X Reaction Buffer | 4 | 1X |
| ATP (100 mM) | 2 | 10mM |
| CTP (100 mM) or 5'-Methylcytidine-5'-Triphophate (100 mM) | 2 | 10mM |
| UTP (100 mM) or Pseudouridine-5'-Triphosphate (100 mM) or 5-Methoxyuridine-5'-Triphosphate (100 mM) N1-Methyl-pseudouridine (100mM) | 2 | 10mM |
| GTP (30 mM) | 2 | 3mM |
| 3'-0-Me-m⁷G(5')ppp(5')G cap analog (ARCA; Anti Reverse Cap Analog) (100 mM) | 2 | 10mM |
| Template DNA* | X | 1µg |
| T7 RNA Polymerase Mix | 2 | |
| **Total** | **20** | |

1 µg DNA was added and the total reaction volume to 20 µl was obtained by adding nuclease free water. The amount of water to be added varied based on the concentration of the template DNA. The reactions at were well mixed by vortexing and incubated at 37 °C for 2 hours in a dry air incubator. The transcription reactions were treated with DNase I to remove the DNA template before proceeding with purification as follows:
1. 70 µl nuclease free water was added to the transcription reactions followed by 10 µl of DNase I reaction buffer.
2. 2 µl DNase I were added to the reactions.
3. The reaction was incubated at 37 °C for 15 minutes.

### Column Purification of Capped mRNA

1. The capped RNA was purified using the MEGAclearKit as per the manufacturer's instructions (any spin column based RNA purification kit may be used).
2. The RNA was quantified using a NanoDrop Spectrophotometer.
3. As per the manufacturer's instructions, RNA sample quality was assessed using the Agilent RNA 6000 Nano Kit and Agilent 2100 Bioanalyzer or by native 1% agarose gel electrophoresis and ethidium bromide staining (see, Fig. 8).

### mRNA transfection of HeLa cells, HEK293 cells, HPMEC cells, HPAEC cells and HUVEC cells

HeLa cells, were grown in DMEM complete medium. The human embryonic kidney 293 cell line (HEK293) is grown in EMEM (EBSS) + 2mM Glutamine + 1% Non Essential Amino Acids (NEAA) + 10% FCS culture medium.

The human embryonic kidney 293 cell line (HEK293) is grown in EMEM (EBSS) + 2mM Glutamine + 1% Non Essential Amino Acids (NEAA) + 10% FCS culture medium. Subculture Routine is to split sub-confluent cultures (70-80%) 1:2 to 1:6 i.e. seeding at 2-5x10,000 cells/cm² using 0.25% trypsin or trypsin/EDTA; 5% CO₂; 37°C. mRNA transfection are performed with the Lipofectamine^{™} MessengerMAX^{™} Transfection Reagent (Invitrogen^{™}) according to the manufactures protocol.

Primary Human Pulmonary Microvascular Endothelial Cells (HPMEC) and Primary Human Pulmonary Artery Endothelial Cells (*HPAEC*) are isolated from human pulmonary arteries are most appropriate for studying human lung diseases and are isolated from the lung from a single donor. Since lung tissue contains blood and lymphatic capillaries, HPMEC comprise Blood and Lymphatic Microvascular Endothelial Cells.

The cell type of HPMEC's are characterized by immunofluorescent staining. They stain positive for CD31 and von Willebrand factor and negative for smooth muscle alpha-actin.

HUVEC, HPAEC and HPMEC cells were grown in Endothelial Cell Growth Medium as recommended from PromoCell (Heidelberg Germany,) respectively. The Endothelial Cell Growth Medium is basal Medium supplemented with Fetal Calf Serum (0.05 ml/ml), Endothelial Cell Growth Supplement (bovine hypothalamic extract; 0.004 ml ml), Heparin 90µg/ml and Hydrocortisone 1µg/ml. The experiments with HUVEC, HPAEC and HPMEC cells are performed on cells with passage numbers below 8.

RNA transfections and more mRNA transfections were carried out using Lipofectamin MessengerMAX (Invitrogen, Carlsbad, CA; Thermo Fisher Scientific).. For mRNA transfection in 24-well plate format 1×10⁵ cells/well were seeded 24h before transfection to reach 70-90 confluence immediately before transfection. For one single well 1.5µl Lipofectamine MessengerMAX Reagent was added to 25µl OptMEM (Invitrogen, Carlsbad, CA; Thermo Fisher Scientific), vortexed and incubated for 10 min at RT. In a second well 500ng or 1µg mRNA were added to 25µl OptiMEM, and vortexed. After incubation diluted mRNA was added to diluted Lipofectamin, mixed well, incubated for 5 min at RT and added dropwise to cells in the presence of 0,5 ml of complete medium for Hela and HEK293. For HUVEC, HPAEC and HPMEC the Endothelial Cell Growth medium was replaced and the cells washed with OptiMEM and then the Lipofectamin MessengerMAxX mRNA complex solution added to the well with 0,5 ml OptiMEM prewarmed to 37 °C . Cells were incubated at 37°C for 2h and the OptiMEM medium replaced with complete Endothelial Cell Growth Medium (PromoCell, Heidelberg Germany).

Cell Lysates from Transfected cells or serum were analyzed at the indicated time points. Alternatively, mRNA-LNPs based on the cationic lipids L-Arginyl-β-alanine-N-palmityl-N-oleyl-amide or β-(L-Arginyl)-L-2,3-diamino propionic acid-N-palmityl-N-oleyl-amide in combination with neutral and PEGylated co-lipids were used for in vitro transfection. The co-lipids were Diphytanoyl-PE and the PEGylated lipid is methoxyPEG2000-DSPE

Successful introduction of mRNA into host cells was monitored using various known methods, such as a fluorescent marker, such as Green Fluorescent Protein (GFP), such as reporter enzymes (e.g. luciferase derivatives). Alternatively, transfection of a modified mRNA could also be determined by measuring the protein expression level of the target polypeptide by e.g., Western Blotting or immunocytochemistry or ELISA.

Fully modified mRNAs with 5-methylcytidine and/or pseudouridine (or 5-methoxyuridine or N1-methylpseudouridine) are transfected into HUVEC or HPMEC (Human Pulmonary Microvascular Endothelial Cells, PromoCell, Heidelberg) using Lipofectamin MessengerMAX (Invitrogen, Carlsbad, CA; Thermo Fisher Scientific). The cell lysates are harvested and run by ELISA (or on immunoblot assays (western) at different time points (30", 1 h, 2h, 4h, 6h hours) after transfection to determine the protein expression.

### Nano-Luc Luciferase activity reporter measurement

A time dependent quantitative detection of Nano-Glo-Luciferase expression (Promega) was performed for secreted versions by analyzing samples from the tissue culture supernatant according to the reporter assay technical manual Nano-Glo Luciferase Assay System (Promega). In brief, 5-20µl of serum or lysate were diluted in 100µl H₂O final volume and equal volume of reconstituted Nano-Glo luciferase assay reagent was combined in a 96 well GloMax 96 Microplate. After at least 3 min incubation at RT luminescence were measured in an appropriate luminometer.

### Immunoblot detection of Tie-2 and derivates

For immunoblot detection of Tie-2 and derivatives protein lysates are loaded on NuPage SDS-PAGE system (chambers and power supply) with 1.5mm ready-to-use Bis-Tris gels and 4-12% acrylamide gradient with MOPS-buffer as running aid (all Life Technologies, Grand Island, NY). Each lysate sample is prepared to 40 µl final volume. This sample contains 25 µg protein lysate in variable volume, RIPA buffer to make up volume to 26 µl, 4 µl of l0x reducing agent and l0 µl 4x SDS loading buffer (both from Life Technologies, Grand Island, NY). Samples are heated at 95°C for 5min and loaded on the gel. Standard settings are chosen by the manufacturer, 200V, 120mA and max. 25 W. Run time is 60min, but no longer than running dye reaching the lower end of the gel.

After the run is terminated, the plastic case is cracked and the encased gel transferred to a ready-to-use nitrocellulose membrane kit and power supply (iBLOT; LifeTechnologies, Grand Island, NY). Using default settings, the protein lysate is transferred by high Ampere electricity from the gel to the membrane.

After the transfer, the membranes are incubated in 5% BSA in IX TBS for 15 minutes then in 5% BSA in IX TBS + 0.1 % Tween for another 15 minutes. Primary antibodies against human Tie-2 proteins or Phospho-specific Antibodies (P-Tie-2, P-Akt, see below) are applied in 3ml of 5% BSA in IX TBS solution at a 1:500 to 1:2000 dilution for 3 hours at room temperature and gentle agitation on an orbital shaker. Membranes are washed 3 times with IX TBS/0.1 % Tween, 5 minutes each time with gentle agitation. The secondary antibody (Goat anti-rabbit HRP conjugate; Abeam, Cambridge, MA) is conjugated to horse radish peroxidase and binds to the primary antibody antibodies. The secondary antibody is diluted from 1:1000 to 1 :5000 in 5% BSA in IX TBS and incubated for 3 hrs at RT. At the end of incubation time, the membranes are washed 3 times with IX TBS/0.1 % Tween, 5 minutes each time with gentle agitation. The membranes are developed in 5ml Pierce WestPico Chemiluminescent Subtrate (Thermo Fisher, Rockford, IL) as directed.

### Example 2: Expression of luciferase, Ang-1 or Ang-1-derivatives expressing recombinant nucleic acid constructs in HPMEC, HUVEC, HPAEC and HeLa cells

### mRNAtransfection of HeLa, HUVEC, HPAEC and HPMEC cells

Primary Human Pulmonary Microvascular Endothelial Cells (HPMEC) are most appropriate for studying human lung diseases and are isolated from the lung from a single donor. Since lung tissue contains blood and lymphatic capillaries, HPMEC comprise Blood and Lymphatic Microvascular Endothelial Cells. The cells were routinely analyzed by immunofluorescent staining: they stain positive for CD31 and von Willebrand factor and negative for smooth muscle alpha-actin. HeLa cells, HUVEC, HPMEC and HPAEC (human pulmonary artery endothelial cells) cells, respectively, were grown in DMEM complete medium or Endothelial Cell Growth Medium (PromoCell, Heidelberg Germany, CatNo.: C-22020) respectively. The Endothelial Cell Growth Medium was basal Medium supplemented with Fetal Calf Serum (0.05 ml/ml), Endothelial Cell Growth Supplement (bovine hypothalamic extract; 0.004 ml ml), Heparin 90µg/ml and Hydrocortisone 1µg/ml. The experiments with HUVEC and HPMEC cells were performed on cells with passage numbers below 8.

mRNA transfections were carried out using Lipofectamin MessengerMAX (Invitrogen, Carlsbad, CA; Thermo Fisher Scientific). For mRNA transfection in 24-well plate format 1×10⁵ cells/well were seeded 24h before transfection to reach 70-90 confluence immediate before transfection. For one single well 1.5µl Lipofectamine MessengerMAX Reagent was added to 25µl OptiMEM, vortex and incubated for 10 min at RT. In a second well add 500ng or 1µg mRNA were added to 25µl OptiMEM, vortex. After incubation diluted mRNA was added to diluted Lipofectamin, mixed well, incubated for 5 min at RT and added dropwise to cells in the presence of 0,5 ml of DMEM complete medium for HeLa. For HUVEC, HPMEC and HPAEC the Endothelial Cell Growth medium was replaced and the cells washed with OptiMEM and then added the Lipofectamin MessengerMAX mRNA complex solution to the well with 0,5 ml OptiMEM prewarmed to 37 °C. Cells were incubated at 37°C for 2h and the OptiMEM medium was replaced with complete Endothelial Cell Growth Medium (PromoCell, Heidelberg Germany) and transfected cells or serum were analyzed at the indicated time points. Alternatively, mRNA-LNPs based on the cationic lipids *L*-Arginyl-β-alanine-*N-*palmityl-*N*-oleyl-amide or β-(*L*-Arginyl)-*L*-2,3-diamino propionic acid-*N*-palmityl-*N*-oleyl-amide in combination with neutral and PEGylated co-lipids can be used for in vitro transfection.

Successful introduction of mRNA into host cells can be monitored using various known methods, such as a fluorescent marker, such as Green Fluorescent Protein (GFP), such as reporter enzymes (e.g. luciferase derivatives) or transfection of a modified mRNA can also be determined by measuring the protein expression level of the target polypeptide by e.g., Western Blotting or immunocytochemistry or ELISA.

### Nano-Luc Luciferase activity reporter measurement

A time dependent quantitative detection of Nano-Glo-Luciferase expression (Promega) was performed for secreted versions by analyzing samples from the tissue culture supernatant according to the reporter assay technical manual Nano-Glo Luciferase Assay System (Promega). In brief, 5-20µl of serum or lysate were diluted in 100µl H₂O final volume and equal volume of reconstituted Nano-Glo luciferase assay reagent was combined in a 96 well GloMax 96 Microplate. After at least 3 min incubation at RT luminescence could be measured in an appropriate luminometer.

### Detection of Ang-1, COM-Ang-1, CMP-Ang-1 protein in cell lysates and supernatant

A time dependent quantitative detection of secreted Ang-1 and secreted COMP-Ang-1 or COMP-Ang-2 expression is performed by analyzing samples from the supernatant tissue culture by ELISA (Human Angiopoietin-1 Quantikine ELISA Kit (DANG10, , R&D systems) or by Western blot according to the technical manual.

500 ng of COMP-Ang-1 (mRNA sequence shown in SEQ ID NO: PAN05) with poly A tail of approximately 120 nucleotides not shown in sequence; 5 'cap,) fully modified with 5-methylcytidine and pseudouridine (COMP-Ang-1, 5mC/pU), fully modified with 5-methylcytidine and Nl-methyl-pseudouridine (COMP-Ang-1,5mC/NlmpU) or unmodified (COMP-Ang-, unmod) is transfected into HUVEC or HPMEC (Human Pulmonary Microvascular Endothelial Cells, PromoCell, Heidelberg) using Lipofectamin MessengerMAX (Invitrogen, Carlsbad, CA; Thermo Fisher Scientific). The supernatant is harvested and run by ELISA 4 hours after transfection to determine the protein expression and cytokine induction.

For immunoblot detection of Ang-1 and derivatives protein lysates were loaded on NuPage SDS-PAGE system (chambers and power supply) with 1.5mm ready-to-use Bis-Tris gels and 4-12% acrylamide gradient with MOPS-buffer as running aid (all Life Technologies, Grand Island, NY). Each lysate sample was prepared to 40ul final volume. This sample contained 25ug protein lysate in variable volume, RIPA buffer to make up volume to 26ul, 4ul of l0x reducing agent and 10µl 4x SDS loading buffer (both from Life Technologies, Grand Island, NY). Samples were heated at 95oC for 5min and loaded on the gel. Standard settings were chosen by the manufacturer, 200V, 120mA and max. 25 W. Run time was 60min, but no longer than running dye reaching the lower end of the gel.

After the run is terminated, the plastic case is cracked and the encased gel transferred to a ready-to-use nitrocellulose membrane kit and power supply (iBLOT; LifeTechnologies, Grand Island, NY). Using default settings, the protein lysate is transferred by high Ampere electricity from the gel to the membrane.

After the transfer, the membranes were incubated in 5% BSA in IX TBS for 15 minutes then in 5% BSA in IX TBS + 0.1 % Tween for another 15 minutes. Primary antibodies (Ang-1 ab183701 Abeam, Cambridge, UK) against human Ang-1 proteins are applied in 3ml of 5% BSA in IX TBS solution at a 1:5000 dilution overnight at 4°C and gentle agitation on an orbital shaker. Membranes are washed 3 times with 1X TBS/0.1 % Tween, 5 minutes each time with gentle agitation. The secondary antibody (Goat anti-rabbit HRP conjugate; Abeam, Cambridge, MA) is conjugated to horse radish peroxidase and binds to the primary antibody antibodies. The secondary antibody is diluted of 1:10000 in 5% BSA in IX TBS and incubated for 1 hr at RT. At the end of incubation time, the membranes are washed 3 times with IX TBS/0.1 % Tween, 5 minutes each time with gentle agitation. The membranes are developed in 5ml Pierce WestPico Chemiluminescent Subtrate (Thermo Fisher, Rockford, IL) as directed. The Western Blot detects protein around the expected size of 70 kd for human Ang-1, and of 37kDa for COMP-Ang-1 and CMP-Ang1. For reference, recombinant human Ang-1 protein was purchased from R&D Systems (biotechne).

### Results

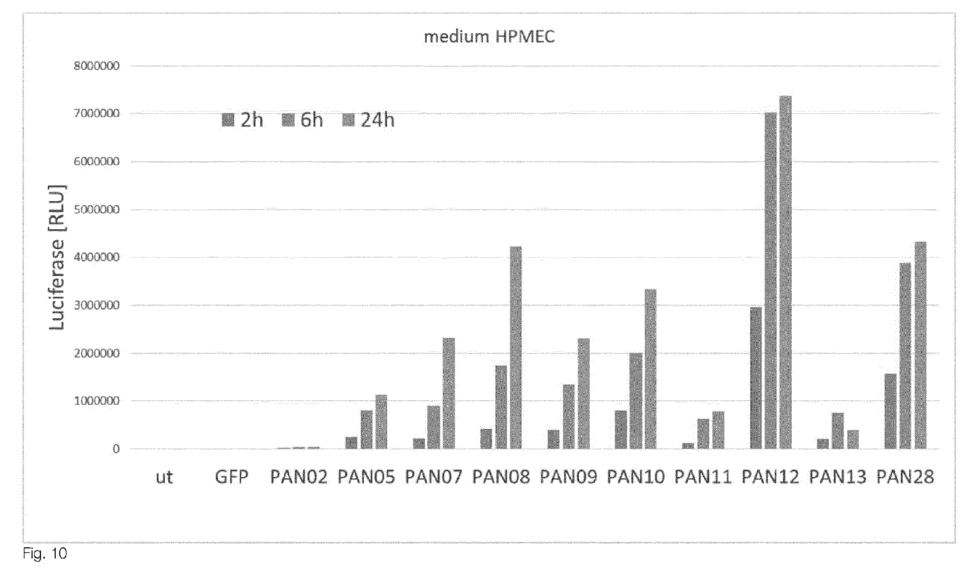

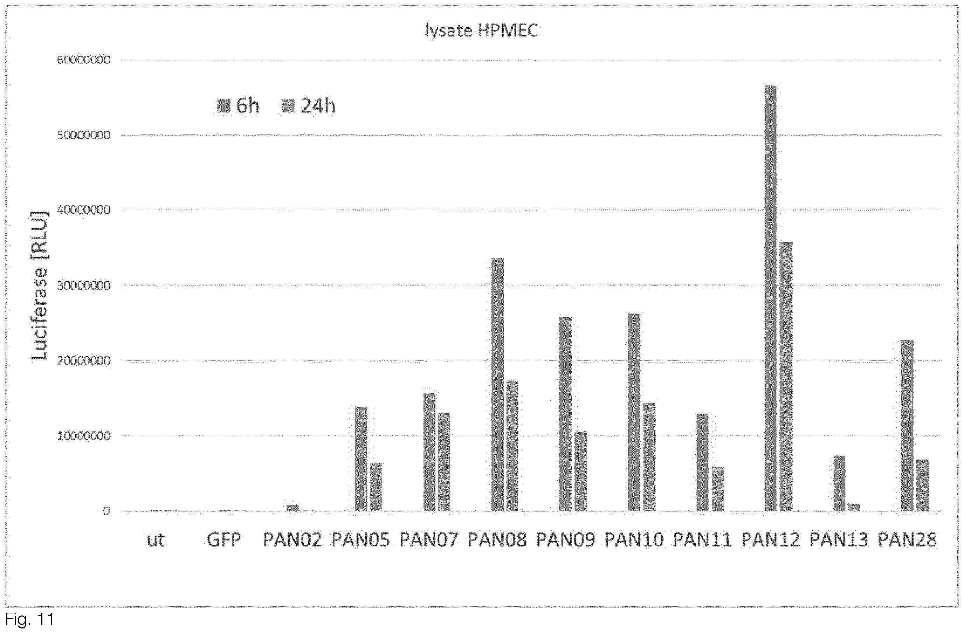

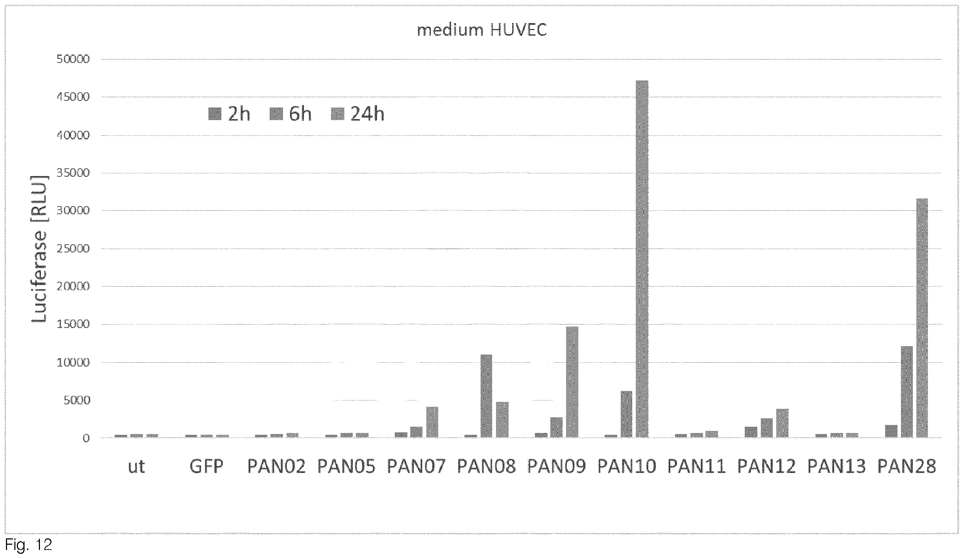

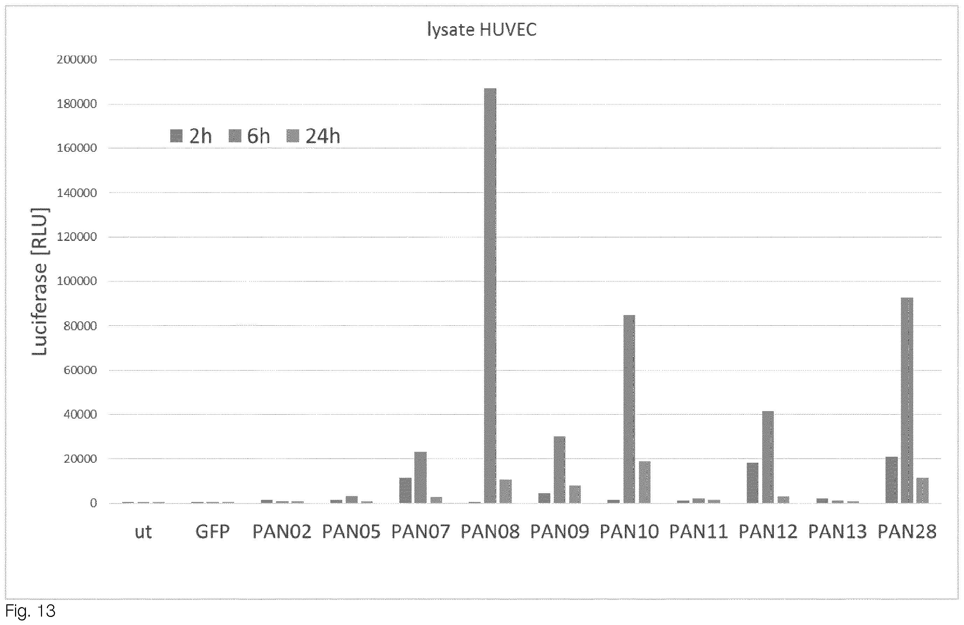

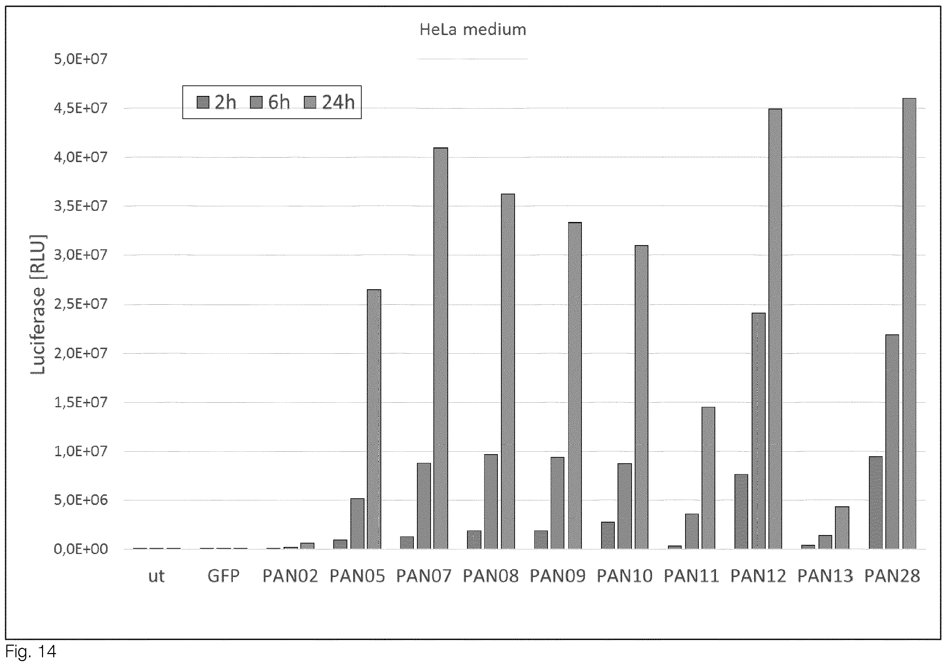

Protein expression data presented in the Figures 10 to 14, 72 to 85 and 92 to 94 underline that combinations of different regulatory nucleotide sequences flanking a coding region can significantly change the protein expression levels. Such change is further impacted by modification of the nucleotides forming the constructs as exemplified by replacing 100% of uridine and 100% cytidine nucleotides by pseudo-uridine and 5-methyl-cytidine (the constructs marked with "*" in Figure 74 and Figures 77 to 85. Changes are not only observed in a particular cell type but also across different cell types. For example, PAN02 (wild-type Ang-1 mRNA) shows the lowest Luciferase activity in all cell lines (Figures 10 to 14). All other constructs show enhanced protein expressions throughout all cell types. Therefore, the approach of using regulatory sequences from different genes can increase protein expression activity. To this end, replacing the endogenous Ang-1 sequences have shown superior effects on the protein expression of the reporter.

Worth noting, even the same regulatory sequences showed distinct protein expression activity in different cell types (Figures 75 to 81. These data further support our approach that the use of specific regulatory sequences can influence the activity of protein expression in a context-specific, i.e. cell-type specific cellular environment.

Furthermore, we have shown that our data are also concerning a different aspect i.e. secretion. For example, the regulatory sequences used in construct PAN12 show cell-type specific (HPMEC) expression in whole cell lysates but also in medium (supernatant) (Figures 10-14). This indicated that these sequences are not only optimal for protein expression

(translation) but also secretion. In contrast, construct PAN28 shows expression in all cell types more or less equally. The regulatory sequences used in construct PAN29 (and PAN58), namely the 5'-UTR and the signal peptide sequence of MCP-1 in combination with the 3'-UTR of vWF, were found to be particularly useful for highly efficient protein expression in human (microvascular) pulmonary endothelial cells (see e.g. Figures 72 and 75). The regulatory sequences used in construct PAN54, namely the 5'-UTR of spinach chloroplast RPL12, the signal peptide sequence of MCP-1 and the 3'-UTR of vWF, were found to be equally efficient for protein expression and secretion as shown in Figure 92. As shown in Fig. 98 the regulatory sequences of PAN29 are also functional using three different ORFs, namely wt Ang-1 in PAN52; hCOMP-Ang-1 in PAN53 and CMP-Ang-1 in PAN59 in directing expression in primary human pulmonary microendothelial cells (HPMEC). In contrast the mRNA construct containing the endogenous 5'-and 3'-UTRs of human Ang-1 (PAN01) is not translated (Fig. 98). Fig. 99 shows that all four mRNA constructs display comparable quality (integrity and purity).

### Example 3: Tie-2 pathway activation after transfection of modified mRNAs encoding the wildtype Tie-2 (PAN60) or the activating Tie-2 mutations (e.g. R849W (PAN61))

Modified mRNAs containing heterologous 5' and 3' UTRs are generated by in vitro transcription of PCR templates as described previously in example 1 above. Different concentration of this mRNA encoding the activating Tie-2 mutations (e.g. R849W) are transfected in different cell lines (HeLa, primary endothelial cells such as HUVECs or HPMEC or HPAEC) next to mRNAs encoding the wild-type Tie-2 using Lipofectamin MessengerMAX (Invitrogen, Carlsbad, CA; Thermo Fisher Scientific.). For qualitative Western blot cell lysates at different time points post transfection of mRNAs (1-24 h) are probed with anti-phosphotyrosine antibody (R&D systems) to evaluate Tie-2 phosphorylation (pTyr, 140 kD,). Blots are stripped and re-probed with anti-Tie-2 antibody (R&D systems) to detect total Tie-2. For quantitative measurement two different ELISAs are performed measuring human Tie-2 or Phospho-Tie-2 using the human Tie-2 DuoSet ELISA kit or human-Phospho-Tie-2 DuoSet IC ELISA according to the manufactures (e.g. both ELISA-Kits from R&D systems) protocol.

Transfection of Tie-2 mutation (PAN61, R849W) encoding mRNAs increase the presence of Phospho-Tie-2 in comparison to cell lysates derived from cells transfected with mRNAs encoding wildtype Tie-2 mRNA or in comparison to non-transfected cells. This increase in Tie-2 phosphorylation is observed in the presence or absence of co-stimulation with recombinant human Ang-1 ligand and can be observed in different cell lines. To confirm the activation of the Tie-2 signaling pathway and to demonstrate a biological relevant functional downstream signal transduction by expressing the Tie-2 (R849W) derivate a phosphorylation of Akt is demonstrated in a time course experiment. These experiments indicate that mRNAs encoding for the Tie-2 activating mutation (R849W) are functional and stronger in activating the Tie-2 signalling pathway than wt Tie-2 encoding mRNAs. These hyperactivation of the pathway can be demonstrated in the absence or presence of Tie-2 ligands (e.g. Ang-1) in human endothelial and/or non-endothelial cells.

### Example 4: mRNA Formulation in cationic LNPs for in vivo applications by intravenous administration

For *in vivo* experiments mRNA-LNPs are prepared in a formulation process with β-(*L*-Arginyl)-*L*-2,3-diamino propionic acid-*N*-palmityl-*N*-oleyl-amide as cationic lipid. Alternatively, the cationic lipid *L*-Arginyl-β-alanine-*N*-palmityl-*N*-oleyl-amide can be used in an identical procedure to prepare mRNA-LNPs.

mRNA-LNP formulations are prepared using a modified procedure of a method described for siRNA (Chen, S., Tam, Y.Y., Lin, P.J., Sung, M.M., Tam, Y.K., and Cullis, P.R. (2016), Influence of particle size on the in vivo potency of lipid nanoparticle formulations of siRNA. J. Control. Release 235, 236-244. & (ii) patent application US20170121712).

Briefly, lipids are dissolved in ethanol at appropriate molar ratios (e.g. 50:49:1 β-(*L*-arginyl)-*L*-2,3-diamino propionic acid-*N*-palmityl-*N*-oleyl-amide: DPyPE: mPEG2000-DSPE). The lipid mixture is combined with an isotonic Sucrose solution of mRNA at a volume ratio of 2:1 (aqueous:ethanol) using a microfluidic mixer (NanoAssemblr^{®}; Precision Nanosystems, Vancouver, BC) and flow rates of 18ml/min. Similarly, LNP formulations can be obtained using citrate or acetate buffered mRNA solutions (pH 3-4) and a slightly differing mixing ratio of 3:1 (v/v; aqueous:ethanol) and flow rates of 12ml/min.

After the mixing process, the formulations are dialyzed against 10mM HEPES or TRIS buffered isotonic Sucrose solution using 3.5 K MWCO Slide-A-Lyzer Dialysis Cassettes (Thermo Fisher Scientific) for at least 18 hours at 4°C. Instead of Sucrose, other sugars like Trehalose or Glucose can be equally used within the formulation process.

Subsequently, the formulations are tested for particle size (Zetasizer Nano ZS instrument (Malvern Instruments Ltd, Malvern, UK), RNA encapsulation (Quant-iT RiboGreen RNA Assay Kit following manufacturer's (Thermo Fisher Scientific) protocol), and endotoxin and are found to be between 30 to 100 nm in size with a Zeta-potential of >25mV, display greater than 90% mRNA encapsulation and < 1 EU/ml of endotoxin.

mRNA-LNP formulations are stored at -80 °C at a concentration of RNA of ~ 0.3 µg/µl and an RNA to total lipid ratio of ~ 0.03-0.05 (wt/wt) until further *in vitro* or *in vivo* use.

## Claims

1. A recombinant nucleic acid construct comprising in 5'-> 3' direction
- a 5' UTR,
- a coding region coding for an effector molecule, and
- a 3' UTR,
wherein the 5' UTR is a 5' UTR of a gene coding for MCP-1 or a derivative thereof having a nucleotide identity of at least 85 %,
wherein the 3' UTR is a 3' UTR of a gene coding for vWF or a derivative thereof having a nucleotide identity of at least 85 %,
wherein the effector molecule is effective in restoring a cellular function of a cell or is effective in exercising a therapeutic effect in or on a cell, and
wherein the recombinant nucleic acid construct is different from a wild type mRNA coding for the effector molecule.

2. The recombinant nucleic acid construct of claim 1, wherein the 5' UTR and the 3' UTR of the recombinant construct are from different species.

3. The recombinant nucleic acid construct of claim 1, wherein the 5' UTR and the 3' UTR of the recombinant construct are from the same species.

4. The recombinant nucleic acid construct of any one of claims 1 to 3, wherein the construct comprises
(a) a poly-A tail, preferably at the 3' terminal end of the recombinant nucleic acid construct; and/or
(b) a CAP structure, preferably at the 5' terminal end of the recombinant nucleic acid construct;
and/or
(c) a IRES (internal ribosomal entry site) sequence, preferably at the 5' terminal end of the recombinant nucleic acid construct.

5. The recombinant nucleic acid construct of any one of claims 1 to 4, wherein the construct comprises a nucleic acid sequence coding for a signal peptide, preferably the signal peptide is in-frame with the nucleic acid sequence coding for an effector molecule and is arranged between the 5' UTR and the coding region coding for an effector molecule, more preferably the nucleotide sequence coding for a signal peptide is selected from the group comprising a nucleotide sequence coding for a signal peptide of MCP-1 or a derivative thereof having a nucleotide identity of at least 85 %, nucleotide sequence coding for a signal peptide of IL-6 or a derivative thereof having a nucleotide identity of at least 85 %, a nucleotide sequence coding for a signal peptide of Ang-2 or a derivative thereof having a nucleotide identity of at least 85 %, and a nucleotide sequence coding for a signal peptide of Ang-1 or a derivative thereof having a nucleotide identity of at least 85 %, most preferably the signal peptide allows secretion of the effector molecule.

6. The recombinant nucleic acid construct of any one of claims 1 to 5, wherein the cell a cellular function of which is restored and/or the cell in or on which a therapeutic effect is exercised is an endothelial cell, preferably a vascular endothelial cell, more preferably the vascular endothelial cell is a microvascular endothelial cell.

7. The recombinant nucleic acid construct of any one of claims 1 to 6, wherein the cellular function is one which can be restored by an effector molecule having anti-permeability effect of endothelial cells, an anti-vascular leakage effect, an anti-apoptotic effect of endothelial cells or an anti-inflammatory effect of endothelial cells or an anti-stress response effect.

8. The recombinant nucleic acid construct of claim 7, wherein the effect is linked to or associated with the TIE-2 signalling pathway, VEGF-receptor pathway, NOTCH signalling pathway, PI3-kinase pathway, eNOS signalling pathway, sirtuin-dependent metabolic and energy homeostasis pathway, oxidative stress pathway, shear stress response pathway, ET-1 signal transduction pathway, NO-mediated signal transduction pathway, and mechanochemical transduction pathway.

9. The recombinant nucleic acid construct of any one of claims 1 to 8, wherein the recombinant nucleic acid construct is an mRNA.

10. The recombinant nucleic acid construct of any one of claims 1 to 9, wherein the recombinant nucleic acid construct consists of ribonucleotides, deoxyribonucleotides or a combination thereof.

11. A vector comprising a nucleic acid construct of any one of claims 1 to 10.

12. A cell comprising a nucleic acid construct of any one of claims 1 to 10 and/or a vector of claim 11.

13. A delivery vehicle comprising a nucleic acid construct of any one of claims 1 to 10, wherein the delivery vehicle is a cationic lipid delivery particle, preferably the particle is a nanoparticle, more preferably the average size of the nanoparticle is from about 30 nm to about 200 nm, preferably from about 30 nm to about 140 nm and more preferably from about 30 nm to about 60 nm.

14. A pharmaceutical composition comprising a nucleic acid construct of any one of claims 1 to 10, a vector of claim 11, a cell of claim 12 and/or a delivery vehicle of claims 13, and a pharmaceutically acceptable diluent.

15. The recombinant nucleic acid construct of any one of claims 1 to 10, for use in a method for the treatment and/or prevention of a disease or for use in a method for restoring a cellular function of a cell.

## Patentansprüche

1. Rekombinantes Nukleinsäurekonstrukt umfassend in 5'-> 3' - Richtung
- eine 5' UTR,
- eine für ein Effektormolekül codierende Region, und
- eine 3' UTR,
wobei die 5' UTR eine 5' UTR eines für MCP-1 codierenden Gens ist oder ein Derivat davon mit einer Nukleotididentität von wenigstens 85 %,
wobei die 3' UTR eine 3' UTR eines für vWF codierenden Gens ist oder ein Derivat davon mit einer Nukleotididentität von wenigstens 85 %,
wobei das Effektormolekül wirksam ist bei der Wiederherstellung einer Zellfunktion einer Zelle oder wirksam ist bei der Entfaltung einer therapeutischen Wirkung in einer Zelle oder auf eine Zelle, und
wobei das rekombinante Nukleinsäurekonstrukt verschieden ist von einer mRNA vom Wildtyp, die für das Effektormolekül codiert.

2. Rekombinantes Nukleinsäurekonstrukt nach Anspruch 1, wobei die 5' UTR und die 3' UTR des rekombinanten Konstruktes von verschiedenen Arten stammen.

3. Rekombinantes Nukleinsäurekonstrukt nach Anspruch 1, wobei die 5' UTR und die 3' UTR des rekombinanten Konstruktes von der gleichen Art stammen.

4. Rekombinantes Nukleinsäurekonstrukt nach einem der Ansprüche 1 bis 3, wobei das Konstrukt umfasst:
(a) einen Poly-A-Schwanz, bevorzugterweise am 3'- terminalen Ende des rekombinanten Nukleisäurekonstruktes; und/oder
(b) eine CAP-Struktur, bevorzugterweise am 5'-terminalen Ende des rekombinanten Nukleinsäurekonstruktes; und/oder
(c) eine IRES (interne Ribosomeneintrittsstellen) - Sequenz, bevorzugterweise am 5'-terminalen Ende des rekombinanten Nukleinsäurekonstruktes.

5. Rekombinantes Nukleinsäurekonstrukt nach einem der Ansprüche 1 bis 4, wobei das Konstrukt eine Nukleinsäuresequenz umfasst, die für ein Signalpeptid codiert, bevorzugterweise ist das Signalpeptid im Leserahmen mit der Nukleinsäuresequenz, die für ein Effektormolekül codiert, und zwischen der 5' UTR und der für ein Effektormolekül codierenden codierenden Region angeordnet, bevorzugtererweise ist die für ein Signalpeptid codierende Nukleotidsequenz ausgewählt aus der Gruppe umfassend eine für ein Signalpeptid von MCP-1 codierende Nukleotidsequenz oder ein Derivat davon mit einer Nukleotididentität von wenigstens 85 %, eine für ein Signalpeptid von IL-6 codierende Nukleotidsequenz oder ein Derivat davon mit einer Nukleotididentität von wenigstens 85 %, eine für ein Signalpeptid von Ang-2 codierende Nukleotidsequenz oder ein Derivat davon mit einer Nukleotididentität von wenigstens 85 % und eine für ein Signalpeptid von Ang-1 codierende Nukleotidsequenz oder ein Derivat davon mit einer Nukleotididentität von wenigstens 85 %, am bevorzugtesten erlaubt das Signalpeptid Sekretion des Effektormoleküls.

6. Rekombinantes Nukleinsäurekonstrukt nach einem der Ansprüche 1 bis 5, wobei die Zelle, von der eine Zellfunktion wiederhergestellt ist, und/oder die Zelle, in der oder an der eine therapeutische Wirkung entfaltet ist, eine Endothelzelle ist, bevorzugterweise eine vaskuläre Endothelzelle, bevorzugtererweise ist die vaskuläre Endothelzelle eine mikrovaskuläre Endothelzelle.

7. Rekombinantes Nukleinsäurekonstrukt nach einem der Ansprüche 1 bis 6, wobei die zelluläre Funktion eine solche ist, die wiederhergestellt werden kann durch ein Effektormolekül mit einer Antipermeabilitätswirkung von Endothelzellen, mit einer der Leckage von Vaskulatur entgegentretende Wirkung, mit einer anti-apoptotischen Wirkung von Endothelzellen, oder einer anti-inflammatorischen Wirkung von Endothelzellen oder einer Anti-Stress-Antwort-Wirkung.

8. Rekombinantes Nukleinsäurekonstrukt nach Anspruch 7, wobei die Wirkung verknüpft oder verbunden ist mit dem TIE-2 Signal-Weg, dem VEGF-Rezeptor-Weg, dem NOTCH-Signal-Weg, dem PI3-Kinase-Weg, dem eNOS-Signal-Weg, dem Sirtuin-abhängigen metabolischen und Energie-Homöostase-Weg, dem oxidativen Stress-Weg, dem Scherstress-Antwort-Weg, dem ET-1-Signalübertragungsweg, dem NO-vermittelten Signalübertragungsweg und dem mechanochemischen Übertragungsweg.

9. Rekombinantes Nukleinsäurekonstrukt nach einem der Ansprüche 1 bis 8, wobei das rekombinante Nukleinsäurekonstrukt eine mRNA ist.

10. Rekombinantes Nukleinsäurekonstrukt nach einem der Ansprüche 1 bis 9, wobei das rekombinante Nukleinsäurekonstrukt aus Ribonukleotiden, Desoxyribonukleotiden oder einer Kombination davon besteht.

11. Vektor umfassend ein Nukleinsäurekonstrukt nach einem der Ansprüche 1 bis 10.

12. Zelle umfassend ein Nukleinsäurekonstrukt nach einem der Ansprüche 1 bis 10 und/oder einen Vektor nach Anspruch 11.

13. Abgabevehikel umfassend ein Nukleinsäurekonstrukt nach einem der Ansprüche 1 bis 10, wobei das Abgabevehikel ein kationisches Lipidabgabepartikel ist, bevorzugterweise ist das Partikel ein Nanopartikel, bevorzugtererweise beträgt die Durchschnittsgröße des Nanopartikels etwa 30 nm bis etwa 200 nm, bevorzugterweise etwa 30 nm bis etwa 140 nm und bevorzugtererweise etwa 30 nm bis etwa 60 nm.

14. Pharmazeutische Zusammensetzung umfassend ein Nukleinsäurekonstrukt nach einem der Ansprüche 1 bis 10, einen Vektor nach Anspruch 11, eine Zelle nach Anspruch 12 und/oder ein Abgabevehikel nach Anspruch 13, und ein pharmazeutisch akzeptables Verdünnungsmittel.

15. Rekombinantes Nukleinsäurekonstrukt nach einem der Ansprüche 1 bis 10, zur Verwendung in einem Verfahren zur Behandlung und/oder Prävention einer Erkrankung oder zur Verwendung in einem Verfahren zum Wiederherstellen einer Zellfunktion einer Zelle.

## Revendications

1. Construction d'acide nucléique de recombinaison comprenant dans la direction 5'-> 3'
- une 5' UTR,
- une région codante codant pour une molécule effectrice, et
- une 3' UTR,
dans laquelle la 5' UTR est une 5' UTR d'un gène codant pour MCP-1 ou un dérivé de celui-ci ayant une identité nucléotidique d'au moins 85 %,
dans laquelle la 3' UTR est une 3' UTR d'un gène codant pour vWF ou un dérivé de celui-ci ayant une identité nucléotidique d'au moins 85 %,
dans laquelle la molécule effectrice est efficace pour restaurer une fonction cellulaire d'une cellule ou est efficace pour exercer un effet thérapeutique dans ou sur une cellule, et
dans laquelle la construction d'acide nucléique de recombinaison est différente d'un ARNm de type sauvage codant pour la molécule effectrice.

2. Construction d'acide nucléique de recombinaison selon la revendication 1, dans laquelle la 5' UTR et la 3' UTR de la construction de recombinaison proviennent d'espèces différentes.

3. Construction d'acide nucléique de recombinaison selon la revendication 1, dans laquelle la 5' UTR et la 3' UTR de la construction de recombinaison proviennent de la même espèce.

4. Construction d'acide nucléique de recombinaison selon l'une quelconque des revendications 1 à 3, dans laquelle la construction comprend
(a) une queue poly-A, de préférence à l'extrémité 3' terminale de la construction d'acide nucléique de recombinaison ;
et/ou
(b) une structure CAP, de préférence à l'extrémité 5' terminale de la construction d'acide nucléique de recombinaison ;
et/ou
(c) une séquence IRES (site d'entrée ribosomique interne), de préférence à l'extrémité 5' terminale de la construction d'acide nucléique de recombinaison.

5. Construction d'acide nucléique de recombinaison selon l'une quelconque des revendications 1 à 4, dans laquelle la construction comprend une séquence d'acide nucléique codant pour un peptide signal, de préférence le peptide signal est en cadre avec la séquence d'acide nucléique codant pour une molécule effectrice et est disposé entre la 5' UTR et la région codante codant pour une molécule effectrice, plus préférablement la séquence nucléotidique codant pour un peptide signal est choisie dans le groupe comprenant une séquence nucléotidique codant pour un peptide signal de MCP-1 ou un dérivé de celui-ci ayant une identité nucléotidique d'au moins 85 %, une séquence nucléotidique codant pour un peptide signal d'IL-6 ou un dérivé de celui-ci ayant une identité nucléotidique d'au moins 85 %, une séquence nucléotidique codant pour un peptide signal d'Ang-2 ou un dérivé de celui-ci ayant une identité nucléotidique d'au moins 85 % et une séquence nucléotidique codant pour un peptide signal d'Ang-1 ou un dérivé de celui-ci ayant une identité nucléotidique d'au moins 85 %, le plus préférablement le peptide signal permet la sécrétion de la molécule effectrice.

6. Construction d'acide nucléique de recombinaison selon l'une quelconque des revendications 1 à 5, dans laquelle la cellule dont une fonction cellulaire est restaurée et/ou la cellule dans ou sur laquelle un effet thérapeutique est exercé est une cellule endothéliale, de préférence une cellule endothéliale vasculaire, plus préférablement la cellule endothéliale vasculaire est une cellule endothéliale microvasculaire.

7. Construction d'acide nucléique de recombinaison selon l'une quelconque des revendications 1 à 6, dans laquelle la fonction cellulaire est une fonction qui peut être restaurée par une molécule effectrice ayant un effet anti-perméabilité des cellules endothéliales, un effet anti-fuite vasculaire, un effet anti-apoptotique des cellules endothéliales ou un effet anti-inflammatoire des cellules endothéliales ou un effet de réponse anti-stress.

8. Construction d'acide nucléique de recombinaison selon la revendication 7, dans laquelle l'effet est lié ou associé à la voie de signalisation TIE-2, la voie du récepteur VEGF, la voie de signalisation NOTCH, la voie PI3-kinase, la voie de signalisation eNOS, la voie de l'homéostasie métabolique et énergétique dépendante de la sirtuine, la voie du stress oxydatif, la voie de réponse au stress de cisaillement, la voie de transduction du signal ET-1, la voie de transduction du signal médiée par NO et la voie de transduction mécanochimique.

9. Construction d'acide nucléique de recombinaison selon l'une quelconque des revendications 1 à 8, dans laquelle la construction d'acide nucléique de recombinaison est un ARNm.

10. Construction d'acide nucléique de recombinaison selon l'une quelconque des revendications 1 à 9, dans laquelle la construction d'acide nucléique de recombinaison consiste en des ribonucléotides, des désoxyribonucléotides ou une combinaison de ceux-ci.

11. Vecteur comprenant une construction d'acide nucléique selon l'une quelconque des revendications 1 à 10.

12. Cellule comprenant une construction d'acide nucléique selon l'une quelconque des revendications 1 à 10 et/ou un vecteur selon la revendication 11.

13. Véhicule d'administration comprenant une construction d'acide nucléique selon l'une quelconque des revendications 1 à 10, dans lequel le véhicule d'administration est une particule d'administration de lipide cationique, de préférence la particule est une nanoparticule, plus préférablement la taille moyenne de la nanoparticule est d'environ 30 nm à environ 200 nm, de préférence d'environ 30 nm à environ 140 nm et plus préférablement d'environ 30 nm à environ 60 nm.

14. Composition pharmaceutique comprenant une construction d'acide nucléique selon l'une quelconque des revendications 1 à 10, un vecteur selon la revendication 11, une cellule selon la revendication 12 et/ou un véhicule d'administration selon la revendication 13, et un diluant pharmaceutiquement acceptable.

15. Construction d'acide nucléique de recombinaison selon l'une quelconque des revendications 1 à 10, destinée à être utilisée dans un procédé de traitement et/ou de prévention d'une maladie ou destinée à être utilisée dans un procédé de restauration d'une fonction cellulaire d'une cellule.
